# EUROPEAN PATENT SPECIFICATION

(11) **EP 4 552 700 B1**
(45) Date of publication and mention of the grant of the patent: **25.11.2026**
(21) Application number: 25162840.0
(22) Date of filing: 26.02.2021
(51) Int. Cl.: A61K 35/74, A61K 31/405, A61K 31/685, A61K 45/06, A61P 3/10, A61P 9/00, A61P 37/00

(54) **INTERVENTION STRATEGY FOR PREVENTION OR TREATMENT OF DIABETES MELLITUS, AUTOIMMUNE DISEASE OR INFLAMMATORY DISEASE**
INTERVENTIONSSTRATEGIE ZUR PRÄVENTION ODER BEHANDLUNG VON DIABETES MELLITUS, AUTOIMMUNKRANKHEIT ODER ENTZÜNDUNGSKRANKHEIT
STRATÉGIE D'INTERVENTION POUR LA PRÉVENTION OU LE TRAITEMENT DU DIABÈTE SUCRÉ, D'UNE MALADIE AUTO-IMMUNE OU D'UNE MALADIE INFLAMMATOIRE

(30) Priority: 28.02.2020 NL 2025020; 28.02.2020 NL 2025021; 28.02.2020 NL 2025022
(43) Date of publication of application: 14.05.2025
(60) Divisional application: 26191610.0 / 4 803 156
(62) Document number(s) of the earlier application(s) in accordance with Art. 76 EPC: 21708001.9 / 4 110 328
(73) Proprietor: Stichting Amsterdam UMC, 1081 HV Amsterdam (NL); Wageningen Universiteit, 6708 PB Wageningen (NL)
(72) Inventor: NIEUWDORP, Max, 1018 HD AMSTERDAM (NL); DE VOS, Willem Meindert, 1082 PB AMSTERDAM (NL)
(74) Representative: EP&C

(56) References cited:
- WO-A1-2017/072278
- STEFANAKI CHARIKLEIA ET AL: "Examining the gut bacteriome, virome, and mycobiome in glucose metabolism disorders: Are we on the right track?", METABOLISM, W.B. SAUNDERS CO., PHILADELPHIA, PA, US, vol. 73, 1 May 2017 (2017-05-01), pages 52 - 66, XP085134871, ISSN: 0026-0495, DOI: 10.1016/J.METABOL.2017.04.014

## Description

### Technical field

The present invention relates to the prevention and/or treatment of an inflammation-related disease chosen from the group consisting of Diabetes mellitus, autoimmune disease, and inflammatory disease, more specifically compositions comprising specific microorganisms for use in said prevention and/or treatment.

### Background of the invention

The role of inflammation in both type 1 and type 2 Diabetes mellitus has generated increasing interest in targeting inflammation in order to improve prevention and treatment of the disease. Evidence has shown that inflammatory pathways are the principal mediators in the pathology of diabetes under the stimulus of risk factors including obesity or being overweight.

Also, the relationship between diabetes and cardiovascular disease is well established, with the risk for cardiovascular disease being significantly elevated in patients with diabetes. Atherosclerosis represents the most common cause of coronary artery disease, and the characterization of the disease as a chronic low-grade inflammatory condition is now largely accepted.

In addition, the classic sign of autoimmune diseases is also inflammation. The diseases may have flare-ups, i.e. when they get worse, and remissions, i.e. when symptoms get better or disappear. Treatment of autoimmune diseases depends on the type of disease, but in most cases one important goal is to reduce inflammation.

The current therapeutics for Diabetes, cardiovascular diseases, autoimmune diseases and inflammatory diseases have anti-inflammatory properties in addition to their major modes of action. Non-pharmacological treatments, such as lifestyle interventions reduce inflammation status, for examples assessed as circulating C-reactive protein (CRP) and Interleukin-6 (IL-6) concentrations, and improve cardiovascular and all-cause mortality.

This makes therapeutic approaches that target inflammation an attractive area for research.

Novel therapeutic strategies are needed to improve quality of life in patients with inflammation related disorders including Diabetes mellitus, autoimmune disease, inflammatory disease, and cardiovascular disease.

WO2017072278A1 discloses *faecalibacterium prausnitzii* and *desulfovibrio piger for* use in the treatment or prevention of type 2 diabetes and bowel diseases.

There remains a need to develop a new or improved prevention and/or treatment strategy. It is an objective of the present disclosure to meet this need.

### Summary of the invention

The invention is as set out in the appended claims.

The present inventors investigated whether administration of fecal transplants, from either allogenic (healthy donor) or autologous (own) sources, may have beneficial effects in patients with autoimmune disease. Confirming earlier studies, it was found that administration of autologous fecal transplants can bring about an immune reset within patients with autoimmune disease, and thereby reduction of the severity of the autoimmune disease.

The present inventors surprisingly found that the beneficial effects could be traced back to specific constituents in the fecal matter. These constituents with therapeutic application were found to relate most specifically to
- bacteria of the genus *Desulfovibrio,* preferably chosen from the group consisting of *Desulfovibrio piger, Desulfovibrio fairfieldensis, Desulfovibrio desulfuricans, desulfovibrio indonensis, Desulfovibrio alaskensis, Desulfovibrio vulgaris, Desulfovibrio vietnamensis* and *Desulfovibrio gigas,* most preferably *Desulfovibrio piger.* and
- the metabolites 6-bromotryptophan, 1-myristoyl-2-arachidonoyl-glycero-phosphocholine (MA-GPC) and 1-arachidonoyl-glycero-phosphocholine (A-GPC), or, more generally, chloro, fluoro or bromo-substituted tryptophans, or mono- or di-fatty acid substituted glycerol phosphocholines, or any derivative or functional equivalent of these.

Upon further research, it was surprisingly found that *Desulfovibrio* species and/or metabolites according to the present disclosure, in particular 6-bromotryptophan, have an anti-inflammatory effect, i.e. reduce inflammation status and reduce inflammation markers e.g. related to type 1 and type 2 Diabetes mellitus, autoimmune disease, cardiovascular disease, and inflammatory disease such as Systemic Inflammatory Response Syndrome (SIRS) or sepsis.

Further, an inverse relationship was found between plasma 6-bromotryptophan levels and presence of type 2 Diabetes mellitus. This suggests that *Desulfovibrio* species and/or metabolites according to the present disclosure, in particular 6-bromotryptophan, may contribute to the prevention of type 2 Diabetes mellitus and may contribute to the prevention or treat (microvascular and macrovascular) cardiovascular complications. Further, it was found that *Desulfovibrio* species and/or metabolites according to the present disclosure, in particular 6-bromotryptophan, may promote insulin secretion by beta cells.

Mechanistically, the present inventors unraveled that the biological actions of 6-BT appear distinct from the ones of tryptophan. 6-BT does not act through activation of AhR, but it does inhibit NFkB activation and enhance mitochondrial metabolism. The latter is typically used by cells harboring an anti-inflammatory phenotype. Because of its broad effects on multiple cell types, its inhibitory action on NFkB signaling and its promotion of mitochondrial metabolism and fitness, 6-BT can be a very useful therapeutic not only in the context of type 1 and type 2 diabetes but also in many other inflammation related disorders such as sepsis, Systemic Inflammatory Response Syndrome (SIRS), and cardiovascular diseases.

In addition, without being bound by any theory, the present inventors consider that said *Desulfovibrio* species and said metabolites according to the present disclosure may modulate the immune system, e.g. by resetting B-cell clone function and regulatory T-cells, which in turn may inhibit autoimmune response.

It is thought that during early life, the immune system is trained via continuous crosstalk with a developing intestinal microbiome composition. In this way, the intestinal microbiome plays an essential role in modulating adaptive immune cell development, composition, and function (see e.g. Agace and McCoy Immunity 46, April 18, 2017). It is this process, amongst others, that leads to a proper functioning immune system, devoid of autoimmune factors.

However, the crosstalk between the immune system and the intestinal microbiome, or the end result thereof, may be disturbed, which can lead to the production of autoimmune antibodies (by B cells) and formation of autoreactive T cells. The treatment according to the present disclosure may overcome this disturbance, by re-initiating the crosstalk between the immune system and the intestinal microbiome (including its specific bacteria, and/or derived products such as metabolites) and resulting in inhibition of the auto-immune response.

Accordingly, the use of the above-mentioned *Desulfovibrio* species and/or metabolites in autoimmune diseases, may stop the autoimmune destruction of targeted tissue and reestablish immune tolerance. The present disclosure can achieve this by stimulating the immune system, wherein the *Desulfovibrio* species and/or metabolites are preferably administered to the duodenum (directly or indirectly such as via oral administration). The present disclosure preferably does not aim to alter the intestinal microbiome, i.e. the gut microbiota composition.

WO2019168401 discloses the use of fecal matter in the prevention and treatment of autoimmune disease, wherein the fecal matter is autologous to the subject, and preferably administered to the small intestine, preferably the duodenum, where it can initiate an immune reset and thereby reduce severity of the autoimmune disease. However, there is room for improvement in the therapeutic efficacy of the method of WO2019168401, and its treatment is laborious and difficult to upscale.

Hence, the present disclosure discloses the prevention or treatment of inflammation-related diseases chosen from the group consisting of
- type 1 diabetes mellitus;
- type 2 diabetes mellitus,
- cardiovascular diseases, particularly coronary artery disease (also known as coronary heart disease and ischemic heart disease), peripheral arterial disease, cerebrovascular disease (for example stroke or TIA, Transient Ischemic Attack), atherosclerosis, stenosis, renal artery stenosis, aortic disease, aortic aneurysm, cardiomyopathy, hypertensive heart disease, hypertension, heart failure, pulmonary heart disease, cardiac dysrhythmias, cardiovascular inflammation, inflammatory heart disease, endocarditis, inflammatory cardiomegaly, myocarditis, eosinophilic myocarditis, valvular heart disease, congenital heart disease, or rheumatic heart disease;
- inflammatory disease, in particular cardiovascular inflammation, for example carditis, endocarditis, myocarditis, pericarditis, vasculitis, arteritis, phlebitis, or capillaritis, or for example inflammation of the gastrointestinal tract, e.g. esophagitis, gastritis, gastroenteritis, enteritis, colitis, enterocolitis, duodenitis, ileitis, caecitis, appendicitis, or proctitis. Inflammatory diseases further include hepatic inflammation, pulmonary inflammation, skeletal inflammation, Systemic Inflammatory Response Syndrome (SIRS), sepsis; and
- autoimmune diseases, particularly (endocrine) autoimmune diseases (e.g. Hashimoto hypothyroidism, Graves hyperthyroidism, Rheumatoid arthritis, Celiac disease, Asthma/COPD, Addison's disease, IBD (Crohn and colitis ulcerosa), Systemic lupus erythematosus, Vasculitis, Guillain Barre and CIDP, Multiple sclerosis, Psoriasis (arthritis), Vitiligo, and Bechterew's disease.

Additionally or alternatively, the use according to the present disclosure may be for improving general health and/or reducing inflammation status.

The present disclosure thus also encompasses prevention of the recited diseases, i.e. type 1 or type 2 diabetes mellitus, cardiovascular disease, inflammatory disease or autoimmune disease. Accordingly, the said *Desulfovibrio* species and/or metabolites according to the present disclosure can be administered to a subject in order to avoid onset of any of said diseases, for example in subjects wherein risk markers associated with pre-stage or early stage of the respective disease have been detected (before diagnosis of the respective disease). Such primary or secondary prevention strategy may prevent the development of the disease.

Some of the autoimmune diseases as referred herein are currently treated with immune therapy, such as by using antibodies to TNFα. However, these expensive immune therapies may only be successful in a subset of patients. This has been ascribed to deviations in the intestinal microbiota (Kolho et al 2015 Am J Gastroenterol.110(6):921-30). The present inventors envisage that treatment with a TNFα antagonist or anti-TNFα may be synergistic with treatment according to the present invention, e.g. administration of the *Desulfovibrio* species and/or metabolites according to the present disclosure.

### Detailed description of the invention

The present disclosure relates to the prevention or treatment of inflammation-related diseases chosen from the group consisting of type 1 or type 2 Diabetes mellitus, autoimmune disease, cardiovascular disease and inflammatory disease, by using one or more agent(s) chosen from the group consisting of:
- *Desulfovibrio* species, preferably chosen from the group consisting of *Desulfovibrio piger, Desulfovibrio fairfieldensis, Desulfovibrio desulfuricans, desulfovibrio indonensis, Desulfovibrio alaskensis, Desulfovibrio vulgaris, Desulfovibrio vietnamensis* and *Desulfovibrio gigas,* most preferably *Desulfovibrio piger,* and
- the compounds (metabolites) 6-bromotryptophan, 1-myristoyl-2-arachidonoyl-glycero-phosphocholine (MA-GPC) and 1-arachidonoyl-glycero-phosphocholine (A-GPC), or, more generally, chloro, fluoro or bromo-substituted tryptophans, or mono- or di-fatty acid substituted glycerol phosphocholines, or any derivative or functional equivalent thereof.

Accordingly, the present disclosure provides for a method of prevention or treatment of a subject in need thereof, particularly a subject having an inflammation-related disease, e.g. type 1 or type 2 Diabetes mellitus, autoimmune disease (such as an endocrine autoimmune disease), cardiovascular disease or inflammatory disease, comprising the step of administrating one or more of the above-mentioned agent(s).

In comparison to administration of autologous fecal matter, as described in WO2019168401, it was found that the method according to the present disclosure has improved therapeutic efficacy in autoimmune disease, is less laborious, easier to administer, easier to produce e.g. under certified Quality Management Systems (QMS) or under Good Manufacturing Practice (GMP), and/or easier to upscale.

The present disclosure, at least *a priori,* preferably does not aim to alter the intestinal microbiome, i.e. the gut microbiota composition or particularly the colon microbiota composition.

In the context of the present disclosure, the autoimmune disease can be any autoimmune disease, including systemic and localized (organ specific) autoimmune diseases, particularly an autoimmune disease chosen from the group consisting of endocrine autoimmune disease (e.g. Type 1 Diabetes mellitus, Hashimoto's disease, Graves's disease, or Addison's disease); skin autoimmune disease (e.g. Psoriasis or Vitiligo); rheumatoid autoimmune diseases (e.g. rheumatoid arthritis, Systemic lupus erythematosus, Vasculitis or Bechterew's disease), and gastrointestinal autoimmune disease (e.g. Celiac disease, Inflammatory Bowel Disease), Neurological diseases (Guillain Barre, CIDP and Multiple sclerosis) and Lung diseases (COPD/Asthma).

In the context of the present disclosure, the cardiovascular disease can be any cardiovascular disease, for example coronary artery disease (also known as coronary heart disease and ischemic heart disease), peripheral arterial disease, cerebrovascular disease (for example stroke or TIA, Transient Ischemic Attack), atherosclerosis, stenosis, renal artery stenosis, aortic disease, aortic aneurysm, cardiomyopathy, hypertensive heart disease, hypertension, heart failure, pulmonary heart disease, cardiac dysrhythmias, cardiovascular inflammation, inflammatory heart disease, endocarditis, inflammatory cardiomegaly, myocarditis, eosinophilic myocarditis, valvular heart disease, congenital heart disease, or rheumatic heart disease (optionally, also any of these diseases may be excluded from the present application).

Further, in the context of the present disclosure, the inflammatory disease can be any inflammatory disease, in particular cardiovascular inflammation, for example carditis, endocarditis, myocarditis, pericarditis, vasculitis, arteritis, phlebitis, or capillaritis, or for example inflammation of the gastrointestinal tract, e.g. esophagitis, gastritis, gastroenteritis, enteritis, colitis, enterocolitis, duodenitis, ileitis, caecitis, appendicitis, or proctitis. The inflammatory disease according to the present disclosure may further refer to hepatic inflammation, pulmonary inflammation, or skeletal inflammation. Alternatively, the inflammatory disease may refer to systemic inflammatory response syndrome (SIRS) or sepsis (optionally, also any of these diseases may be excluded from the present application).

### DIABETES MELLITUS

### Type 1 Diabetes mellitus

Type 1 Diabetes mellitus is a chronic endocrine autoimmune disease wherein the pancreas produces too little or no insulin. It is generally regarded as associated with progressive beta cell destruction, and linked to an increased morbidity and mortality risk compared to healthy subjects. As beta cell function can also deteriorate in Type 2 Diabetes mellitus, the present disclosure may also concern prevention and/or treatment of Type 2 Diabetes mellitus.

It was found that the agent(s) according to the present disclosure can be used to prevent and/or treat Type 1 Diabetes mellitus. Such treatment may also extend the honeymoon phase in Type 1 Diabetes mellitus, i.e. the period following diagnosis wherein the own pancreas is still able to produce a significant enough amount of insulin to limit exogenous insulin needs in the body and maintain blood glucose control. Extending this period can dramatically improve quality of life in patients. The treatment can also be applied to reduce severity of symptoms of Type 1 Diabetes mellitus, for example symptoms or complications related to impaired function of eye(s), kidney(s), nerves and/or brain.

More specifically, the treatment may inhibit decay of beta cell function and/or inhibit production of autoantibodies associated with Type 1 Diabetes mellitus, such as islet (beta) cell autoantibodies, autoantibodies to insulin, autoantibodies to GAD (GAD65), autoantibodies to the tyrosine phosphatases IA-2 and IA-2β, and/or autoantibodies to zinc transporter 8 (ZnT8).

The symptoms of Type 1 Diabetes mellitus may include polyuria, polydipsia, polyphagia, weight loss, fatigue, nausea, and blurred vision. The onset of symptomatic disease can be sudden. In this regard, it is not unusual that patients with Type 1 Diabetes mellitus suffer from diabetic ketoacidosis (DKA). The following diagnostic criteria can be applied for Type 1 and Type 2 Diabetes mellitus (American Diabetes Association, ADA):
- A fasting plasma glucose (FPG) level ≥126 mg/dL (7.0 mmol/L), or
- A 2-hour plasma glucose level ≥200 mg/dL (11.1 mmol/L) during a 75-g oral glucose tolerance test (OGTT), or
- A random plasma glucose ≥200 mg/dL (11.1 mmol/L) in a patient with classic symptoms of hyperglycemia or hyperglycemic crisis.

Additionally and/or alternatively, C-peptide response after a mixed meal test can be assessed, as described in the Example and/or as described by Lachin et al (2011 PLoS ONE Vol. 6(11) e26471).

Type 1 Diabetes mellitus and/or its preceding symptoms can be confirmed by the presence of one or more autoimmune markers which include islet (beta) cell autoantibodies, autoantibodies to insulin, autoantibodies to GAD (GAD65), autoantibodies to the tyrosine phosphatases IA-2 and IA-2β, and autoantibodies to zinc transporter 8 (ZnT8) as well as increased HbA1c and altered glucose tolerance.

### Type 2 Diabetes mellitus

Type 2 diabetes is a common metabolic condition that develops when the body fails to produce enough insulin or when insulin fails to work properly, which is referred to as insulin resistance. Insulin is the hormone that stimulates cells to uptake glucose from the blood to use for energy. When this is the case, cells are not instructed by insulin to take up glucose from the blood, meaning the blood sugar level rises (referred to as hyperglycemia).

People usually develop type 2 diabetes after the age of 40 years, although people of South Asian origin are at an increased risk of the condition and may develop diabetes from the age of 25 onwards. The condition is also becoming increasingly common among children and adolescents across all populations. Type 2 diabetes often develops as a result of overweight, obesity and lack of physical activity and diabetes prevalence is on the rise worldwide as these problems become more widespread. Type 2 diabetes accounts for approximately 90% of all diabetes cases (the other form being type 1 diabetes) and treatment approaches include lifestyle changes and the use of medication.

It was found that the agent(s) according to the present disclosure can be used to prevent and/or treat Type 2 Diabetes mellitus. The treatment can be applied to reduce severity of symptoms of Type 2 Diabetes mellitus, for example symptoms or complications related to polyuria, polydipsia. More specifically, the treatment may reduce the need of exogenous hormone supplementation.

### CARDIOVASCULAR DISEASES

### Coronary artery disease

Coronary artery disease is the most common of the cardiovascular diseases. It involves the reduction of blood flow to the heart muscle due to build-up of plaque (atherosclerosis) in the arteries of the heart. A common symptom is chest pain or discomfort which may travel into the shoulder, arm, back, neck, or jaw. In many cases, the first sign is a heart attack. Other complications include heart failure or an abnormal heartbeat.

Risk factors include high blood pressure, smoking, diabetes, lack of exercise, obesity, high blood cholesterol, poor diet, depression, and excessive alcohol. A number of tests may help with diagnoses including: electrocardiogram, cardiac stress testing, coronary computed tomographic angiography, and coronary angiogram, among others.

### INFLAMMATORY DISORDERS

A subject suffering from or at risk of developing inflammatory disease can be identified by methods known in the art, e.g., gross examination of tissue or detection of inflammation associated in tissue or blood. Symptoms of inflammation include pain, redness and swelling of the affected tissue.

### Systemic Inflammatory Response Syndrome (SIRS) and sepsis

Systemic inflammatory response syndrome (SIRS) is an exaggerated defense response of the body to for example an infection, trauma, surgery, acute inflammation, ischemia or reperfusion, or malignancy. It involves the release of acute-phase reactants which are direct mediators of widespread autonomic, endocrine, hematological and immunological alteration in the subject. Even though the purpose is defensive, the dysregulated cytokine storm has the potential to cause massive inflammatory cascade leading to reversible or irreversible end-organ dysfunction and even death. SIRS with a suspected source of infection is termed sepsis. Sepsis with one or more end-organ failure is called severe sepsis and with hemodynamic instability in spite of intravascular volume repletion is called septic shock.

SIRS may be diagnosed by the satisfaction of any two of the criteria below:
- Body temperature over 38 or under 36 degrees Celsius;
- Heart rate greater than 90 beats/minute;
- Respiratory rate greater than 20 breaths/minute or partial pressure of CO2 less than 32 mmHg;
- Leucocyte count greater than 12000 or less than 4000 /microliters or over 10% immature forms or bands.

Additionally or alternatively, the use according to the present disclosure may be for improving general health and/or reducing inflammation status, the latter preferably measured by higher erythrocyte sedimentation rate in comparison to healthy individuals (e.g. ESR values of at least 35, 40, 45, 50, 55, 60 mm/h) and/or decreased level of C-reactive protein in the blood (plasma), e.g. relative to not administering the composition according to the present disclosure. C-reactive protein may for example be measured after 1-12, 1-4, 2-8, 4-12 weeks or after 1-12 months or 1-12 years of administration of the composition of the present disclosure. C-reactive protein (CRP) is a protein made by the liver. CRP levels in the blood increase when there is a condition causing inflammation somewhere in the body. A CRP test measures the amount of CRP in the blood to detect inflammation status.

Additionally or alternatively, the use according to the present disclosure may be for inducing weight loss in a subject, or for reducing Body Mass Index (BMI).

### AUTOIMMUNE DISEASES

Autoimmune diseases are a class of diseases in which the immune system produces an inappropriate response against a subject's own cells, tissues and/or organs. This may result in inflammation, damage and loss of function. Common autoimmune diseases are Hashimoto hypothyroidism, Graves hyperthyroidism, Rheumatoid arthritis, Celiac disease, Asthma/COPD, Addison's disease, IBD (Crohn's disease and colitis ulcerosa), Systemic lupus erythematosus, Vasculitis, Guillain Barré and Chronic Inflammatory Demyelinating Polyneuropathy (CIDP), Multiple sclerosis, Psoriasis (arthritis), Vitiligo, Type 1 diabetes mellitus and Bechterew's disease.

The causes of autoimmune diseases are not clear. However, factors such as infections and genetic disposition may play a role in triggering autoimmune diseases. Autoimmune diseases are usually diagnosed using a combination of clinical history and blood tests (detecting amongst others autoantibodies, or markers of inflammation or organ function).

Although there is a wide range of treatment options, which depend on the stage and type of autoimmune disease, there is no definitive cure for autoimmune diseases.

Treatment strategies are generally directed to relieve symptoms, minimize organ or tissue damage and preserve organ function. For example, treatment options may include replacement of organ functions (such as administering insulin in Type 1 diabetes mellitus and thyroxine in Hashimoto's hypothyroidism), non-steroidal anti-inflammatory medications (NSAIDS), corticosteroid anti-inflammatory medications (such as prednisolone), TNFα inhibitors, immunosuppressive medications, or immunoglobulin replacement therapy.

### Endocrine autoimmune diseases

Among the various autoimmune diseases, autoimmune endocrine disorders are most common. The endocrine system comprises glands that produce hormones and deliver these directly into the circulatory system, as well as feedback loops to achieve homeostasis. The organs of the endocrine system can be affected by several autoimmune diseases, characterized by different impact and severity. Sometimes multiple organs are involved, such as in polyglandular autoimmune syndrome.

Among the different autoimmune endocrine diseases, Type 1 Diabetes mellitus, Hashimoto's disease, Graves' disease, and Addison's disease are especially frequent in clinical practice.

### Hashimoto's disease

Hashimoto's disease is an organ specific autoimmune disorder with the highest occurrence. It is also referred to as Hashimoto's thyroiditis, or chronic lymphocytic thyroiditis and is regarded as an autoimmune disease in which the thyroid gland is gradually destroyed. The causes of Hashimoto's disease are still unclear, although an inappropriate cell-mediated immune response and autoantibody production against the thyroid gland are generally thought to be involved. Both B (CD20+ and CD79 alpha+ ) cells are seen in the mononuclear lymphatic infiltrates (destruction of thyroid follicles and thyrocytes) as well as excessively stimulated T cells CD4+ (T helper type 2 Th2 cells lead to an excessive stimulation and production of B cells that produce antibodies against thyroid antigens, subsequently driving thyroiditis in the thyroid gland Marazuela et al J Clin Endocrinol Metab. 2006 Sep;91(9):3639-46).

Until thyroid hypofunction becomes apparent, an enlargement of the thyroid is typically the only symptom. However, the disease can progress into hypothyroidism, thereby often leading to symptoms including edema, weight gain, and fatiguability (susceptible to fatigue), sensitivity to cold and diarrhea, and physical findings such as dry skin, hoarseness, bradycardia, and/or a prolonged relaxation phase of the Achilles tendon reflex.

Hashimoto's disease may be confirmed by the presence of anti-thyroid peroxidase (TPO) antibodies and anti-thyroglobulin (Tg) antibodies in the patient's serum. Further, an elevated level of thyroid-stimulating hormone (TSH), and lowered levels of free **T4** (FT4), lowered levels of free T3, and/or elevated levels of anti-microsomal antibodies, in comparison to the average in healthy individuals, can help obtain positive diagnosis.

Hashimoto's disease is currently treated with thyroid hormone replacement agents such as levothyroxine (FT4 supplementation), triiodothyronine (T3 supplementation) or desiccated thyroid extract. The present inventors found that the agent(s) according to the present disclosure can be used to prevent and/or treat Hashimoto's disease, optionally in combination with thyroid hormone replacement agents as described above. The treatment according to the present disclosure can also be applied to reduce severity of symptoms of Hashimoto's disease, for example one or more symptoms or complications as described above.

### Graves' disease

Graves' disease is an autoimmune disease that affects the thyroid, and is the most common cause of hyperthyroidism. The disease can be characterized by the presence of autoantibodies in the serum that bind the thyrotropin receptor, i.e. the thyroid stimulating hormone (TSH) receptor. These anti-TSH receptor antibodies (TBII) overstimulate the thyroid gland which may lead to goiter and signs of thyrotoxicosis as well as involvement of the eye muscles in a subset of patients (Graves ophthalmopathy).

Among the symptoms are hyperthyroidism, goiter, and orbitopathy. Other major symptoms include weight loss (with increased appetite), fatigability, shortness of breath, hyperhidrosis, finger tremors, diarrhea, periodic paralysis (in male), and muscle weakness. With regard to Graves ophthalmopathy, patients may suffer from proptosis of the eyes, blurred vision and dry / red eyes (in rare cases it can lead to blindness). Two signs are truly specific of Graves' disease and not seen in other hyperthyroid conditions: exophthalmos and pretibial myxedema.

Graves' disease may be confirmed by low serum TSH level (sometimes not detectable) and/or elevations in free T3 and free **T4,** in comparison to health individuals. Patients may typically be positive for anti-TSH receptor antibodies (TBII) in their serum.

Current treatment of Graves' disease may involve administration of antithyroid drugs (block and replacement therapy), radioiodine (radioactive iodine I-131); and/or thyroidectomy (surgical excision of the gland). Usually, strumazol and methimazole (PTU) are prescribed followed by thyroid hormone replacement agents such as levothyroxine (FT4 supplementation), triiodothyronine (T3 supplementation) or desiccated thyroid extract.

Alternatively or in combination with the above-described treatment, the present inventors found that the agent(s) according to the present disclosure can be used to prevent and/or treat Graves' disease including ophthalmopathy. The treatment according to the present disclosure can also be applied to reduce severity of symptoms of Graves' disease, for example one or more symptoms or complications as described above.

### Addison's disease

Addison's disease is a chronic endocrine autoimmune disorder in which the adrenal glands do not produce sufficient steroid hormones. The disease is caused by destruction of the adrenal glands (both cortex and medulla produced hormones). The disease may be a manifestation of polyglandular autoimmune syndrome involving complications by other organ-specific autoimmune disorders (e.g., Type 1 Diabetes mellitus, Hashimoto's disease, Vitiligo).

Hyperpigmentation due to increased secretion of ACTH is a characteristic clinical sign of Graves' disease. Other symptoms include abdominal pain in the stomach region, orthostasis and weight loss.

Medical examination will typically determine if orthostasis, hypoglycemia, hyponatremia, hyperkalemia, and peripheral blood eosinophilia are present. To confirm Addison's disease, demonstration of low adrenal hormone levels even after stimulation (called the ACTH stimulation test or synacthen test) with synthetic pituitary ACTH hormone tetracosactide is generally performed for the diagnosis.

Treatment generally involves replacement therapy with oral hydrocortisone and/or mineralocorticoids like fludrocortisone (if the adrenal medulla is also involved). The present inventors found that the agent(s) according to the present disclosure can be used to prevent and/or treat Addison's disease, optionally in addition to treatment with hydrocortisone. The treatment according to the present disclosure can also be applied to reduce severity of symptoms of Addison's disease, for example one or more symptoms or complications as described above.

### Skin autoimmune disease

### Psoriasis (arthritis)

Psoriasis is a chronic autoimmune disease that leads to rapid production of skin cells. The underlying etiology is that T cells attack healthy skin cells, which causes the skin cell production process to go into overdrive. The new cells are pushed to the skin's surface, where they pile up. This results in the plaques and red inflamed areas of skin, which are most commonly associated with psoriasis. Subtypes of psoriasis include
(1) plaque psoriasis, which is the most frequently occurring type of psoriasis. It is characterized by red, inflamed patches that cover areas of the skin, typically on the elbows, knees, and scalp. These patches are often covered with whitish-silver scales or plaques;
(2) Guttate psoriasis, which is the form of psoriasis which is common in children and causes small pink spots, typically on the torso, arms, and legs;
(3) Pustular psoriasis, which is more common form of psoriasis in adults and causes white, pus-filled blisters and areas of red inflamed skin, typically on the hands or feet;
(4) Inverse psoriasis, which causes bright areas of red, shiny, inflamed skin. Patches of inverse psoriasis typically develop under armpits or breasts, in the groin, or around skinfolds;
(5) Erythrodermic psoriasis, which is a severe and rare type of psoriasis. This form often covers large sections of the body where the skin may appear sunburned. A person with this type of psoriasis may run a fever or become very ill, and this form of psoriasis can be life-threatening;
(6) Psoriatic arthritis with involvement of the joints.

Psoriasis symptoms are different among patients. Common symptoms include red patches of skin covered with thick, silvery scales, small scaling spots (commonly seen in children), dry, cracked skin that may bleed, itching, burning or soreness, thickened, pitted or ridged nails, and/or swollen and stiff joints. Most types of psoriasis can go through cycles, flaring for a few weeks or even months, then subsiding for a period or even going into remission. Psoriasis arthritis (or psoriatic arthritis) is a condition wherein swollen, sore joints of arthritis occur together with psoriasis.

For mild disease that involves only small areas of the body, topical treatments (applied on the skin), such as creams, lotions, and sprays, are generally prescribed. Occasionally, a local injection of steroids directly into a tough or resistant isolated psoriatic plaque may be helpful.

Tumor necrosis factor (TNF) antagonists (or anti TNFα therapy) have become first-line agents in the treatment of moderate-to-severe psoriasis or psoriatic arthritis. Examples include infliximab, etanercept, and adalimumab. Anti TNFα therapy has been found effective in treating both psoriasis and psoriatic arthritis and may also reduce the risk of cardiovascular events. The present inventors found that, in addition or alternatively, the agent(s) according to the present disclosure can be used to prevent and/or treat psoriasis and/or psoriatic arthritis. In addition, the treatment according to the present disclosure can also be applied to reduce severity of symptoms of psoriasis and psoriatic arthritis, for example one or more symptoms or complications as described above. Particularly the combined treatment with a TNF antagonist or anti-TNFα and treatment according to the present disclosure may be synergistic.

### Vitiligo

Vitiligo is a disease wherein white patches of skin appear on different parts of the body. It is generally thought that this is due to autoimmune processes that destroy the cells that make pigment (color) in the skin, i.e. melanocytes. Vitiligo can also occur in mucous membranes (such as inside the mouth and nose) and in the eye.

Recent studies reveal dysbiosis in the diversity of microbial community structure in the skin microbiome of vitiligo subjects. Although the individual specific microbiome signature is dominant over the vitiligo-specific microbiota, a clear decrease in taxonomic richness and evenness can be noted in lesional patches (Ganju et al Sci Rep. 2016 Jan 13;6:18761).

The white patches of vitiligo are more common in areas where the skin is exposed regularly to sunlight. The patches may be on the hands, feet, arms, face, and lips, but occasionally also on the armpits and groin, around the mouth, eyes, nostrils, navel, genitals, rectal areas. Further, people with vitiligo often have hair that turns gray early (e.g. before age 35).

Ultraviolet (UV) light can be used particularly in the early phase of vitiligo for diagnosis and to determine the effectiveness of UV treatment. Skin with vitiligo, when exposed to UV, typically will glow blue. In contrast, healthy skin will show no reaction.

Vitiligo can be classified into segmental vitiligo (SV) and non-segmental vitiligo (NSV), where NSV is the most common type of vitiligo.

In non-segmental vitiligo (NSV), there typically is symmetry in the location of the patches of depigmentation. In extreme cases, little pigmented skin remains, which is referred to as vitiligo universalis. NSV can initiate at any age, whereas segmental vitiligo is far more prevalent in teenage years.

Segmental vitiligo (SV) tends to affect areas of skin that are associated with dorsal roots from the spinal cord and is most often unilateral. It is much more stable/static in course. SV typically does not improve with UV light therapy, but surgical treatments such as cellular grafting can be effective.

There is no definitive cure for vitiligo but several treatment options are available, including ultraviolet light and/or creams. Topical preparations (i.e. creams) of immune suppressing medications including corticosteroids or glucocorticoids (such as clobetasol and/or betamethasone) and calcineurin inhibitors (such as tacrolimus and/or pimecrolimus) are considered to be first-line vitiligo treatments, while UV(B) therapy is considered a second-line treatment for vitiligo.

The present inventors found that, in addition or alternatively to the above-described treatment(s), the agent(s) according to the present disclosure can be used to prevent and/or treat vitiligo. Further, the treatment according to the present disclosure can also be applied to reduce severity of symptoms of vitiligo, for example one or more symptoms or complications as described above.

### Rheumatoid disorder

### Rheumatoid arthritis

Rheumatoid arthritis (RA) can be seen as an autoimmune disease in which the immune system attacks the joints. This leads to inflammation that causes the tissue that lines the inside of joints (the synovium) to thicken, resulting in painful joints.

If not treated, RA can damage cartilage, the elastic tissue that covers the ends of bones in a joint, and even the bones themselves. Eventually, there can be loss of cartilage, joints can become loose, unstable, painful and lose their mobility, or even deform. Unfortunately, joint damage generally cannot be reversed, and therefore early diagnosis and treatment is recommended to control RA.

RA most commonly occurs in the joints of the hands, feet, wrists, elbows, knees and ankles. RA can also affect body systems, such as the cardiovascular or respiratory systems, and is then called systemic RA. In the early stages, people with RA may experience tenderness and pain in the joints.

Symptoms of RA include stiffness and joint pain, specifically small joints (wrists, certain joints of the hands and feet), and typically for six weeks or longer. Along with pain, many people experience fatigue, loss of appetite and a mild fever.

No single test can definitely confirm RA, but blood tests can be performed which measure inflammation levels and look for biomarkers such as antibodies that are linked with RA.

A high erythrocyte sedimentation rate and a high C-reactive protein (CRP) level, in comparison to healthy individuals, are biomarkers of inflammation. A high ESR or high CRP is not specific to RA, but when combined with the presence of RA-related antibodies, can confirm RA diagnosis.

Rheumatoid factor (RF) is an antibody found in the majority of people with RA. Because RF can occur in other inflammatory diseases, it is not a definitive sign of having RA. However, a different antibody - anti-cyclic citrullinated peptide (anti-CCP) - occurs primarily in RA patients. That makes a positive anti-CCP test a stronger indication of RA. In addition, an X-ray, ultrasound or magnetic resonance imaging scan can be performed to look for joint damage, such as erosions and narrowing of joint space.

With respect to treatment, nonsteroidal anti-inflammatory drugs (NSAIDs) are generally prescribed, which can ease arthritis pain and inflammation. Examples of NSAIDs include ibuprofen, ketoprofen and naproxen sodium. Further, corticosteroids, including prednisone, prednisolone and methyprednisolone, can be administered as anti-inflammatory medications.

DMARDs, i.e. disease-modifying antirheumatic drugs, may be used to slow down the progression of the disease. DMARDs include methotrexate, hydroxycholorquine, sulfasalazine, leflunomide, cyclophosphamide and azathioprine. A subcategory of DMARDs is known as "JAK inhibitors" which block the Janus kinase, or JAK, pathways. An example is Tofacitinib.

Biologicals may work more quickly than traditional DMARDs, and are injected or given by infusion. In many people with RA, a biological can slow, modify or stop the disease. Particularly preferred are tumor necrosis factor (TNF) antagonists (anti TNFα therapy).

The present inventors found that, in addition or alternatively to the above-described treatment(s), the agent(s) according to the present disclosure can be used to prevent and/or treat Rheumatoid arthritis and/or one or more of its symptoms as described above. The combined treatment according to the present disclosure with a TNF antagonist or anti-TNFα may be synergistic.

### Bechterew's disease

Bechterew's disease (or Ankylosing Spondylitis) is a chronic autoimmune rheumatoid disorder involving particularly the axial skeleton. Typically, it presents in male adults of 20-30 years of age.

The most serious symptoms are neck and lower back pain. A typical symptom is nocturnal pain, as well as inflammation of the sacroiliac joint. In a some patients, bony deformities of the spine can occur, which may result in motion restriction. Apart from these spinal complaints, inflammation of peripheral joints is common.

In order to diagnose Bechterew's disease, examination of the vertebral column is performed to assess restrictions in cervical and lumbar spine mobility. A Schober test can be helpful in estimating the amount of lumbar forward flexion restriction. The diagnosis could be confirmed by discovery of HLA-B27 antigens in patient's blood.

Treatment options include administration of NSAID, sulfasalazine, methotrexate, leflunomide, corticosteroid, TNFα inhibitor(s). The present inventors found that, in addition or alternatively to the above-described treatment(s), the agent(s) according to the present disclosure can be used to prevent and/or treat Bechterew's disease and/or one or more of its symptoms as described above. Particularly the combination of treatment according to the present disclosure with a TNF antagonist or anti-TNFα may be synergistic.

### Systemic lupus erythematosus

Systemic lupus erythematosus (SLE), also known simply as lupus, is an autoimmune disease in which the body's immune system mistakenly attacks healthy tissue in many parts of the body. Symptoms vary between people and may be mild to severe. SLE significantly increases the risk of cardiovascular disease with this being the most common cause of death. With modern treatment about 80% of those affected survive more than 15 years after diagnosis. Common symptoms include painful and swollen joints, fever, chest pain, hair loss, mouth ulcers, swollen lymph nodes, feeling tired, and a red rash which is most commonly on the face. Often there are periods of illness, called flares, and periods of remission during which there are few symptoms. There is no cure for SLE. Treatments may include NSAIDs, corticosteroids, immunosuppressants, hydroxychloroquine, and methotrexate. Although corticosteroids are rapidly effective, long term use results in side effects. The present inventors found that, in addition or alternatively to the above-described treatment(s), the agent(s) according to the present disclosure can be used to prevent and/or treat SLE disease and/or one or more of its symptoms as described above.

### Vasculitis

Vasculitis is seen ranging from large to small vessels that are inflamed. Large vessel vasculitis diseases are Giant cell arteritis or arteriitis temporalis), Takayasu's disease (Takayasu arteritis). Medium large vessel vasculitis diseases are Polyarteritis Nodosa (PAN) and Kawasaki's disease. Small vessel vasculitis diseases comprise of Microscopic polyangiitis, GPA (Granulomatosis with PolyAngiitis also known as Wegener's disease), EGPA (Eosinophilic Granulomatosis with PolyAngiitis, also known as Churg-Strauss Syndrome), Henoch-Schönlein syndrome, Anti-GBM ( Goodpasture's syndrome) and Cryoglobulinemia-associated vasculitis. The present inventors found that the agent(s) according to the present disclosure can be used to prevent and/or treat vasculitis and/or one or more of its symptoms as described above.

### Gastrointestinal autoimmune disease

### Celiac disease

Celiac disease (or coeliac disease) is an autoimmune disorder where the ingestion of gluten leads to damage of the small intestinal epithelial cells. It may typically occur in genetically predisposed people and in combination with type 1 diabetes. Celiac disease and Type 1 Diabetes mellitus may have similar pathogenesis wherein heritable genetic factors as well as dietary and microbial exposures may play a role, particularly in early life (see e.g. Verdu and Danska Nature Immunology | VOL 19 | JULY 2018 | 685-695).

When people with celiac disease eat gluten (a protein found in wheat, rye and barley), their body initiates an immune response that attacks the small intestine, leading to damage of the villi (small fingerlike projections that line the small intestine). When the villi get damaged, nutrients cannot be absorbed properly by the intestine. Symptoms are abdominal cramps, malnutrition and osteoporosis.

There are several serologic (blood) tests available that screen for celiac disease antibodies, but the most commonly used is a tTG-IgA test. For this test to work, the patient must be consuming gluten. In addition, diagnosis for Celiac disease can be reached by an endoscopic biopsy. A biopsy is then taken of the small intestine, which can subsequently be analyzed to see if there is any damage consistent with celiac disease. The diagnosis may be confirmed when improvement is seen while on a gluten-free diet.

Currently, the only treatment for celiac disease is a strict gluten-free diet. People living gluten-free must avoid foods with wheat, rye and barley, for example bread and beer. Ingesting small amounts of gluten can trigger small intestine damage. The present inventors found that, in addition or alternatively to the above-described treatment(s), the agent(s) according to the present disclosure can be used to prevent and/or treat Celiac disease and/or one or more of its symptoms as described above.

### Inflammatory Bowel Disease

Inflammatory bowel disease (IBD) is a term for two conditions (Crohn's disease and colitis ulcerosa) that are characterized by chronic inflammation of the gastrointestinal (GI) tract. IBD is thought to be caused by a dysregulated immune response. Symptoms of IBD include persistent diarrhea, abdominal pain, rectal bleeding/bloody stools, weight loss, and fatigue. In IBD, the immune system responds incorrectly to environmental triggers, which causes inflammation of the gastrointestinal tract. There also appears to be a genetic component-someone with a family history of IBD is more likely to develop this inappropriate immune response.

IBD is diagnosed using a combination of endoscopy (for Crohn's disease) or colonoscopy (for ulcerative colitis) and imaging studies, such as contrast radiography, magnetic resonance imaging (MRI), or computed tomography (CT).

Several types of medications may be used to treat IBD: aminosalicylates, corticosteroids (such as prednisone), immunomodulators, and the newest class approved for IBD-the "biologics", such as anti-TNFalpha. Several vaccinations for patients with IBD are recommended to prevent infections. Severe IBD may require surgery to remove damaged portions of the gastrointestinal tract, but advances in treatment with medications mean that surgery is less common than it was a few decades ago. The present inventors found that, in addition or alternatively to the above-described treatment(s), the agent(s) according to the present disclosure can be used to prevent and/or treat IBD and/or reduce severity of one or more of its symptoms as described above.

### Neurological diseases

### Guillain barre

Guillain-Barré syndrome (GBS) is a rapid-onset muscle weakness caused by the immune system damaging the peripheral nervous system (acute polyneuropathy). The initial symptoms are typically changes in sensation or pain along with muscle weakness, beginning in the feet and hands, often spreading to the arms and upper body, with both sides being involved. The symptoms may develop over hours to a few weeks. During the acute phase, the disorder can be life-threatening, with about 15 percent of people developing weakness of the breathing muscles and, therefore, requiring mechanical ventilation.

Although the cause is unknown, the underlying mechanism involves an autoimmune disorder in which the body's immune system mistakenly attacks the peripheral nerves and damages their myelin insulation. Sometimes this immune dysfunction is triggered by an infection or, less commonly by surgery and rarely by vaccination. Diagnosis usually made based on the signs and symptoms, through the exclusion of alternative causes, and supported by tests such as nerve conduction studies and examination of the cerebrospinal fluid. There are a number of subtypes based on the areas of weakness, results of nerve conduction studies and the presence of certain antibodies. Treatment with intravenous immunoglobulins or plasmapheresis, together with supportive care, will lead to good recovery in the majority of people. Recovery may take weeks to years, with about a third having some permanent weakness. The present inventors found that, in addition or alternatively to the above-described treatment(s), the agent(s) according to the present disclosure can be used to prevent and/or treat GBS and/or reduce severity of one or more of its symptoms as described above.

### CDIP

Chronic inflammatory demyelinating polyneuropathy (CDIP) is an acquired immune-mediated inflammatory disorder of the peripheral nervous system. The disorder is sometimes called chronic relapsing polyneuropathy (CRP) or chronic inflammatory demyelinating polyradiculoneuropathy (because it involves the nerve roots). CIDP is closely related to Guillain-Barré syndrome and it is considered the chronic counterpart of that acute disease. The present inventors found that the agent(s) according to the present disclosure can be used to prevent and/or treat CDIP and/or reduce severity of one or more of its symptoms as described above.

### Multiple sclerosis

Multiple sclerosis (MS) is a demyelinating disease in which the insulating covers of nerve cells in the brain and spinal cord are damaged. This damage disrupts the ability of parts of the nervous system to transmit signals, resulting in a range of signs and symptoms, including physical, mental, and sometimes psychiatric problems Specific symptoms can include double vision, blindness in one eye, muscle weakness and trouble with sensation or coordination. MS takes several forms, with new symptoms either occurring in isolated attacks (relapsing forms) or building up over time (progressive forms). Between attacks, symptoms may disappear completely; however, permanent neurological problems often remain, especially with the advancement of the disease. While the cause is unclear, the underlying mechanism is thought to be either destruction by the immune system or failure of the myelin-producing cells. Proposed causes for this include genetics and environmental factors such as being triggered by a viral infection. MS is usually diagnosed based on the presenting signs and symptoms and the results of supporting medical tests. There is no known cure for multiple sclerosis. Treatments attempt to improve function after an attack and prevent new attacks. The present inventors found that the agent(s) according to the present disclosure can be used to prevent and/or treat MS and/or reduce severity of one or more of its symptoms as described above.

### Asthma and COPD

In the context of the present disclosure, also the prevention and/or treatment of asthma is foreseen, in view of autoimmune mechanisms which might be operating in asthma as well.

Asthma is a common chronic inflammatory disease of the airways of the lungs. It can be characterized by reversible airflow obstruction and bronchospasm. Symptoms include episodes of coughing, wheezing, chest tightness, and shortness of breath.

There is currently no definitive diagnostic test for asthma, and diagnosis is typically based on the pattern of symptoms and response to therapy over time. A diagnosis of asthma can be made if there is a history of recurrent wheezing, coughing or difficulty breathing and these symptoms occur or worsen due to exercise, viral infections, allergens and/or air pollution; also FEV1 test upon bronchodilators are done to study effect on lung function.

An effective treatment for asthma is identifying what triggers the disease, such as cigarette smoke, pets, or aspirin, and eliminating exposure to these triggers. In addition, bronchodilators are often recommended. In the case of mild but persistent disease, low-dose inhaled corticosteroids or alternatively, leukotriene antagonists or mast cell stabilizers can be applied. For serious asthma, i.e. patients who have daily attacks, inhaled corticosteroids, i.e. in a higher dose, can be used.

The present inventors found that, in addition or alternatively to the above-described treatment(s), the agent(s) according to the present disclosure can be used to prevent and/or treat asthma and/or one or more of its symptoms as described above.

The effectiveness of the treatment according to the present disclosure confirms a link between intestinal microbiome composition and risk of developing asthma which has been postulated by Korpela et al (Nat Commun. 2016 Jan 26;7:10410).

(Lung) emphysema is one of the diseases that comprises COPD (chronic obstructive pulmonary disease). Emphysema involves gradual damage of lung tissue, specifically thinning and destruction of the alveoli or air sacs. The agent(s) according to the present disclosure can be used to prevent and/or treat COPD, or specifically (lung) emphysema, and/or one or more of its symptoms as described above.

### Other conditions

The present disclosure may also be used in the context of preventing and/or treating other autoimmune diseases, particularly including autoimmune hepatitis, Diabetes mellitus Type 1a and/or 1b, polyglandular autoimmune syndrome, Myasthenia gravis, Pernicious anemia, Primary biliary cirrhosis, Sclerosing cholangitis, Antiphospholipid antibody syndromes, Dermatomyositis, Mixed connective tissue disease, Polymyalgia rheumatica, Polymyositis, Scleroderma and Sjögren's syndrome. However, it is also envisaged that any of the above mentioned diseases is excluded from the present disclosure.

Additionally, the agent(s) according to the present disclosure may be used to prevent and/or treat an allergy, also known as allergic diseases, which are conditions caused by hypersensitivity of the immune system to typically harmless substances in the environment. Common allergies include hay fever (plant pollen allergy) and food allergy (relating e.g. to cow's milk, soy, eggs, wheat, peanuts, tree nuts, fish, and/or shellfish).

The present disclosure may also allow for the prevention and/or treatment of the following diseases, but optionally these diseases are excluded from the scope of the present disclosure: gastrointestinal disorders, Clostridium difficile infection, Morbus Crohn (Crohn's disease), ulcerative colitis or Inflammatory Bowel Disease (IBD), and/or Irritable bowel syndrome (IBS). Alternatively and/or additionally, any of the following diseases may be excluded from the present disclosure: systemic and localized (organ specific) autoimmune diseases, endocrine autoimmune disease, Type 1 Diabetes mellitus, Type 2 Diabetes mellitus, Hashimoto's disease, Graves's disease, or Addison's disease, skin autoimmune disease, Psoriasis or Vitiligo, rheumatoid autoimmune diseases, rheumatoid arthritis, Bechterew's disease, and gastrointestinal autoimmune disease, Celiac disease, vasculitis, COPD, CIDP, MS, SLE, Guillain-Barré. The disease according to the present invention need not be inflammation-related.

### Treatment according to the present disclosure

The agent for use in the prevention or treatment of an autoimmune disease as described herein may be a *Desulfovibrio* species, wherein the *Desulfovibrio* species is preferably chosen from the group consisting of *Desulfovibrio piger* (ATCC 29098), *Desulfovibrio fairfieldensis* (ATCC700045), *Desulfovibrio desulfuricans* (Essex 6 ATCC 29577), D. *desulfuricans* (MB ATCC 27774), *Desulfovibrio indonensis* (NCIMB 13468), *Desulfovibrio alaskensis* (NCIMB 13491), *Desulfovibrio vietnamensis* (DSM 10520), *Desulfovibrio gigas* (DSM 1382), *Desulfovibrio intestinalis* (DSM 11275), *Desulfovibrio longreachensis* (ACM *3958), Desulfovibrio termitidis* (DSM 5308), *Desulfovibrio vulgaris subsp. vulgaris* (DSM 644), and *Desulfovibrio vulgaris subsp. oxamicus* (DSM 1925). Additionally or alternatively, the agent may be a *Bacteroides* species, preferably *Bacteroides stercoris,* or relative thereof such as a relative having at least 70, 80, 85, 90, 95, 96, 97, 98, 99, 99.5, 99.9% sequence identity with the 16S rDNA sequence of the type strain of *Bacteroides stercoris.*

Most preferably the *Desulfovibrio* species is *Desulfovibrio piger,* or a relative thereof having at least 70, 80, 85, 90, 95, 96, 97, 98, 99, 99.5, 99.9% sequence identity with the 16S rDNA sequence of *Desulfovibrio piger* (e.g. SEQ ID NO:1). Such cut-off value based on 16S rDNA similarity can define species with similar characteristics and/or functionality.

Preferably, an amount of at least 10⁴, 10⁶, 10⁶, 10⁷, 10⁸ *Desulfovibrio* cells may be used, e.g. in a composition wherein the *Desulfovibrio* species is comprised, for example per ml or per g of said composition. Alternatively or additionally, a total of between 10⁴ and 10¹⁶, 10⁴ and 10¹⁵, 10⁴ and 10¹⁴, 10⁴ and 10¹², 10⁶ and 10¹², preferably between 10⁸ and 10¹⁰, *Desulfovibrio* cells may preferably be used, e.g. per ml or per g composition wherein the *Desulfovibrio* species is comprised.

Alternatively or additionally, the *Desulfovibrio* cells may be viable, but it is also envisaged that (only) attenuated or dead cells are used, e.g. obtained after pasteurization, or for example obtained after incubation at 50-100, 60-80, 65-75, or 70 degrees Celsius, preferably for a period of at least 5, 10, 15, 20, 25, 30, 40, 50 minutes, or obtained exposure to UV or gamma irradiation, preferably for a period of at least 1, 5 , 10, 20 30 seconds, or 1, 5, 10, 15, 20 , 25, 30, 40, 50 minutes, or obtained after incubation with oxygen, e.g. gas comprising at least 15, 20, 25, 30, 35, 40, 45, 50, 60 ,70, 80, 90, 99, 100 vol. % oxygen, preferably for a period of at least 1, 5 , 10, 20 30 seconds, or 1, 5, 10, 15, 20 , 25, 30, 40, 50 minutes. Preferably, the *Desulfovibrio* species is the first, second, third, fourth, or fifth most dominant bacterial species in the composition, i.e. has the highest cell count in comparison to other bacterial species contained in the composition, or is at least in the top 5.

The *Desulfovibrio* species according to the present disclosure is preferably not comprised in fecal matter, or, if it is comprised in fecal matter (e.g. as an alternative to the above-mentioned composition), it is enriched, i.e. the number of *Desulfovibrio* cells is higher than in prior art fecal matter, for example *Desulfovibrio* cells have been added to the fecal matter, or the fecal matter has been exposed to conditions favoring growth of said *Desulfovibrio* species. If the *Desulfovibrio* species according to the present disclosure is comprised in fecal matter, preferably at least at least 10⁴, 10⁵, 2x10⁵, 3x10⁵, 4x10⁵, 5x10⁵, 6x10⁵, 7x10⁵, 8x10⁵, 9x10⁵, 10⁶, 2x10⁶, 3x10⁶, 4x10⁶, 5x10⁶, 6x10⁶, 7x10⁶, 8x10⁶, 9x10⁶, 10⁷, 2x10⁷, 3x10⁷, 4x10⁷, 5x10⁷, 6x10⁷, 7x10⁷, 8x10⁷, 9x10⁷, 10⁸, 10⁹, 10¹⁰, 10¹¹, 10¹², 10¹³ *Desulfovibrio* cells are comprised in said fecal matter, for example per ml or per g fecal matter. Preferably, the *Desulfovibrio* species is the first, second, third, fourth, or fifth most dominant bacterial species in the fecal matter, i.e. has the highest cell count in comparison to other bacterial species contained in the fecal matter, or is at least in the top 5.

The agent according to the present disclosure may additionally or alternatively be an amino acid substituted with one or more halogens, preferably one halogen, preferably a chloro, fluoro or bromo-substituted amino acid. There is a preference for an aromatic amino acid, optionally substituted with one or more halogens, preferably one halogen, e.g. on the 6-position, preferably a chloro, fluoro or bromo-substituted, e.g. on the 6 position, aromatic amino acid. There is a higher preference for tryptophan, tyrosine, or phenylalanine, optionally substituted with one or more halogens, preferably one halogen (e.g. on the 6 position), preferably a chloro, fluoro or bromo-substituted, e.g. on the 6 position, tryptophan, tyrosine, or phenylalanine, for example a a chloro, fluoro or bromo-substituted tryptophan. Even more preferred is a halogenated tryptophan, e.g. on the 6-position, preferably chlorotryptophan, fluorotryptophan or bromotryptophan. The highest preference is 6-bromotryptophan, or any derivative or functional equivalent of thereof. The agent may be used in an amount of at least 0.1, 0.2, 0.3, 0.4, 0.5, 0.6, 0.7, 0.8, 0.9, 1, 2, 3, 4, 5, 6, 7, 8, 9, 10, 15, 20, 25, 30, 40, 50, 60, 70, 80, 90, 100, 200, 300, 400, 500, 600, 700, 800, 900 µg (microgram) for example per ml or per g of a composition wherein it is comprised. Alternatively or additionally, a total of between 0.1-10, 0.5-15, 1-20, 1-100, 5-100, 1-500, 50-750 µg (microgram) may preferably be used, e.g. per ml or per g composition wherein the agent(s) is comprised. Alternatively, the agent may be used in an amount of at least 0.1, 0.2, 0.3, 0.4, 0.5, 0.6, 0.7, 0.8, 0.9, 1, 2, 3, 4, 5, 6, 7, 8, 9, 10, 15, 20, 25, 30, 40, 50, 60, 70, 80, 90, 100, 200, 300, 400, 500, 600, 700, 800, 900 mg, for example per ml or per g of a composition wherein it is comprised. Alternatively or additionally, a total of between 0.1-10, 0.5-15, 1-20, 1-100, 5-100, 1-500, 50-750 µg (microgram) may preferably be used, e.g. per ml or per g composition wherein the agent is comprised. Alternatively or additionally, a total of between 0.1-10, 0.5-15, 1-20, 1-100, 5-100, 1-500, 50-750 mg may preferably be used, e.g. per ml or per g composition wherein the agent(s) is comprised. The administration may be oral administration, subcutaneous or intravenous administration. The total amount to be administered may depend on body weight of the subject to be treated, and can be determined by the skilled person. For example, a single dose may comprise between 10 microgram and 100 g, or between 10 mg and 50 g or between 50 mg and 10 g or between 100 mg and 5 g. The dose may be administered periodically as described elsewhere herein. Additionally or alternatively, the agent is preferably not comprised in fecal matter, or, if it is comprised in fecal matter (e.g. as an alternative to the above-mentioned composition), it is enriched, i.e. the amount of said agent is higher than in prior art fecal matter, i.e. at least 1, 2, 3, 4, 5, 6, 7, 8, 9, 10 wt% higher in comparison to any fecal matter or fecal microbiota transplant to which the agent has not been added. According to the present invention, said agent can be added to the fecal matter. If the agent is comprised in fecal matter, preferably at least 0.1, 0.2, 0.3, 0.4, 0.5, 0.6, 0.7, 0.8, 0.9, 1, 2, 3, 4, 5, 6, 7, 8, 9, 10, 15, 20, 25, 30, 40, 50, 60, 70, 80, 90, 100, 200, 300, 400, 500, 600, 700, 800, 900 ng, or at least 0.1, 0.2, 0.3, 0.4, 0.5, 0.6, 0.7, 0.8, 0.9, 1, 2, 3, 4, 5, 6, 7, 8, 9, 10, 15, 20, 25, 30, 40, 50, 60, 70, 80, 90, 100, 200, 300, 400, 500, 600, 700, 800, 900 µg (microgram), or at least 0.1, 0.2, 0.3, 0.4, 0.5, 0.6, 0.7, 0.8, 0.9, 1, 2, 3, 4, 5, 6, 7, 8, 9, 10, 15, 20, 25, 30, 40, 50, 60, 70, 80, 90, 100 mg of the agent is comprised in said fecal matter, for example per ml or per g fecal matter. Preferably, the agent is the first, second, third, fourth, or fifth most dominant metabolite in the fecal matter, i.e. has the highest weight amount in comparison to other metabolites contained in the fecal matter, or is at least in the top 10, or top 5.

Additionally or alternatively, the agent according to the present disclosure may be a mono or di fatty acid substituted glycerol phosphocholine (GPC), preferably wherein the fatty acid(s) are (independently) saturated or (mono or poly) unsaturated fatty acids.

There is a preference for unsaturated fatty acids such as myristoleic acid, palmitoleic acid, sapienic acid, oleic acid, elaidic acid, vaccenic acid, linoleic acid, linoelaidic acid, α-linolenic acid, arachidonic acid, eicosapentaenoic acid, erucic acid, docosahexaenoic acid. There is a higher preference for a substituted glycerol phosphocholine (GPC) that contains one or more of myristoleic acid and arachidonic acid. There is a higher preference and good results have been obtained with 1-myristoyl-2-arachidonoyl-glycero-phosphocholine (MA-GPC) and 1-arachidonoyl-glycero-phosphocholine (A-GPC), or any derivative or functional equivalent of these. The agent may be used in an amount of at least 0.1, 0.2, 0.3, 0.4, 0.5, 0.6, 0.7, 0.8, 0.9, 1, 2, 3, 4, 5, 6, 7, 8, 9, 10, 15, 20, 25, 30, 40, 50, 60, 70, 80, 90, 100, 200, 300, 400, 500, 600, 700, 800, 900 µg (microgram) for example per ml or per g of a composition wherein it is comprised. Alternatively or additionally, a total of between 0.1-10, 0.5-15, 1-20, 1-100, 5-100, 1-500, 50-750 µg (microgram) may preferably be used, e.g. per ml or per g composition wherein the agent(s) is comprised. Alternatively, the agent may be used in an amount of at least 0.1, 0.2, 0.3, 0.4, 0.5, 0.6, 0.7, 0.8, 0.9, 1, 2, 3, 4, 5, 6, 7, 8, 9, 10, 15, 20, 25, 30, 40, 50, 60, 70, 80, 90, 100, 200, 300, 400, 500, 600, 700, 800, 900 mg, for example per ml or per g of a composition wherein it is comprised. Alternatively or additionally, a total of between 0.1-10, 0.5-15, 1-20, 1-100, 5-100, 1-500, 50-750 µg (microgram) may preferably be used, e.g. per ml or per g composition wherein the agent is comprised. Alternatively or additionally, a total of between 0.1-10, 0.5-15, 1-20, 1-100, 5-100, 1-500, 50-750 mg may preferably be used, e.g. per ml or per g composition wherein the agent(s) is comprised. The administration may be oral administration, subcutaneous or intravenous administration. The total amount to be administered may depend on body weight of the subject to be treated, and can be determined by the skilled person. For example, a single dose may comprise between 10 microgram and 100 g, or between 10 mg and 50 g or between 50 mg and 10 g or between 100 mg and 5 g. Alternatively or additionally, administration may be such that a plasma concentration is achieved in the subject to be treated of preferably between 0.1-100, 0.2-50, 0.5-25, 0.5-20, 0.5-3, 1-15, 2-10, 2-5 nmol/ml or between 0.1-100, 0.2-50, 0.5-3, 0.5-25, 0.5-20, 1-15, 2-10, 2-5 µmol/ml, or 50% thereof in case of pediatric use. The dose may be administered periodically as described elsewhere herein. Additionally or alternatively, the agent is preferably not comprised in fecal matter, or, if it is comprised in fecal matter (e.g. as an alternative to the above-mentioned composition), it is enriched, i.e. the amount of said agent is higher than in prior art fecal matter, i.e. at least 1, 2, 3, 4, 5, 6, 7, 8, 9, 10 wt% higher in comparison to any fecal matter or fecal microbiota transplant to which the agent has not been added. According to the present invention, said agent can be added to the fecal matter. If the agent is comprised in fecal matter, preferably at least 0.1, 0.2, 0.3, 0.4, 0.5, 0.6, 0.7, 0.8, 0.9, 1, 2, 3, 4, 5, 6, 7, 8, 9, 10, 15, 20, 25, 30, 40, 50, 60, 70, 80, 90, 100, 200, 300, 400, 500, 600, 700, 800, 900 ng, or at least 0.1, 0.2, 0.3, 0.4, 0.5, 0.6, 0.7, 0.8, 0.9, 1, 2, 3, 4, 5, 6, 7, 8, 9, 10, 15, 20, 25, 30, 40, 50, 60, 70, 80, 90, 100, 200, 300, 400, 500, 600, 700, 800, 900 µg (microgram), or at least 0.1, 0.2, 0.3, 0.4, 0.5, 0.6, 0.7, 0.8, 0.9, 1, 2, 3, 4, 5, 6, 7, 8, 9, 10, 15, 20, 25, 30, 40, 50, 60, 70, 80, 90, 100 mg of the agent is comprised in said fecal matter, for example per ml or per g fecal matter. Preferably, the agent is the first, second, third, fourth, or fifth most dominant metabolite in the fecal matter, i.e. has the highest weight amount in comparison to other metabolites contained in the fecal matter, or is at least in the top 10, or top 5.

The agent(s) according to the present disclosure may be used in any combination in the prevention or treatment of an autoimmune disease as described herein. For example, the *Desulfovibrio* species may be combined with the chloro, fluoro or bromo-substituted tryptophan, e.g. 6-BT, and/or with the mono or di fatty acid substituted glycerol phosphocholine (GPC), e.g. with MA-GPC or with A-GPC. Alternatively, the chloro, fluoro or bromo-substituted tryptophan, e.g. 6-BT may be combined with the mono or di fatty acid substituted glycerol phosphocholine (GPC), e.g. with MA-GPC or with A-GPC. Or alternatively, MA-GPC may be combined with A-GPC.

The agent(s) according to the present disclosure may modulate the immune system - by resetting B cell clone function and regulatory T-cells which in turn may inhibit autoimmune response.

Preferably, the agent(s) according to the present disclosure is not comprised or combined with fecal matter, although the agent(s) may be derived therefrom. Additionally or alternatively, the agent(s) may be comprised in a composition comprising not more than 100, 90, 80, 70, 60, 50, 40, 30, 20, 10, 9, 8,7 ,6, 5, 4, 3, 2, 1 bacterial species.

Preferably, the agent(s) according to the present disclosure is comprised in a (pharmaceutical) composition in an amount of at least 0.1, 0.2, 0.3, 0.4, 0.5, 0.6, 0.7, 0.8, 0.9, 1, 2, 3, 4, 5, 6, 7, 8, 9, 10, 15, 20, 25, 30, 40, 50, 60, 70, 80, 90, 100, or between 0.1-10, 0.5-15, 1-20, 1-100 mg, between 5-50 mg, or between 1-25 mg, for example per g or per ml of the composition or carrier (e.g. an aqueous solution with 0.5-1.5 wt.% NaCl, e.g. 0.9 wt% NaCl, particularly in case of intraveneous administration).

The prevention and/or treatment according to the present disclosure may involve administering the agent(s) orally, or to the small intestine, preferably the duodenum, of said subject. In this regard, the fecal matter may be administered by enteral, preferably by oral, nasal or rectal administration, and/or by duodenal administration such as by means of a (naso)duodenal tube. Also foreseen is intravenous administration and subcutaneous administration (e.g. by match stick size (4x44mm) implant device subcutaneous delivery system for delivering and effective and consistent chronic dose for 3-6 months treatment (after which it is replaced again)), in particular for any one of the agent(s) substituted amino acid according to the present disclosure, and particularly for GPC according ot the present disclosure, e.g. MA-GPC and A-GPC.

The agent(s) according to the present disclosure may be administered to the gastrointestinal tract of the subject, preferably the small intestine, most preferably the duodenum, of the subject. The duodenum is the first section of the small intestine in most higher vertebrates, including mammals. The duodenum precedes the jejunum and ileum and is the shortest part of the small intestine. In humans, the duodenum is a hollow tube of 25-38 cm which connects the stomach to the distal duodenum. It begins with the duodenal bulb and ends at the suspensory muscle of duodenum. Although it is also possible to administer the agent(s) to the colon (or cecum) of the subject, administration to the colon (or cecum) of the subject is preferably not encompassed by the present disclosure.

The agent(s) according to the present disclosure may be combined with bacteria, i.e. microbiota or intestinal microbial cells, wherein the phylum may be one (or a combination) chosen from the group consisting of:
- *Firmicutes,* such as belonging to the genera *Eubacterium, Intestinimonas, Faecalibacterium, Christensenella, Anaerostipes, Agathobacter, Roseburia, Coprococcus, Clostridium, Subdoligranulum, Anaerotruncus, Flavinobacter, Ruminococcus, Butyricicoccus, Butyrovibrio, Sporobacter, Papilibacter, Oscillobacter, Oscillospora, Veilonella, Lactobacillus, Streptococcus;*
- *Proteobacteria* such as belonging to the genera *Escherichia* or *Enterobacter,*
- *Actinobacteria* such as belonging to the genera *Bifidobacterium* or *Colinsella;*
- *Bacteroidetes* such as belonging to the genera *Bacteroides, Prevotella* or *Alistipes;* and/or
- *Verrucomicrobia* such as belonging to the genus *Akkermansia.*

The agent(s) may also be combined with microbiota or intestinal microbial cells chosen from Eukarya, Archaea, and Bacteria, preferably chosen from the group of 1057 species as disclosed by Rajilić-Stojanović and de Vos (2014 FEMS Microbiol Rev. 38(5):996-1047).

A total of between 10⁴ and 10¹⁶, 10⁴ and 10¹⁵, 10⁴ and 10¹⁴, 10⁶ and 10¹², preferably between 10⁸ and 10¹⁰, of any of the above-mentioned microbial cells may preferably be used, e.g. per ml or per mg carrier.

The agent(s) may be applied in an effective amount, i.e. a quantity sufficient to achieve a desired therapeutic and/or prophylactic effect, e.g. an amount which results in the treatment and/or prevention of the respective condition. In the context of therapeutic or prophylactic applications, the amount to be administered to the subject may depend on the type and severity of the disease or condition and on the characteristics of the subject, such as general health, age, sex, body weight and tolerance to drugs. It may also depend on the degree, severity and type of disease or condition. The skilled artisan will be able to determine appropriate dosages depending on these and other factors.

In a preferred embodiment, the prevention and/or treatment according to the present disclosure involves at least 1, 2, 3, 4, 5, 6, 7, 8, 9, 10, and/or at most 10, 20, 30, 40, 50 separate administrations of the agent(s), preferably with intervals of at least 1, 2, 3, 4, 5, 6, 7, 8, 10, and/or at most 10, 20, 30, 40, 50 weeks between said separate administrations. The prevention and/or treatment may also involve daily, weekly, monthly administrations, such as once or twice within every 1, 2, 3, 4, 5, 6, 7, 8, 9, 10 days/weeks/months and/or may be during a period of at least 1, 2, 3, 4, 5, 6, 7, 8, 9, 10, 20, 30, 40, 50 weeks (or months or even years).

The agent(s) may be comprised in liquid medium and/or are preferably not combined with (e.g. in a composition comprising) solids having a diameter of more than 1, 2, 3, 4, 5, 10, 15, 20, 25, 50, 75, 100, 200, 400, 600, 800, or 1000 µm. The liquid medium may be an aqueous solution with 0.5-1.5 wt.% NaCl, e.g. 0.9 wt% NaCl. With the term "solids" is means discrete particles with at most 30, 20, 10, 5, 1 wt.% water.

It is further envisaged that the agent(s) according to the present disclosure is comprised in a composition, preferably a pharmaceutical composition, more preferably a liquid or solid dosage form, most preferably a capsule, a tablet, or a powder.

For oral administration, the agent(s) may be administered in solid dosage forms, such as capsules, tablets, and powders, or in liquid dosage forms, such as elixirs, syrups, and suspensions. Also, a carrier can be applied, such as activated carbon.

The agent(s) may be used as medicament and/or accompanied by a physiologically acceptable carrier which may be any inert carrier. For instance, non-limiting examples of suitable physiologically or pharmaceutically acceptable carriers include any well-known physiological or pharmaceutical carriers, buffers, diluents, and excipients. It will be appreciated that the choice for a suitable physiological carrier will depend upon the intended mode of administration of the composition as taught herein (e.g., oral) and the intended form of the composition (e.g. beverage, yogurt, powder, capsules, and the like). The skilled person knows how to select a physiologically acceptable carrier, which is suitable for or compatible with the compositions for use as taught herein.

It is particularly preferred that the agent(s) is comprised in and/or encapsulated by an (enteric) coating, preferable wherein said coating does not dissolute and/or disintegrate in the gastric environment of the subject. Such coating may help the agent(s) to reach the intended site for delivery, e.g. the duodenum, without suffering breakdown due to the acidic environment of the stomach. Preferred (enteric) coatings work by presenting a surface that is stable at the highly acidic pH found in the stomach, but breaking down more rapidly at a lower pH. For example, it will not dissolve in the gastric acids of the stomach (pH ~3), but it will dissolve in the alkaline (pH 7-9) environment present in the small intestine, or duodenum.

In an embodiment, the agent(s) according to the present disclosure may be combined with, or comprised in a composition comprising, a mucosal binding agent. The term 'mucosal binding agent' or 'mucosal binding polypeptide' as used herein refers to an agent or a polypeptide that is capable of attaching itself to the gut mucosal surfaces of the gut mucosal barrier of a mammal (e.g. human). A variety of mucosal binding polypeptides have been disclosed in the art. Non-limiting examples of mucosal binding polypeptide include bacterial toxin membrane binding subunits including such as the B subunit of cholera toxin, the B subunit of the E. *coli* heat-labile enterotoxin, *Bordetella pertussis* toxin subunits S2, S3, S4 and/or S5, the B fragment of Diphtheria toxin and the membrane binding subunits of Shiga toxin or Shiga-like toxins. Other suitable mucosal binding polypeptides include bacterial fimbriae proteins such as including E. coli fimbriae K88, K99, 987P, F41, FAIL, CFAIII ICES1, CS2 and/or CS3, CFAIIV ICS4, CS5 and/or CS6), P fimbriae, or the like. Other non-limiting examples of fimbriae include *Bordetella pertussis* filamentous hemagglutinin, vibrio cholerae toxin-coregulate pilus (TCP), Mannose-sensitive hemagglutinin (MSHA), fucose-sensitive hemagglutinin (PSHA), and the like. Still other mucosal-binding agents include viral attachment proteins including influenza and sendai virus hemagglutinins and animal lectins or lectin-like molecules including immunoglobulin molecules or fragments thereof, calcium-dependent (C-type) lectins, selectins, collectins or Helix pomatia hemagglutinin, plant lectins with mucosa-binding subunits include concanavalin A, wheat-germ agglutinin, phytohemagglutinin, abrin, ricin and the like.

In an embodiment, a composition comprising the agent(s) for use as taught herein may be in liquid form, e.g. a stabilized suspension comprising one or more of the agent(s), in solid form, e.g. a powder of lyophilized agent(s) as taught herein. For example, a cryoprotectant such as lactose, trehalose or glycogen may be employed.

Optionally, the agent(s) according to the present disclosure may be encapsulated in capsules such as gelatin capsules, possibly together with inactive ingredients and powder carriers, such as e.g. glucose, lactose, sucrose, mannitol, starch, cellulose or cellulose derivatives, magnesium stearate, stearic acid, sodium saccharin, talcum, magnesium carbonate and the like.

In an embodiment, the agent(s) according to the present disclosure may comprise one or more ingredients, which are suitable for promoting survival and/or viability and/or maintaining the and/or integrity of the agent(s) e.g. during storage and/or during exposure to bile and/or during passage through the gastrointestinal tract of a mammal (e.g. a human). Non-limiting examples of such ingredients include an enteric coating as described herein before, and/or controlled release agents allowing passage through the stomach. The skilled person knows how to select suitable ingredients for ensuring that the fecal matter reaches its intended destination, where it exerts its action.

In an embodiment, the compositions comprising the agent(s) for use as taught herein may further comprise ingredients selected from the group consisting of prebiotics, probiotics, carbohydrates, polypeptides, lipids, vitamins, minerals, medicinal agents, preservative agents, antibiotics, or any combination thereof.

In a particularly preferred embodiment, the agent(s) according to the present disclosure is combined with bacteria from the genus *Eubacterium, Intestinimonas, Bifidobacteria, Lactobacillales* and/or *Akkermansia,* preferably chosen from the group consisting of *Bifidobacterium animalis sub lactis or Bifidobacterium breve, Lactobacillus plantarum. Lactobacillus rhamnosus, Lactobacillus acidophilus, Eubacterium hallii, Intestinimonas butyriciproducens,* and/or *Akkermansia muciniphila.* A total of between 10⁴ and 10¹⁴, 10⁶ and 10¹², preferably between 10⁸ and 10¹⁰, of such bacterial cells may preferably be used (e.g. per ml or per mg). The above-mentioned combination may provide for synergistic effects. The agent(s) and the bacteria may be comprised in different compositions, or together within a single composition (such as in a capsule or other dosage form as described herein).

The agent(s) according to the present disclosure may additionally or alternatively be combined with hormone suppletion (thyroid, hydrocortisone, insulin etc), tumor necrosis factor alpha (TNFα) inhibitor, and/or DMARDs (rheumatoid arthritis) preferably chosen from the group consisting of infliximab, adalimumab, certolizumab pegol, and golimumab. The present inventors consider that treatment with a TNFα inhibitor may increase the response to treatment with agent(s) according to the present disclosure, and/or vice versa that treatment with agent(s) according to the present disclosure may increase the response to treatment with a TNFα inhibitor. Preferably, the TNFα inhibitor is administered in a different or the same composition as the agent(a) (such as in a capsule or other dosage form as described herein). The TNFα inhibitor may be administered at least (or at most) 1, 2, 3, 4 times weekly or daily and/or intravenously/orally in a dose of for example 1-10, 2-8, 3-7, 4-6, or 5 mg/kg.

It is further envisaged that the agent(s), particularly the *Desulfovibrio* species, for use according to the present disclosure is present in lyophilized and/or microencapsulated form, e.g. a capsule comprising said. Preferably, said agent(s), e.g. *Desulfovibrio* species, is present in solid, lyophilized or dried form (i.e. containing less than 20, 10, 5, 2, 1, wt.% water), for example in powder or granular form. For example, it may be present in microencapsulated form. The skilled person is capable of lyophilizing or microencapsulating the agent(s) based on well-known techniques, wherein oxygen-free conditions may be applied to preserve viability of any bacteria contained in the fecal matter.

The technique of microencapsulation is well-known in the art for preserving bacteria (e.g., as reviewed by Serna-Cock and Vallejo-Castillo, 2013. Afr J of Microbiol Res, 7(40): 4743-4753). For example, any of the preservation techniques and preservation systems taught by Serna-Cock and Vallejo-Castillo may be employed in the present disclosure.

Lyophilisation methods include, without limitation, slow, gradual freezing to -40°C before drying, rapid freezing by placing at -80°C before drying, or ultra rapid freezing by dripping cells with cryoprotectant in liquid nitrogen before drying. Cryoprotectants are often employed to protect compositions during lyophilisation and to enhance shelf-life. Without limitation, a cryoprotectant selected from the group consisting of sucrose, maltose, maltodextrin, trehalose, mannitol, sorbitol, inulin, glycerol, DMSO, ethylene glycol, propylene glycol, 2-methyl-2,4-pentanediol, polyethylene glycol, polyvinylpyrrolidone, polyvinyl alcohol, polyglycerol, skim milk powder, milk protein, whey protein, UHT milk, betaine, adonitol, sucrose, glucose, lactose or any combination thereof, may be employed.

Prebiotics such as starch and wheat bran may further be added to the agent(s) of the present disclosure, e.g. before lyophilisation to enhance the efficacy thereof. Addition of antioxidants such as riboflavin, riboflavin phosphate or a physiologically acceptable salt thereof, glutathione, ascorbate, glutathion and cysteine to the lyophilisation mixture may further enhance the viability of any bacteria contained.

The agent(s), particularly the *Desulfovibrio* species can be stored for long time (e.g. at least 10, 20, 40 52 weeks or at least 1, 2, 3 years) after addition of a cryoprotectant as disclosed herein, for example glycerol and/or freezing at -80 °C. In addition, or alternatively, freeze drying stabilizes the agent(s) over such period. Finally, one may also inoculate the *Desulfovibrio* species as described by de Vos (2013 Microb Biotechnol. 2013 Jul;6(4):316-25).

In an embodiment, the agent(s) for use as taught herein may be, or may be comprised in, a food or food supplement composition. Such food or food supplement composition may include a dairy product, more preferably a fermented dairy product, preferably a yogurt or a yogurt drink.

In an embodiment, the agent(s), or compositions comprising said, for use as taught herein may further comprise one or more ingredients, which further enhance the nutritional value and/or the therapeutic value of the fecal matter as taught herein. For instance, it may be advantageous to add one or more ingredients (e.g. nutritional ingredients, veterinary or medicinal agents etc.) selected from proteins, amino acids, enzymes, mineral salts, vitamins (e.g. thiamine HCl, riboflavin, pyridoxine HCl, niacin, inositol, choline chloride, calcium pantothenate, biotin, folic acid, ascorbic acid, vitamin B12, p-aminobenzoic acid, vitamin A acetate, vitamin K, vitamin D, vitamin E, and the like), sugars and complex carbohydrates (e.g. water-soluble and water-insoluble monosaccharides, disaccharides, and polysaccharides), medicinal compounds (e.g. antibiotics), antioxidants, trace element ingredients (e.g. compounds of cobalt, copper, manganese, iron, zinc, tin, nickel, chromium, molybdenum, iodine, chlorine, silicon, vanadium, selenium, calcium, magnesium, sodium and potassium and the like). The skilled person is familiar with methods and ingredients that are suitable to enhance the nutritional and/or therapeutic/medicinal value.

The present disclosure also provides for a method for predicting an autoimmune disease patient's response to therapy by agent(s) according to the present disclosure (or by autologous fecal matter), the method comprising
- measuring level of abundance in said patient's fecal microbiota of at least one bacterium chosen from the group consisting of *Bacteroides caccae* and *Coprococcus catus,*
wherein a measured level higher than a reference level indicates that the autoimmune disease patient is responsive to the therapy. Said reference level may for example be a level of between 50-150%, preferably between 75-125, more preferably between 90-120, 95-110, 98-105% of the level of abundance in healthy control subject(s) fecal microbiota of said at least one bacterium chosen from the group consisting of *Bacteroides caccae* and *Coprococcus catus.*

In the context of the present disclosure, the subject receiving the treatment is preferably an animal, more preferably a mammal, most preferably a human. As will be clear, the present treatment is preferably not performed as control or placebo treatment and/or within a clinical trial, i.e. a study in which participants are assigned to groups that either receive one or more intervention/treatment, one or more control or placebo intervention/treatment, or no intervention, so that researchers can evaluate the effects of the interventions on biomedical or health-related outcomes.

In this document and in its claims, the verb "to comprise" and its conjugations is used in its non-limiting sense to mean that items following the word are included, but items not specifically mentioned are not excluded. In addition, reference to an element by the indefinite article "a" or "an" does not exclude the possibility that more than one of the element is present, unless the context clearly requires that there be one and only one of the elements. The indefinite article "a" or "an" thus usually means "at least one".

"Sequence identity" can be determined by alignment of two peptide or two nucleotide sequences using alignment algorithms (when optimally aligned by for example the programs GAP or BESTFIT using default parameters). GAP uses the Needleman and Wunsch global alignment algorithm to align two sequences over their entire length, maximizing the number of matches and minimizes the number of gaps. Generally, the GAP default parameters are used, with a gap creation penalty = 50 (nucleotides) / 8 (proteins) and gap extension penalty = 3 (nucleotides) / 2 (proteins). For nucleotides the default scoring matrix used is nwsgapdna and for proteins the default scoring matrix is Blosum62 (Henikoff & Henikoff, 1992, PNAS 89, 915-919). Sequence alignments and scores for percentage sequence identity may be determined using computer programs, such as the GCG Wisconsin Package, Version 10.3, available from Accelrys Inc., 9685 Scranton Road, San Diego, CA 92121-3752 USA, or EmbossWin version 2.10.0 (using the program "needle"). Alternatively, percent similarity or identity may be determined by searching against databases, using algorithms such as FASTA, BLAST, etc. As an illustration, by a polynucleotide having a nucleotide sequence having at least, for example, 95% "identity" to a reference nucleotide sequence encoding a polypeptide of a certain sequence, it is intended that the nucleotide sequence of the polynucleotide is identical to the reference sequence except that the polynucleotide sequence may include up to five point mutations (which may be (conservative) substitutions, deletions and/or insertions) per each 100 nucleotides of the reference polypeptide sequence. In other words, to obtain a polynucleotide having a nucleotide sequence at least 95% identical to a reference nucleotide sequence, up to 5% of the nucleotides in the reference sequence may be deleted and/or substituted with another nucleotide, and/or a number of nucleotides up to 5% of the total nucleotides in the reference sequence may be inserted into the reference sequence. These mutations of the reference sequence may occur at the 5' or 3' terminal positions of the reference nucleotide sequence, or anywhere between those terminal positions, interspersed either individually among nucleotides in the reference sequence or in one or more contiguous groups within the reference sequence. Similarly, by a polypeptide having an amino acid sequence having at least, for example, 95% "identity" to a reference amino acid sequence of SEQ ID NO: 1 is intended that the amino acid sequence of the polypeptide is identical to the reference sequence except that the polypeptide sequence may include up to five amino acid alterations per each 100 amino acids of the reference amino acid of SEQ ID NO: 1. In other words, to obtain a polypeptide having an amino acid sequence at least 95% identical to a reference amino acid sequence, up to 5% of the amino acid residues in the reference sequence may be deleted or substituted with another amino acid, or a number of amino acids up to 5% of the total amino acid residues in the reference sequence may be inserted into the reference sequence. These alterations of the reference sequence may occur at the amino or carboxy terminal positions of the reference amino acid sequence or anywhere between those terminal positions, interspersed either individually among residues in the reference sequence or in one or more contiguous groups within the reference sequence. Sequence identity can be determined over the entire length of the sequence(s) to be considered.

### Sequence listing

16S rDNA sequence of Desulfovibrio piger (SEQ ID NO:1)
1 agagtttgat cctggctcag attgaacgct ggcggcgtgc ttaacacatg caagtcgtac
61 gcgaaaggga cttcggtccc gagtaaagtg gcgcacgggt gagtaacacg tggataatct
121 gcctctatga tggggataac agttggaaac gactgctaat accgaatacg ctcatgatga
181 actttgtgag gaaaggtggc ctctgcttgc aagctatcgc atagagatga gtccgcgtcc
241 cattagctag ttggtggggt aacggcctac caaggcaacg atgggtagcc gatctgagag
301 gatgatcggc cacactggaa ctgaaacacg gtccagactc ctacgggagg cagcagtggg
361 gaatattgcg caatgggcga aagcctgacg cagcgacgcc gcgtgaggga tgaaggtctt
421 cggatcgtaa acctctgtca gaagggaaga aactagggtg ttctaatcat catcctactg
481 acggtacctt caaaggaagc accggctaac tccgtgccag cagccgcggt aatacggagg
541 gtgcaagcgt taatcggaat cactgggcgt aaagcgcacg taggctgtta tgtaagtcag
601 gggtgaaagc ccacggctca accgtggaac tgcccttgat actgcacgac tcgaatccgg

661 gagagggtgg cggaattcca ggtgtaggag tgaaatccgt agatatctgg aggaacatca
721 gtggcgaagg cggccacctg gaccggtatt gacgctgagg tgcgaaagcg tggggagcaa
781 acaggattag ataccctggt agtccacgcc gtaaacgatg gatgctagat gtcgggatgt
841 atgtctcggt gtcgtagtta acgcgttaag catcccgcct ggggagtacg gtcgcaaggc
901 tgaaactcaa agaaattgac gggggcccgc acaagcggtg gagtatgtgg tttaattcga
961 tgcaacgcga agaaccttac ctaggtttga catctgggga accctcccga aatgaggggg
1021 tgcccttcgg ggagccccaa gacaggtgct gcatggctgt cgtcagctcg tgtcgtgaga
1081 tgttgggtta agtcccgcaa cgagcgcaac ccctatgcat agttgccagc aagtaaagtt
1141 gggcactcta tgcagactgc ccgggttaac cgggaggaag gtggggacga cgtcaagtca
1201 tcatggccct tacacctagg gctacacacg tactacaatg gcacgcacaa agggcagcga
1261 taccgtgagg tggagccaat cccaaaaaac gtgtcccagt ccggattgca gtctgcaact
1321 cgactgcatg aagtcggaat cgctagtaat tcgaggtcag catactcggg tgaatgcgtt
1381 cccgggcctt gtacacaccg cccgtcacac cacgaaagtc ggttttaccc gaagccggtg
1441 agccaactag caatagaggc agccgtctac ggtagggccg atgattgggg tgaagtcgta
1501 acaaggtagc cgtaggggaa cctgcggctg gatcacctcc tt

### Brief description of the Figures

Figure 1: Top 10 small intestinal microbiota with relative importances that best predicted treatment group allocation allocation (XGBoost predictive modeling algorithm). Percentages are scaled towards the largest which is set at 100%. The top 4 microbiota stand out with higher relative importances.

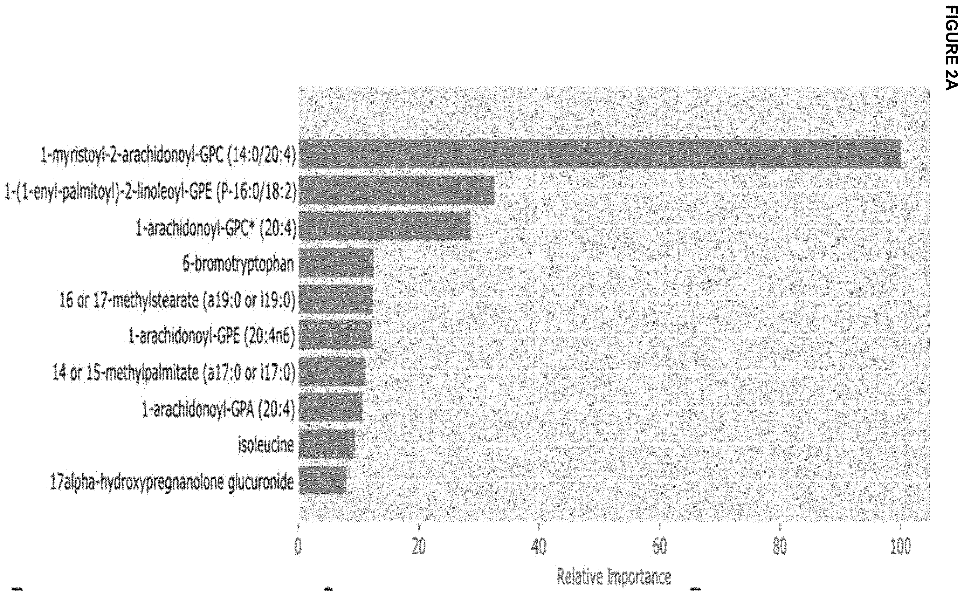

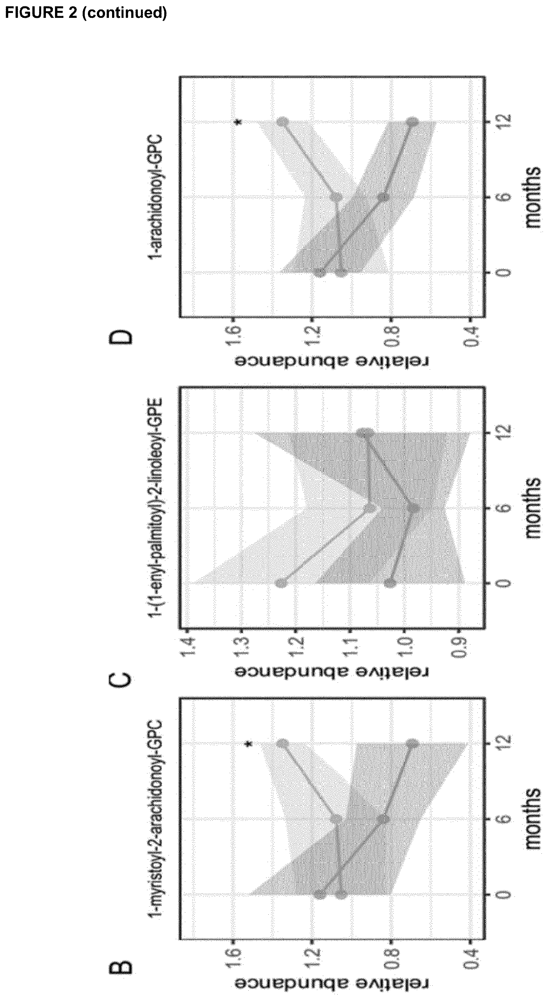

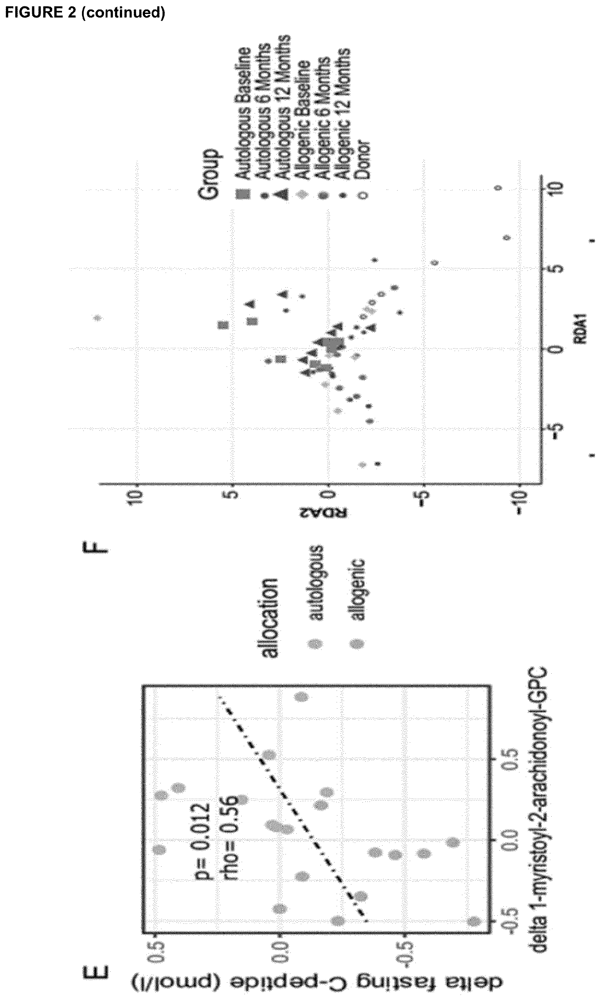

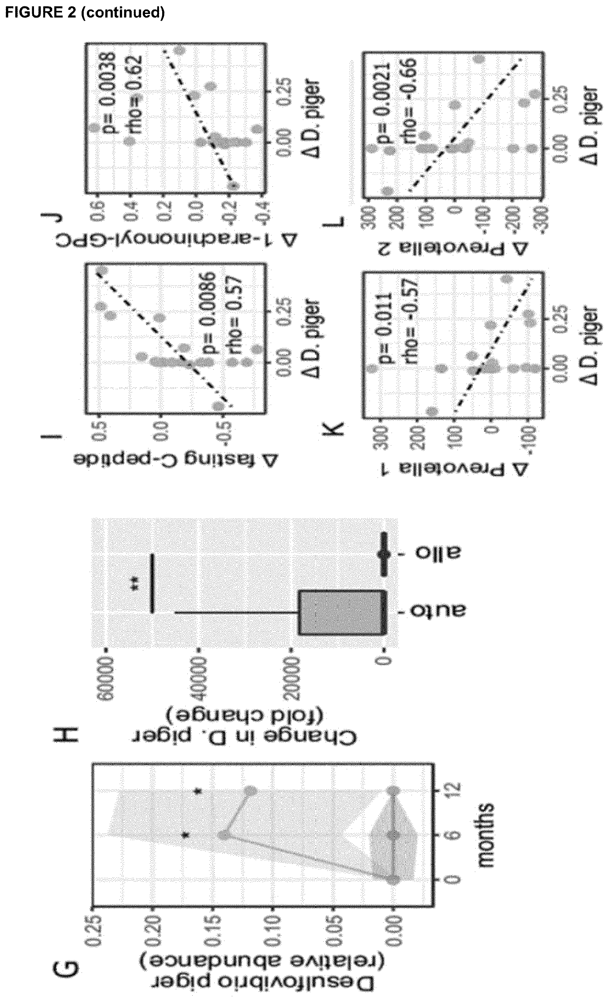

Figure 2: A: Top 10 metabolites that best predicted treatment group allocation allocation (XGBoost predictive modeling algorithm). Percentages are scaled towards the largest which is set at 100%. Top 3 metabolites stand out with higher relative importances in the analysis. B-D: Relative abundance of top 3 metabolites plotted against time for each treatment group (in each graph, the line that is predominantly the upper line represents the autologous FMT group; the line that is predominantly the bottom line represents the allogenic FMT group). Medians +- IQR are reported. P-values were calculated using Mann-Whitney U test between groups at 12 months. 1-myristoyl-2-arachidonoyl-GPC is different between groups at 12 months, p-value = 0.020. 1-arachidonoyl-GPC is different between groups at 12 months, p-value = 0.020. E: Spearman correlation between change in fasting C-peptide and change in 1-myristoyl-2-arachidonoyl-GPC. F: Abundance of fecal *D. piger* over time. P-values were calculated using Mann-Whitney U test. At 6 months p-value = 0.024, at 12 months p-value = 0.023. G: Fold change in D. *piger* between the groups (the predominant upper line represents the autologous FMT group). The delta p-value was calculated by doing Mann-Whitney U test on the delta's between 0 and 12 months of each group, p-value = 0.006. H: Spearman correlation plot of delta (0-12 months) fecal *D. piger* and delta (0-12 months) of fasting C-peptide. I: correlation plot of fecal *D. piger* and 1-arachidonoyl-GPC. J: correlation plot of fecal *D. piger* and small intestinal *Prevotella* 1 K: correlation plot of fecal *D. piger* and small intestinal *Prevotella 2.*
Figure 3: Predictive modeling output showing top 30 differentially changed fecal microbiota between treatment groups.

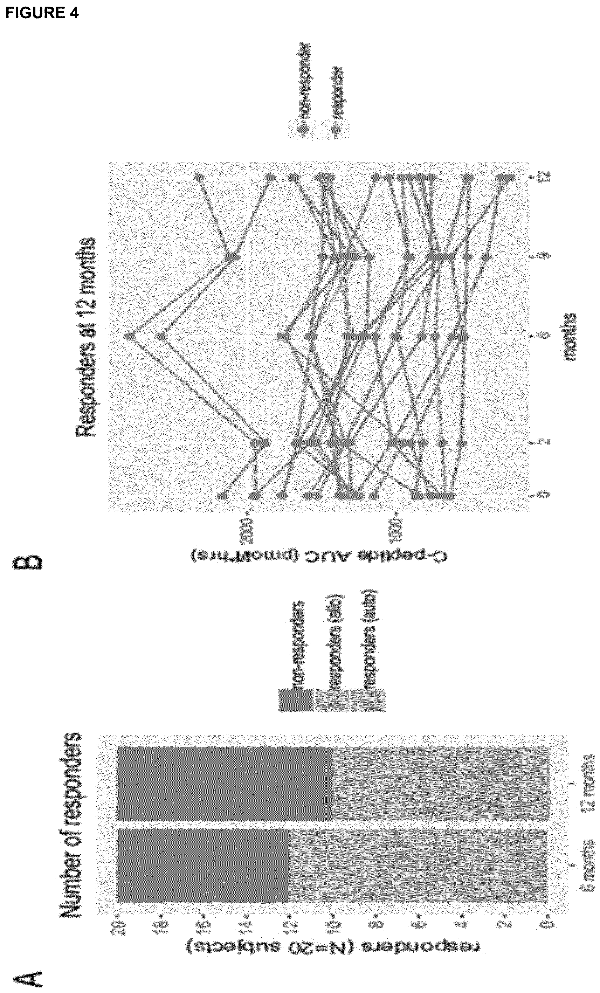

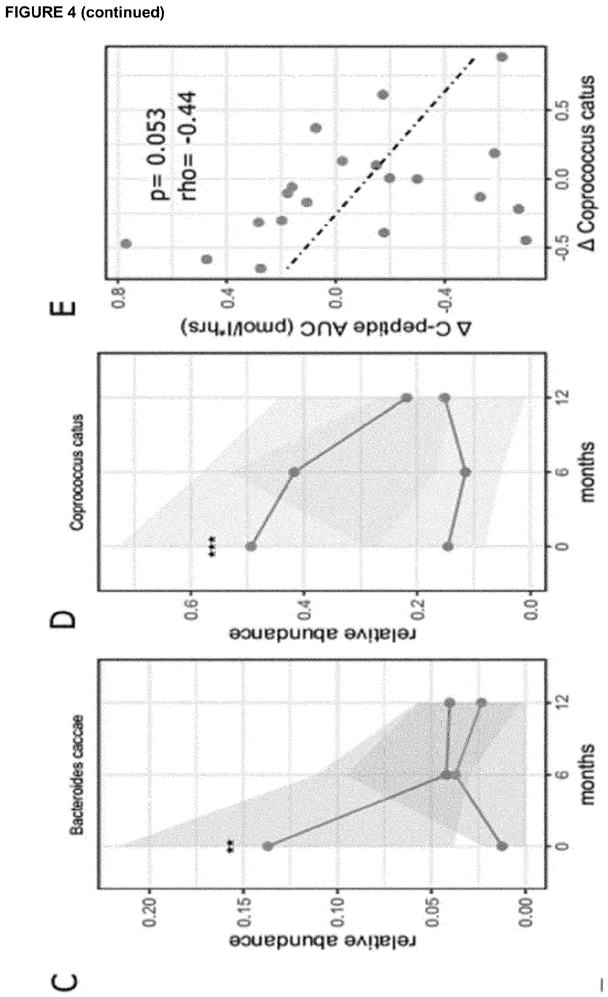

Figure 4: A: Shows the number of responders at 6 months and at 12 months and how many subjects were in each treatment group. Response was defined as <10% decline in C-peptide AUC compared to baseline. The 12 months responders were used for all analyses. B: Shows individual subject lines of C-peptide AUC over time. C and D: show the abundance of B. *caccae* and C. *catus* over time respectively. In both graphs, the upper line represent responders. P-values were calculated using Mann-Whitney U test between groups at each timepoint. For B. *caccae* at baseline the p-value = 0.0099, for C. *catus* at baseline the p-value = 0.00049. E: shows the correlation between delta C. *catus* (0-12 months) and delta C-peptide AUC (0-12 months). Spearman's rho (r) is shown and the p-value was calculated using Spearman's Rank.
Figure 5: Predictive modeling output showing top 30 differentially changed fecal microbiota between treatment groups.

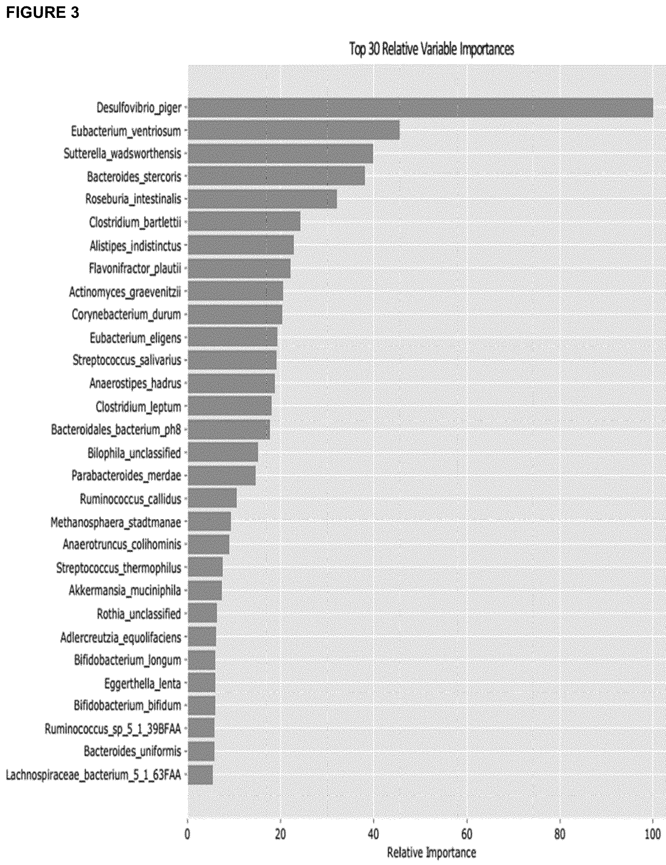

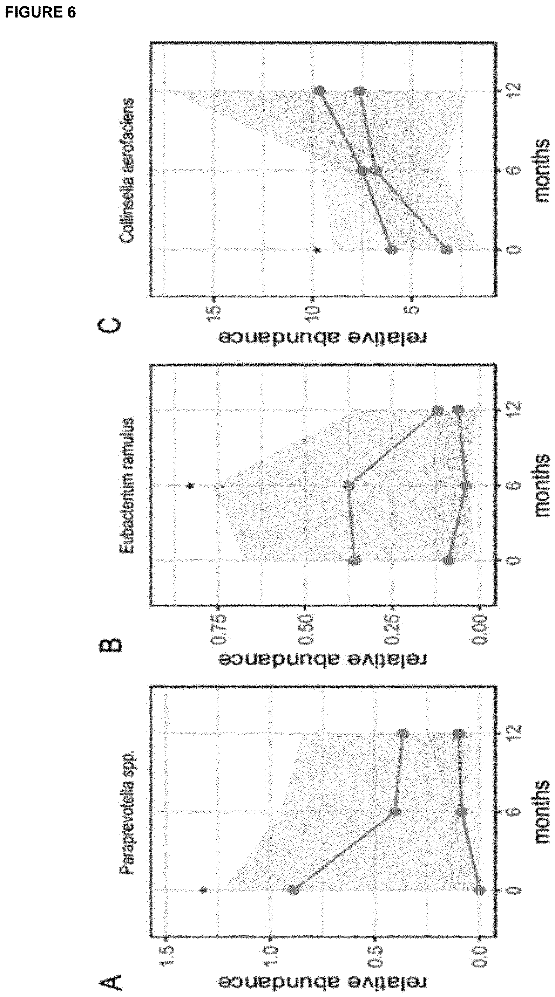

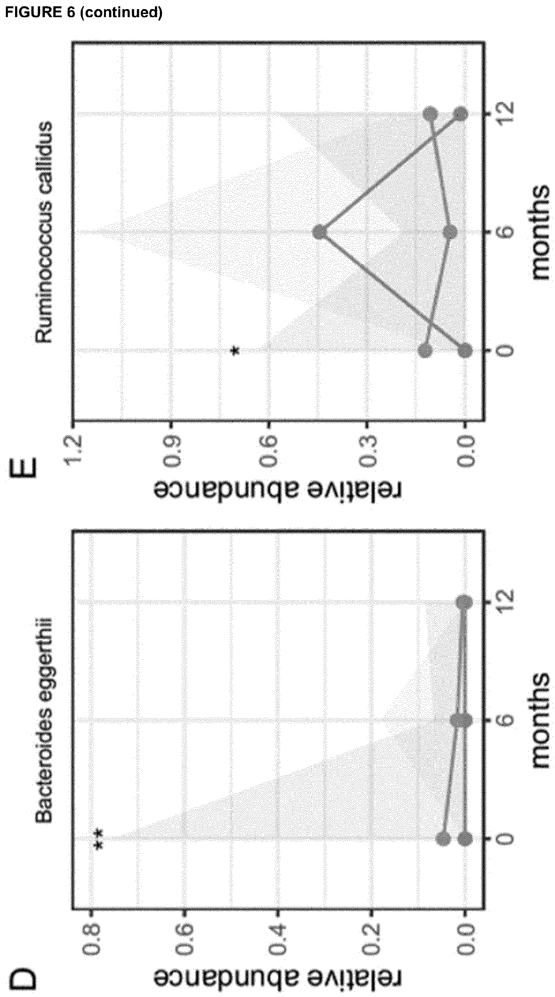

Figure 6: Abundance over time of five fecal microbiota from the top 10 (see Figure 3) that at baseline that best differentiated between responders and non-responders (in A, B, and D, the upper line represents responders, the other line non-responders; in C and E, the upper line represents non responders, the other line responders). Strains that were different between responders and non-responders at baseline or during the course of the study were chosen to display. P-values were calculated using Mann-Whitney U test at each timepoint. A: *Paraprevotella spp.,* p = 0.019, B: *Eubacterium ramulus,* p = 0.043, C: *Collinsella aerofaciens,* p = 0.043, D: *Bacteroides eggerthii,* p = 0.006, E: *Ruminococcus callidus, p* = *0.026. Faecalibacterium prausnitzii* (10th from the top 10), was not significantly different at baseline (p = 0.063).
Figure 7: Effect of 6-bromotryptophan (6-BT), 1-arachidonoyl-glycero-phosphocholine (20:0) (A-GPC), 1-palmitoyl-2-linoleoyl-sn-glycero-3-phosphoethanolamine (16:0-18:2 PE), 1-myristoyl-2-arachidonoyl-glycero-phosphocholine (MA-GPC) on NFκB pathway activation at different doses.
Figure 8: Effect of 6-BT, MA-GPC, A-GPC on myeloid cells: murine monocytes CD11b+ activated with LPS (10 ng/ml) for 24 h - TLR4 stimulation.
Figure 9: Effect of 6-BT, MA-GPC, A-GPC on myeloid cells: murine monocytes CD11b+ activated with Poly)I:C, analog of dsRNA, for 24 h - TLR3 stimulation
Figure 10: Effect of 6-BT on human monocytes in LPS stimulation.
Figure 11: Effect of 6-BT, A-GPC, MA-GPC on T lymphocytes: murine CD4+ T cells activated with anti-CD3 & anti-CD28 mAbs.
Figure 12: INS1e beta-cells treated for 24h with 6-BT: gene expression of beta cell differentiation markers.
Figure 13: INS1e beta-cells treated for 24h with 6-BT: gene expression of beta cell differentiation markers.
Figure 14: Murine bone marrow-isolated monocytes (Christ A,. Cell 2018) or bone marrow-derived macrophages (Swansen, JEM 2017) were exposed for 24h to the indicated concentrations of 6-BT (10-100uM) in presence or not of 10ng/ml LPS, 10ug/ml P3C or 10ug/ml poly(I:C). By means of ELISA assays, the inventors found that 6-BT can inhibits the secretion of proinflammatory cytokine TNFa upon TLR4 and TLR2 engagements and of IFNbeta upon TLR3 activation.
Figure 15: The present inventors investigated the effect of 6-BT on murine DC, differentiated with GM-CSF (40ng/ml) by bone marrow cells. As for the monocytes/macrophages, 6-BT inhibits the secretion of the proinflammatory cytokines TNFa and IFNbeta by DC after activation of , respectively, TLR4 (with 100ng/ml LPS) or TLR3 (10ug/ml poly(I:C) ).
Figure 16: The present inventors further studied the impact of 6-BT on CD4 T cells. To mimic antigen presentation, murine CD4 T cells (isolated from spleens; Uchimura T, Immunity 2019 ) were activated by monoclonal antibodies against CD3 and CD28 (2,5 and 1ug/ml, respectively). In line with the findings on myeloid cells, 6-BT significantly reduced the production of the Th1 cytokine IFNgamma.
Figure 17: The present inventors investigated whether 6-BT can exert a direct effect on beta cells. Indeed, we here see that 6-BT induces, in IS1E beta cells, the gene expression of the transcription factors PDX1 and MAFA, which are important for beta cell maturation and functionality. In agreement, 6-BT also promoted insulin secretion at steady-state and during glucose-stimulated insulin secretion (data shown as the difference between insulin release at starving condition [1mM glucose] and at hyperglycemic state [22mM]), (Paula S, FASEB J 2015).
Figure 18: The present inventors checked 6-BT impact on the activation of the NF-kB pathway, a central pathway in all inflammatory diseases (beyond autoimmunity). Hence, we quantified the expression of the phosphorylated form of the p65 subunit, regarded as a marker of NFkB activation. Upon T cell activation with PMA (50ng/ml) and ionomycin (1ug/ml), 6-BT could inhibit, at very early time-points (5-10minutes after activation), the NF-kB signaling. This effect was found in both murine and human (Jurkat cells) CD4 T cells.
Figure 19: Using the RAW264.7 murine macrophage cell line stably expressing an NFkB luciferase reporter (Groeneweg M, J Lipid Res 2006), the present inventors disclosed that overnight exposure of macrophages to 6-BT (10-200uM) inhibits the transcriptional activity of the NFkB complex in a dose-dependent manner upon 2h stimulation with LPS (10ng/ml).
Figure 20: In murine CD4 T lymphocytes (isolated from murine spleens; Uchimura T, Immunity 2019), 6-BT, but not tryptophan, exerted inhibitory effect on IFNgamma production upon CD3/CD28 engagement. This indicates that 6-BT and tryptophan elicit distinct biological activities.
Figure 21: Exposure of monocytes (isolated from murine bone marrow; Christ A,. Cell 2018) to 6-BT or tryptophan, shows that the anti-inflammatory effects are specific for the 6-bromotryptophan molecule and not for tryptophan.
Figure 22: The present inventors found that 6-BT (100uM) promoted the mitochondrial metabolism in murine and human (Jurkat) CD4 T cells. OCR=oxygen consumption rate, used as a proxy of cellular utilization of mitochondrial oxidative phosphorylation. OCR was measured using a Seahorse XF Analyzer Uchimura T, Immunity 2019; Chou, Nature 2021).
Figure 23: 6-BT exposure could enhance the mitochondrial metabolism of pro-inflammatory M1 macrophages (differentiated in presence of LPS and IFNgamma; Cheng N, JCI Insight 2018), without affecting the glycolytic flux. Intracellular metabolism measured using a Seahorse XF Analyzer Uchimura T, Immunity 2019; Chou, Nature 2021).

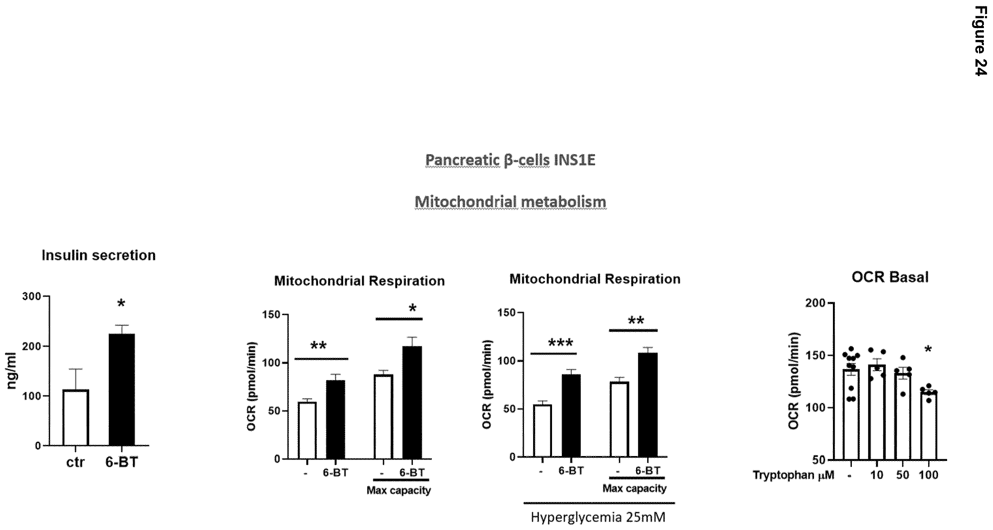

Figure 24: The present inventors investigated whether 6-BT may influence the mitochondrial metabolism of beta cells, which rely on ATP and mitochondrial metabolite production for insulin exocytosis. 6-BT increased mitochondria metabolism both at steady-state and under hyperglycemia (25mM glucose) in beta cells (INS1E beta cells). In addition, we tested the effect of tryptophan on intracellular metabolism and found that, as for the inflammatory markers, it exerted a different effect than 6-BT. Intracellular metabolism measured using a Seahorse XF Analyzer Uchimura T, Immunity 2019; Chou, Nature 2021).
Figure 25: Relative fecal *Desulfovibrio* abundance in Diabetes vs non-Diabetes subjects.
Figure 26: Diabetes: Effect of *Desulfovibrio* genus. Odds ratio for Diabetes.
Figure 27: Relationship between plasma 6BT levels and fecal *Desulfovibrio* genus relative abundance in (1) non Diabetes subjects (upper line) and (2) Diabetes patients (lower line).

### Example 1

Patients with conditions as indicated below are treated with
1. Oral administration, daily for 2 years, of an empty enteric coated capsule.
2. Oral administration, daily for 2 years, of an enteric coated capsule comprising ~1*10⁸ cells of an *Desulfovibrio* species *(Desulfovibrio piger, Desulfovibrio desulfuricans, or Desulfovibrio fairfieldensis)*
3. Oral administration, daily for 2 years, of an enteric coated capsule comprising 50 mg chloro-, fluoro-, or bromo-substituted tryptophan (6-bromotryptophan (6-BT) or 6-fluorotryptophan (6-FT)).
4. Oral administration, daily for 2 years, of an enteric coated capsule comprising 50 mg mono- or di-fatty acid substituted glycerol phosphocholine (GPC) (1-myristoyl-2-arachidonoyl-glycero-phosphocholine (MA-GPC) or 1-arachidonoyl-glycero-phosphocholine (A-GPC)).

**Table 1**

| *Condition* | *Effect treatment 1 (patient 1)* | *Effect treatment 2 (patient 2)* | *Effect treatment 3 (patient 3)* | *Effect treatment 4 (patient 4)* |
|---|---|---|---|---|
| Type 1 Diabetes | No effect | *(Desulfovibrio piger)* residual beta cell reserve is stabilized over time, beneficial changes in T and B cell function | (*6-BT*) residual beta cell reserve is stabilized over time, beneficial changes in T and B cell function | *(MA-GPC)* residual beta cell reserve is stabilized over time, beneficial changes in T and B cell function |
| Type 2 Diabetes | No effect | *(Desulfovibrio piger)* Reduced polyuria, reduced polydipsia, less need of exogenous hormone supplementation, beneficial changes in T and B cell function, less perceived fatigability | (6-BT) Reduced polyuria, reduced polydipsia, less need of exogenous hormone supplementation, beneficial changes in T and B cell function, less perceived fatigability | (A-GPC) Reduced polyuria, reduced polydipsia, less need of exogenous hormone supplementation, beneficial changes in T and B cell function, less perceived fatigability |
| Hashimoto's disease | No effect | *(Desulfovibrio piger)* Slowed down progression of disease, less need of exogenous hormone supplementation, beneficial changes in T and B cell function, less perceived fatigability | (6-BT) Slowed down progression of disease, less need of exogenous hormone supplementation, beneficial changes in T and B cell function, less perceived fatigability | *(MA-GPC)* Slowed down progression of disease, less need of exogenous hormone supplementation, beneficial changes in T and B cell function, less perceived fatigability |
| Graves' disease | No effect | *(Desulfovibrio desulfuricans)* Slowed down progression of the disease, reduced enlargement of thyroid gland, less risk of remission and radioactive iodine treatment need, beneficial changes in T and B cell function | (*6-FT*) Slowed down progression of the disease, reduced enlargement of thyroid gland, less risk of remission and radioactive iodine treatment need, beneficial changes in T and B cell function | *(A-GPC)* Slowed down progression of the disease, reduced enlargement of thyroid gland, less risk of remission and radioactive iodine treatment need, beneficial changes in T and B cell function |
| Addison's disease | No effect | *(Desulfovibrio piger)* Slowed down progression of disease, less need of exogenous hormone supplementation, beneficial changes in T and B cell function, reduced hyperpigmentation | (6-BT) Slowed down progression of disease, less need of exogenous hormone supplementation, beneficial changes in T and B cell function, reduced hyperpigmentation | *(MA-GPC)* Slowed down progression of disease, less need of exogenous hormone supplementation, beneficial changes in T and B cell function, reduced hyperpigmentation |
| Psoriasis | No effect | *(Desulfovibrio fairfieldensis)* Slowed down progression of disease, somewhat reduced red inflamed areas, beneficial changes in T and B cell function | (*6-FT*) Slowed down progression of disease, somewhat reduced red inflamed areas, beneficial changes in T and B cell function | *(A-GPC)* Slowed down progression of disease, somewhat reduced red inflamed areas, beneficial changes in T and B cell function |
| Vitiligo | No effect | *(Desulfovibrio piger)* Slowed down progression of disease, some white patches on the skin disappear, beneficial changes in T and B cell function | (*6-FT*) Slowed down progression of disease, some white patches on the skin disappear, beneficial changes in T and B cell function | *(A-GPC)* Slowed down progression of disease, some white patches on the skin disappear, beneficial changes in T and B cell function |
| Rheumatoid arthritis | No effect | *(Desulfovibrio piger)* Reduced progression of disease symptoms, and less pain around joints, less need of exogenous medication including DMARDS, beneficial changes in T and B cell function | (6-BT) Reduced progression of disease symptoms, and less pain around joints, less need of exogenous medication including DMARDS, beneficial changes in T and B cell function | *(MA-GPC)* Reduced progression of disease symptoms, and less pain around joints, less need of exogenous medication including DMARDS, beneficial changes in T and B cell function |
| Bechterew's disease | No effect | *(Desulfovibrio fairfieldensis)* Slowed down progression of disease, less perceived lower back pain, less need of exogenous medication including DMARDS, beneficial changes in T and B cell function | (6-BT) Slowed down progression of disease, less perceived lower back pain, less need of exogenous medication including DMARDS, beneficial changes in T and B cell function | *(MA-GPC)* Slowed down progression of disease, less perceived lower back pain, less need of exogenous medication including DMARDS, beneficial changes in T and B cell function |
| Celiac disease | Ingestion of small amount of gluten leads to upset stomach, stomach pain, inflammation, diarrhea, gas | *(Desulfovibrio piger)* Ingestion of small amount of gluten leads to less upset stomach but no pain, less diarrhea, less gas, less osteoporosis, beneficial changes in T and B cell function | (6-BT) Ingestion of small amount of gluten leads to less upset stomach but no pain, less diarrhea, less gas, less osteoporosis, beneficial changes in T and B cell function | *(MA-GPC)* Ingestion of small amount of gluten leads to less upset stomach but no pain, less diarrhea, less gas, less osteoporosis, beneficial changes in T and B cell function |
| Asthma | No effect | *(Desulfovibrio piger)* Slowed down disease progression, less episodes of coughing, shortness of breath (improved reversibility of FEV1 upon bronchodilators), less need of exogenous medication including synergy with bronchodilators, beneficial changes in T and B cell function | (6-BT) Slowed down disease progression, less episodes of coughing, shortness of breath (improved reversibility of FEV1 upon bronchodilators), less need of exogenous medication including synergy with bronchodilators, beneficial changes in T and B cell function | *(MA-GPC)* Slowed down disease progression, less episodes of coughing, shortness of breath (improved reversibility of FEV1 upon bronchodilators), less need of exogenous medication including synergy with bronchodilators, beneficial changes in T and B cell function |
| Emphysema (COPD) | No effect | *(Desulfovibrio piger)* Slowed down disease progression, less episodes of coughing, shortness of breath (improved reversibility of FEV1 upon bronchodilators), less need of exogenous medication including synergy with bronchodilators, beneficial changes in T and B cell function | (6-BT) Slowed down disease progression, less episodes of coughing, shortness of breath (improved reversibility of FEV1 upon bronchodilators), less need of exogenous medication including synergy with bronchodilators, beneficial changes in T and B cell function | *(MA-GPC)* Slowed down disease progression, less episodes of coughing, shortness of breath (improved reversibility of FEV1 upon bronchodilators), less need of exogenous medication including synergy with bronchodilators, beneficial changes in T and B cell function |
| Vasculitis | No effect | *(Desulfovibrio piger)* Slowed down progression of the disease, perceived severity of symptoms is reduced, i.e. less fever, fatique, weakness, weight loss, general aches and pains, numbness, beneficial changes in T and B cell function | (*6-FT*) Slowed down progression of the disease, perceived severity of symptoms is reduced, i.e. less fever, fatique, weakness, weight loss, general aches and pains, numbness, beneficial changes in T and B cell function | *(A-GPC)* Slowed down progression of the disease, perceived severity of symptoms is reduced, i.e. less fever, fatique, weakness, weight loss, general aches and pains, numbness, beneficial changes in T and B cell function |
| Systemic lupus erythematosus (SLE) | No effect | *(Desulfovibrio desulfuricans)* Slowed down progression of the disease, less painful and swollen joints, fever, chest pain, hair loss, mouth ulcers, swollen lymph nodes, and perceived fatigue, beneficial changes in T and B cell function | (6-BT) Slowed down progression of the disease, less painful and swollen joints, fever, chest pain, hair loss, mouth ulcers, swollen lymph nodes, and perceived fatigue, beneficial changes in T and B cell function | *(MA-GPC)* Slowed down progression of the disease, less painful and swollen joints, fever, chest pain, hair loss, mouth ulcers, swollen lymph nodes, and perceived fatigue, beneficial changes in T and B cell function |
| Guillain-Barré syndrome (GBS) | No effect | *(Desulfovibrio piger)* Slowed down progression of the disease, less perceived muscle weakness, beneficial changes in T and B cell function | (6-BT) Slowed down progression of the disease, less perceived muscle weakness, beneficial changes in T and B cell function | *(MA-GPC)* Slowed down progression of the disease, less perceived muscle weakness, beneficial changes in T and B cell function |
| Chronic inflammatory demyelinating polyneuropathy (CDIP) | No effect | *(Desulfovibrio piger)* Slowed down progression of the disease, less perceived muscle weakness, beneficial changes in T and B cell function | (*6-FT*) Slowed down progression of the disease, less perceived muscle weakness, beneficial changes in T and B cell function | *(A-GPC)* Slowed down progression of the disease, less perceived muscle weakness, beneficial changes in T and B cell function |
| Multiple sclerosis (MS) | No effect | *(Desulfovibrio piger)* Slowed down progression of the disease, less perceived muscle weakness, less trouble with sensation and coordination, beneficial changes in T and B cell function | (6-BT) Slowed down progression of the disease, less perceived muscle weakness, less trouble with sensation and coordination, beneficial changes in T and B cell function | *(MA-GPC)* Slowed down progression of the disease, less perceived muscle weakness, less trouble with sensation and coordination, beneficial changes in T and B cell function |
| Atherosclerosis | No effect | *(Desulfovibrio piger)* Reduced risk of coronary artery disease, reduced levels of inflammation markers (TNFalfa) | (6-BT) Reduced risk of coronary artery disease, reduced levels of inflammation markers (TNFalfa | (A-GPC) Reduced risk of coronary artery disease, reduced levels of inflammation markers (TNFalfa |

It is expected that results similar to the putative effects as shown in the Table 1 above can be obtained with larger patient cohorts.

### EXAMPLE 2

Recent-onset (<6 weeks) T1D patients were randomized in two groups to receive three *autologous* or *allogenic* (healthy donor) fecal microbiota transplantations (FMTs) over a period of 4 months.

It was found that several (microbiota derived) plasma metabolites and (small) intestinal bacterial strains are linked with improved residual beta cell function in Type 1 Diabetes.

### Materials and methods

A double-blind randomized controlled trial was performed in new onset T1D subjects using computerized randomization. The effects were studied of allogenic (healthy donor) compared to autologous (own) gut microbiota infusion on residual beta cell function and autoimmune T cell response in relation to changes in (small) intestinal microbiota during 1 year after treatment.

### Patient recruitment

New onset T1D patients were recruited from outpatient clinics in the Amsterdam region. Inclusion criteria for patients were males/females, age 18-35, normal BMI (18.5-25kg/m²), and a diagnosis of T1D within a maximum period of six weeks before inclusion, with residual beta cell function (as indicated by plasma C-peptide > 0.2 mmol/I and/or >1.2 ng/mL after MMT). Exclusion criteria were a diagnosis or symptoms of another autoimmune disease (eg hypo- or hyperthyroidism, celiac disease, rheumatoid arthritis or inflammatory bowel), (expected) prolonged compromised immunity (due to recent cytotoxic chemotherapy or HIV infection with a CD4 count < 240) as well as antibiotic use in the last 3 months, use of proton pump inhibitors and any other kind of systemic medication barring insulin.

### Donor recruitment

Lean (BMI < 25 kg/m²), omnivorous, healthy Caucasian male and females were recruited to serve as fecal donors. They completed questionnaires regarding dietary and bowel habits, travel history, comorbidity including family history of diabetes mellitus and medication use. Donors were screened for the presence of infectious diseases as described previously (van Nood et al., 2013). Blood was screened for human immunodeficiency virus; human T-lymphotropic virus; Hepatitis A, B, and C; cytomegalovirus (CMV); Epstein-Barr virus (EBV); strongyloides; amoebiasis, and lues. Presence of infection resulted in exclusion, although previous and non-active infections with EBV and CMV were allowed. Donors were also excluded if screening of their feces revealed the presence of pathogenic parasites (e.g. blastocystis hominis, dientamoeba fragilis, giardia lamblia), multiresistent bacteria *(Shigella, Campylobacter, Yersinia, MRSA ,ESBL, Salmonella, enteropathogenic E. Coli* and *Clostridium difficile)* or viruses (noro-, rota-, astro-, adeno (40/41/52)-, entero-, parecho- and sapovirus) as previously recommended.

### Study visits

Participants were asked to fill out an online nutritional diary for the duration of one week before every study visit to monitor caloric intake including the amount of dietary carbohydrates, fats, proteins and fibres. During the study visit, blood pressure, length, weight and daily insulin use were documented. Fasting blood samples were taken at each visit and upon centrifugation stored at -80C for later analyses. Whole blood sodium heparin tubes were kept on room temperature and processed within 24 hours for immunological analyses.

Three fecal transplantations using freshly produced feces were performed at 0, 2 and 4 months. Mixed-meal tests (for residual beta cell function), intestinal microbiota analyses were performed at 0, 2, 6, 9, and 12 months. Plasma metabolites were measured at 0, 6, and 12 months. Biometric measurements and fasting plasma to monitor safety parameters were performed on all time points.

### Description per study visit

All visits took place after an overnight fast with subjects taking no long acting insulin the night before. At each visit, blood sampling, fecal and urine sampling and biometric measurements took place. At baseline / 0 months, positioning of a nasoduodenal tube was performed. After placement of the tube, when the patient was properly awake, a standardized 2h mixed meal test (Nestlé sustacal boost^{®} ) was performed as previously described(Moran et al., 2013) to study residual beta cell function. At 2, 9, and 12 months, patients again underwent a mixed-meal test for residual beta cell C-peptide secretion. Then, a duodenal tube was placed by means of CORTRAK enteral access and the fecal transplant procedures were repeated. At 6 months, the mixed-meal test was performed.

### Fecal transplant procedure

Subjects were allocated randomly to receive three autologous or allogenic fecal transplantations. All patients and investigators were masked to treatment assignment. After admission, a duodenal tube was placed by gastroscopy or CORTRAK enteral access system. Each patient then underwent complete colon lavage with 2-4L of Klean prep^{®} (macrogol) by duodenal tube until the researcher judged that the bowel was properly lavaged (i.e. no solid excrement, but clear fluid) for approximately 3 h. Then, between 200 and 300 grams of donor feces was processed by dilution in 500 ml of 0.9% saline solution and filtered through unfolded cotton gauzes. The filtrate was used for transplantation two hours after the last administration of Klean prep^{®} by duodenal tube in around 30 minutes using 50cc syringes. After a short observation period the patient was sent home.

### Mixed meal test

Starting the evening before each mixed meal test, T1D patients paused their long-acting insulin injections. After an overnight fast and without taking their short-acting morning insulin dose, a mixed meal test was performed with Boost High Protein (Nestlé Nutrition, Vervey, Switzerland) at 6 ml/kg body weight with a maximum of 360 ml per person. Subsequent blood sampling for stimulated C-peptide were taken at -10, 0, 15, 30, 45, 60, 90 and 120 minutes. AUC (area under the curve) values were derived according to the trapezoidal rule.

### Plasma metabolites

Fasting plasma metabolite measurements were done by Metabolon (Durham, NC), using ultra high performance liquid chromatography coupled to tandem mass spectrometry (UPLC-MS/MS). Raw data was normalized to account for inter-day differences. Then, the levels of each metabolite were rescaled to set the median equal to 1 across all samples. Missing values, generally due to the sample measurement falling below the limit of detection, were then imputed with the minimum observed value for the respective metabolite.

### Biochemistry

Glucose and C-reactive protein (CRP, Roche, Switzerland) were determined in fasted plasma samples. C-peptide was measured by radioimmunoassay (Millipore). Total cholesterol, high density lipoprotein cholesterol (HDLc), and triglycerides (TG) were determined in EDTA-containing plasma using commercially available enzymatic assays (Randox, Antrim, UK and DiaSys, Germany). All analyses were performed using a Selectra autoanalyzer (Sopachem, The Netherlands). Low density lipoprotein cholesterol (LDLc) was calculated using the Friedewald formula. Calprotectin was determined in feces using a commercial ELISA (Bühlmann, Switserland).

### Fecal sample shotgun sequencing and metagenomic pipeline

Fecal microbiota were analysed using shotgun sequencing on donor fecal samples and fecal samples taken at 0, 6 and 12 months after initiation of study. DNA extraction from fecal samples for shotgun metagenomics was performed. Subsequently, shotgun metagenomic sequencing was performed (Clinical Microbiomics, Copenhagen, Denmark.). Before sequencing, the quality of the DNA samples was evaluated using agarose gel electrophoresis, NanoDrop 2000 spectrophotometry and Qubit 2.0 fluorometer quantitation. The genomic DNA was randomly sheared into fragments of around 350 bp. The fragmented DNA was used for library construction using NEBNext Ultra Library Prep Kit for Illumina (New England Biolabs). The prepared DNA libraries were evaluated using Qubit 2.0 fluorometer quantitation and Agilent 2100 Bioanalyzer for the fragment size distribution. Quantitative real-time PCR (qPCR) was used to determine the concentration of the final library before sequencing. The library was sequenced on a Illumina HiSeq platform to produce 2 x 150 bp paired-end reads. Raw reads were quality filtered using Trimmomatic (v0.38), removing adapters, trimming the first 5 bp, and then quality trimming reads using a sliding window of 4 bp and a minimum Q-score of 15. Reads that were shorter than 70 bp after trimming were discarded. Surviving paired reads were mapped against the human genome (GRCh37_hg19) with bowtie2 (v2.3.4.3) in order to remove human reads. Finally, the remaining quality filtered, non-human reads were sub-sampled to 20 million reads per sample and processed using Metaphlan2 (v2.7.7) to infer metagenomic microbial species composition and Humann2 (v0.11.2) to extract gene counts and functional pathways. In brief, reads were mapped using bowtie2 against microbial pangenomes; unmapped reads were translated and mapped against the full Uniref90 protein database using diamond (v0.8.38). Pathway collection was performed using the MetaCyc database.

### Small intestinal microbiota analyses

Biopsies were added to a bead-beating tube with 300 µl Stool Transport and Recovery (STAR) buffer, 0.25 g of sterilized zirconia beads (0.1 mm). 6ul of Proteinase K (20mg/ml; QIAGEN, Venlo, The Netherlands) was added and incubated for 1hr at 55 °C. The biopsies were then homogenized by bead-beating three times (60 s × 5.5 ms) followed by incubation for 15 min at 95 °C at 1000 rpm. Samples were then centrifuged for 5 min at 4 °C and 14,000 g and supernatants transferred to sterile tubes. Pellets were re-processed using 200 µl STAR buffer and both supernatants were pooled. DNA purification was performed with a customized kit (AS1220; Promega) using 250 µl of the final supernatant pool. DNA was eluted in 50 µl of DNAse- RNAse-free water and its concentration measured using a DS-11 FX+ Spectrophotometer/Fluorometer (DeNovix Inc., Wilmington, USA) with the Qubit^{™} dsDNA BR Assay kit (Thermo Scientific, Landsmeer, The Netherlands). The V5-V6 region of 16S ribosomal RNA (rRNA) gene was amplified in duplicate PCR reactions for each sample in a total reaction volume of 50 µl. A first step PCR using the 27F and the 1369R primer were used for primary enrichment. 1ul of 10uM primer, 1 µl dNTPs mixture, 0.5 µl Phusion Green Hot Start II High-Fidelity DNA Polymerase (2 U/µl; Thermo Scientific, Landsmeer, The Netherlands), 10 µl 5× Phusion Green HF Buffer, and 36.5 µl DNAse- RNAse-free water. The amplification program included 30 s of initial denaturation step at 98°C, followed by 5 cycles of denaturation at 98 oC for 30 s, annealing at 52 °C for 40 s, elongation at 72 °C for 90 s, and a final extension step at 72 °C for 7 min. On the PCR product a nested PCR was performed using the master mix containing 1 µl of a unique barcoded primer, 784F-n and 1064R-n (10 µM each per reaction), 1 µl dNTPs mixture, 0.5 µl Phusion Green Hot Start II High-Fidelity DNA Polymerase (2 U/µl; Thermo Scientific, Landsmeer, The Netherlands), 10 µl 5× Phusion Green HF Buffer, and 36.5 µl DNAse- RNAse-free water. The amplification program included 30 s of initial denaturation step at 98°C, followed by 5 cycles of denaturation at 98 °C for 10 s, annealing at 42 °C for 10 s, elongation at 72 °C for 10 s, and a final extension step at 72 °C for 7 min. The PCR product was visualised in 1% agarose gel (~280 bp) and purified with CleanPCR kit (CleanNA, Alphen aan den Rijn, The Netherlands). The concentration of the purified PCR product was measured with Qubit dsDNA BR Assay Kit (Invitrogen, California, USA) and 200 ng of microbial DNA from each sample were pooled for the creation of the final amplicon library which was sequenced (150 bp, paired-end) on the Illumina HiSeq. 2500 platform (GATC Biotech, Constance, Germany).

Raw reads were demultiplexed using the Je software suite (v2.0.) allowing no mismatches in the barcodes. After removing the barcodes, linker and primers, reads were mapped against the human genome using bowtie2 in order to remove human reads. Surviving microbial forward and reverse reads were pipelined separately using DADA2(Callahan et al., 2016) (v1.12.1). Amplicon Sequence Variants (AVSs) inferred from the reverse reads were reverse-complemented and matched against ASVs inferred from the forwards reads. Only non-chimeric forward reads ASVs that matched reverse-complemented reverse reads ASVs were kept. ASV sample counts were inferred from the forward reads. ASV taxonomy was assigned using DADA2 and the SILVA (v132) database. The resulting ASV table and taxonomy assignments were integrated using the phyloseq R package (v1.28.0) and rarefied to 60000 counts per sample.

### Power calculation and statistics

A sample size of 17 patients in each group (34 patients in total) was needed to provide 80% power to detect a 50% difference in the C-peptide AUC (360 mmol/l/min vs 180 mmol/I/min with a standard deviation of 170) between treatment groups at 12 months, with a two-sided test at α=0·05 and 10% dropout. All analyses were based on the prespecified intention-to-treat cohorts with known measurements (complete case analysis); missing values were assumed to be missing at random. Primary endpoint of the trial was the preservation of residual (MMT stimulated) beta cell function at 6 and 12 months compared to baseline (0 months). Other secondary endpoints were changes during these 12 months in whole blood leukocyte subsets for immunologic markers of autoimmunity , parameters of glycemic control as well as fasting plasma metabolites. Finally, changes between baseline and 6 months after start of the FMT in small intestinal epithelial genes were determined. Analyses were done by intention to treat.

For baseline differences between groups unpaired Student t-test or the Mann-Whitney U test were used dependent on the distribution of the data. Accordingly, data are expressed as mean ± the standard deviation or the median with interquartile range. Post-prandial results (e.g. c-peptide) are described as area under the curves (AUC) for the 2 hour post-prandial follow-up, calculated by using the trapezoidal method. For correlation analyses, Spearman's Rank test was used (as all parameters were non-parametric). For comparison of the primary end point a linear mixed model (LMM) was used (lme4 package in R), where 'allocation' and 'timepoint' were fixed effects and 'patient entry number' was a random effect. The p value for the interaction between 'allocation' and 'timepoint' was reported. Additionally, parameters were compared between groups at various time points using Mann-Whitney U test. A p-value < 0.05 was considered statistically significant. The study was conducted at the Academic Medical Center (Amsterdam), in accordance with the Declaration of Helsinki (updated version 2013). All participants provided written informed consent and all study procedures were approved by the IRB (ethics committee) of the Academic Medical Center. The study was prospectively registered at the Dutch Trial registry (NTR3697).

### Machine learning and follow-up statistical analyses

Extreme Gradient Boosting (XGBoost) machine learning classification algorithm was applied, in combination with a stability selection procedure to identify which parameters (either as values at baseline, or as relative changes) best predicted treatment groups and responders versus non-responders. This technique was used on duodenal microbial composition (16S rRNA sequencing on biopsies), on fecal microbiota composition and metabolic pathway abundance, and on plasma metabolite levels. To predict treatment groups, we used the relative change (delta) of each parameter between 0 and 12 months. For duodenal microbes we used delta 0 vs 6 months. For prediction of responders vs non-responders, baseline values, delta 0 vs 6 months and delta 0 vs 12 months were used. Each analysis produced a ranked list of the top 30 of most discriminative features. The top parameters were selected from each analysis that accurately (i.e.ROC AUC of 0.8 or higher) or moderately (ROC AUC >0.7) predicted group allocation for closer study, using an arbitrary but reasonable cut off. This cut off was generally a relative importance of around 30% or higher. Then, the change in time was visualized of the selected parameters (Wilcoxon's signed rank tests) and between-group differences were studies (Mann-Whitney U tests) at each time point using and finally, using Spearman's rank test, these parameters were correlated with the primary end point and with other key parameters that were identified in this way.

### Results

Patients were randomly assigned to donor FMT (n=11 subjects) or autologous FMT (n=10 subjects). One participant retracted consent after the first study visit. Due to lack of funding, the trial was stopped after 20 subjects were enrolled and completed the study. Seven healthy lean donors (of whom 3 were used twice) donated for the allogenic gut microbiota transfer to 10 new onset DM1 patients, and the same donor was used for the three consecutive FMT's in each DM1 patient. There were no differences at baseline between both groups and also throughout the follow-up period, there were no serious adverse events or adverse changes in plasma biochemistry in both treatment groups.

### Autologous FMT preserves (stimulated) C-peptide levels better than allogenic FMT

Mean fasting plasma C-peptide at baseline was similar between groups (327 pmol/I +/- 89 in the allogenic group vs 319 +/- 118 in the autologous group; p=0.86, Student's T-test), but deteriorated in the allogenic FMT group compared to the autologous FMT group at 12 months (202 +/- 85 vs 348pmol/l +/- 115 , Student's T-test p-value=0.0049; LMM p=0.00019). A similar effect was seen in stimulated C-peptide response AUC, which was similar between groups at baseline (361 +/- 154 mmol/l·min in the allogenic group vs 355 +/- 97 in the autologous group; p=0.92, Student's T-test), but residual betacell function was significantly more preserved at 12 months after autologous FMT (392 +/- 124 vs 248 +/- 153 mmol/l·min, Student's T-test p-value = p=0.033, LMM p-value = 0.000067). As expected, exogenous insulin treatment lowered HbA1c levels in both groups at 12 months. Despite similar amounts of daily exogenous insulin needs between the allogenic (0.45+/- SD IU/kg/day) and the autologous FMT group (0.47 IU/kg/day), and although not significant improved glycemic control was suggested in the autologous FMT compared to the allogenic FMT group (HbA1c 46 vs 53.5 mmol/mol, MWU p=0.19, LMM p-value = 0.12). Glucometabolic parameters at 0, 6 and 12 months were determined. Finally, BMI, fecal calprotectin, microalbuminuria, lipid profiles, and dietary intake (separate assessment of total calories, fat, saturated fat, protein, carbohydrates, and fiber) were not different at baseline nor during the course of the study.

### Treatment success of autologous FMT can be predicted by changes in plasma metabolites as well as microbiota composition

### Small intestinal microbiota differences between FMT treatment groups

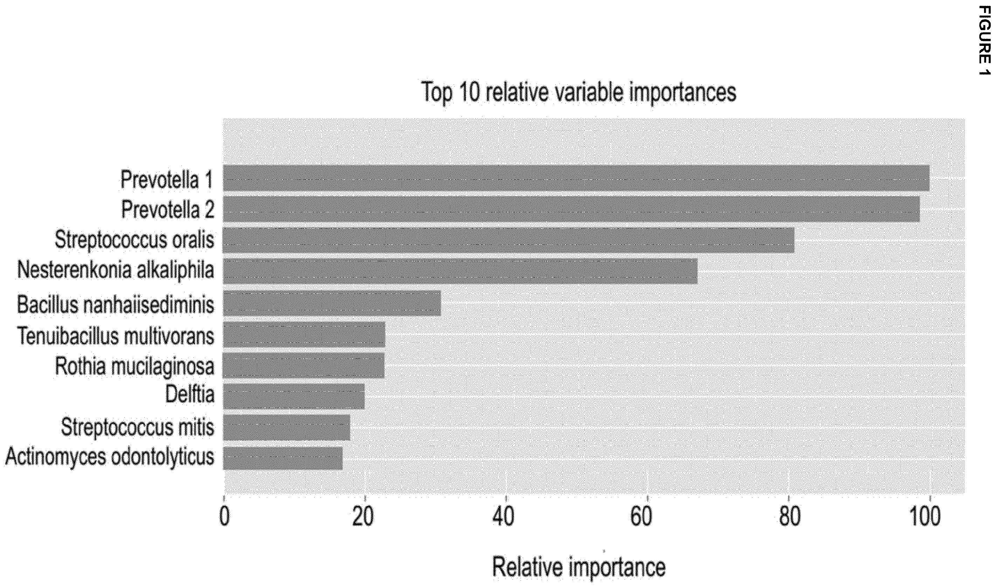

Alpha diversity of the small intestinal microbiota was not significantly different between treatment groups at baseline, but at 6 months there was a significant difference between autologous and allogenic FMT group (p=0.054) which was in line with a significant increase in diversity in the allogenic FMT group (p=0.009). When plotted along ordination axes in a redundancy analysis (RDA-plot), small intestinal microbiota compositions clustered differently at baseline between groups and also changed differentially between treatment groups. FMT treatment group allocation could be predicted reliably by change in specific small intestinal bacterial strains (AUC ROC 0.89 ± 0.18) including two species of *Prevotella* and *Streptococcus oralis* (Figure 1). However, changes on the phylum, family, genus, and species level showed no major shifts in small intestinal microbiota composition. Relative abundances of all these species decreased after autologous fecal transplantation, but increased after allogenic fecal transplantation. Of note, the relative abundance of *Prevotella 1* showed a baseline difference between groups (p=0.033). The delta was significantly different between groups for *Prevotella* 2 (p=0.048), but not for *Prevotella* 1 (p=0.069) or S. *oralis.* Furthermore, a significant inverse correlation was observed between *Prevotella* 1 relative abundance and stimulated C-peptide AUC (Spearman p=0.015, rho=-0.55).

### Fasting plasma metabolite changes upon FMT

Fasting plasma metabolite levels were different between DM1 and donors and were altered upon FMT. Treatment group allocation was reliably predicted by change in fasting plasma metabolites between 0 and 12 months (ROC AUC 0.79 ± 0.23). The relative importance of the ten most predictive metabolites are shown in Figure 2A. From the top 3 metabolites 1-*myristoyl-2-arachidonoyl-GPC* (MA-GPC)(p=0.02) and *1-arachidonoyl-GPC* (A-GPC)(p=0.02, Mann-Whitney U test), but not *1-(1-enyl-palmitoyl)-2-linoleoyl-GPE* (EPL-GPE), were different between groups at 12 months (Figure 2B-D). Also, changes in plasma MA-GPC levels correlated significantly with changes in fasting C-peptide (p=0.012, Mann-Whitney U test ,Figure 2E).

### Fecal microbiota changes upon FMT

Fecal microbiota composition was different between Dm1 and healthy donors at baseline and also changed differentially between treatment groups. However, alpha diversity did not differ significantly between FMT treatment groups at baseline, 6 or 12 months nor between donors and recipients. Some shifts were seen on phylum, family, genus and species level between groups. Group allocation prediction based on fecal microbiota taxonomic changes between 0 and 12 months showed a moderate ROC AUC of 0.72 ± 0.24. *Desulfovibrio piger* stood out as the most differentiating bacterial strain between treatment groups (Figure 3). Treatment group prediction based on metabolic pathways showed a relatively poor ROC AUC of 0.68 ± 0.27. Interestingly, abundance of *D*. *piger* changed differentially between treatment groups at 6 (p=0.024, MWU) and 12 (p=0.023) months follow-up (Figure 2G-H). Furthermore, change in *D. piger* correlates positively with change in fasting C-peptide (p=0.009, Figure 2I) and with plasma 1-arachidonoyl-GPC levels (p=0.004, Figure 2J). Moreover, a change in relative abundance of *D. piger* was inversely correlated with changes in relative abundance of both *Prevotella 1* (Figure 2K) and *Prevotella 2* (Figure 2L). Furthermore, change in *D. piger* correlates positively with change in fasting C-peptide (p=0.009, Figure 2I) and with plasma 1-arachidonoyl-GPC levels (p=0.004, Figure 2J). Moreover, change in relative abundance of *D*. *piger* was inversely correlated with change in relative abundance of both *Prevotella* 1 (Figure 2K) and *Prevotella* 2 (Figure 2L).

### Baseline fecal microbiota composition predicts FMT response.

As gut microbiota composition was different between healthy and T1D subjects in various age groups, the inventors also reasoned that FMT per se is an intervention in autoimmune diseases, as FMT introduces fecal material in the small intestine. Thus, a post hoc analysis was performed studying responders compared to non-responders to FMT, irrespective of treatment group. The inventors thus investigated whether baseline characteristics of T1D patients can predict response to FMT therapy at 12 months follow-up and which bacterial strains and plasma metabolites were associated with this response. Clinical response was defined as <10% decline in beta cell function compared to baseline at 12 months follow-up, which is significantly less than the expected natural 1-year decline of 20% in beta cell function. At 6 months follow-up, 2 months after the final FMT, 12/20 subjects were responders. At 12 months follow-up, clinical response sustained in 10 subjects of whom 3 had received allogenic and 7 had received autologous FMT (Figure 4A-B). The inventors thus chose responders at 12 months for the analyses because the primary end point (MMT stimulated C-peptide) was significantly different at 12 (but not at 6) months and because interference by the honeymoon phase is less at the 12 vs 6 months time point. The inventor next used predictive modeling to determine which baseline parameters (either their baseline values or delta 0-12 month values) were predictors of clinical response to FMT.

### Baseline fecal microbiota composition best predicts clinical response upon FMT

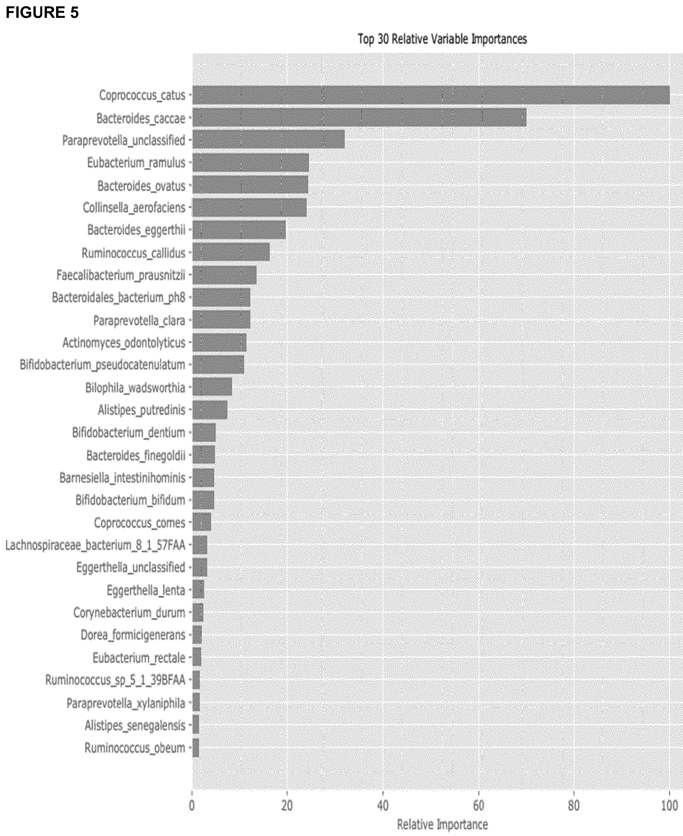

Baseline fecal microbiota composition predicted clinical response upon FMT very accurately (AUC ROC 0.93 ± 0.14). In this regard, intestinal levels of *Bacteroides caccae* and *Coprococcus catus* stood out as most differentiating microbes (Figure 5), both of which were significantly more abundant at baseline in responders than in non-responders (Figure 4C-D). From the top 10 most differentiating intestinal bacterial strains, *Paraprevotella spp, Collinsella aerofaciens, Bacteroides eggerthii* and *Ruminococcus callidus* were also significantly different at baseline between responders and non-responders (Figure 6A-E). A significant (negative) correlation was observed between change in *C*. *catus* abundance and stimulated C-peptide AUC (p=0.053, r=-0.44, figure 4E). Response was predicted less accurately by change in fecal microbiota composition (AUC ROC 0.76 ± 0.23) than by baseline composition suggesting that at T1D diagnosis gut microbiota composition can predict gut microbiota based treatment efficacy. The most differentiating species were *Bacteroidales bacterium ph8, Actinomyces viscosus, Bacteroides thetaoitaomicron, Streptococcus salivarius, Ruminococcus bromii* and *Clostridium leptum,* of which *B*. *bacterium ph8* (p=0.015, Mann-Whitney U test) and *R. bromii* (p=0.013) become less abundant in responders vs non-responders, *S. salivarius* (p=0.045) becomes more abundant in responders vs non-responders and *B*. *thetaiotaomicron* is significantly different at baseline and shows a downward trend in responders.

### Integration of multiomics analyses upon FMT

Correlations between parameters that were found to be significantly affected by FMT were explored. Since responders were found in both treatment groups, correlations were first explored in our pooled dataset (n=20) and then within treatment groups separately and in clinical responders upon FMT. In the pooled dataset an intertwined cluster of notable parameters was found which positively and negatively associated with markers of glucose regulation (i.e. C-peptide AUC, fasting C-peptide and HbA1c). On one hand, the highly correlated plasma metabolites MA-GPC and A-GPC accurately predicting preservation of insulin secretion, correlate positively to *D. piger* which correlates positively to fasting C-peptide. On the other hand, Prevotella *1*, *Prevotella 2* and *S*. *oralis* correlate negatively to glucose regulation and with metabolites MC-GPC and A-GPC. Analyzing treatment groups separately, preserved beta cell function (high C-peptide) in the autologous group was characterized at baseline by high *Coprococcus catus,* as well as a subsequent decrease in *Ruminococcus bromii.* In the allogenic group, preserved beta cell function was characterized by a decrease in fecal *Roseburia intestinalis* (which incidentally correlates positively with *Prevotella 1* and *2*). Finally, in clinical responders, preserved beta cell function was characterized by decreases in duodenal *Prevotella 1 , Prevotella 2,* fecal *Coprococcus catus,* metabolite EPL-GPE, whereas *D. piger* increased.

### Analysis

The inventors here report for the first time that FMT can have an effect on residual beta cell function in new onset T1D patients. This accords with recent observational studies supporting a role for the intestinal microbiota in T1D subjects. Contrary to expectations, autologous FMT performed better than healthy donor FMT, while even in the allogenic group the decline in residual beta cell function appeared less than expected without treatment. An appealing explanation would be that beneficial immunological effects of FMT are more pronounced and durable, when the FMT donor microbiota better matches the immunological tone of the host. This to the extent that beneficial effects of healthy donor stool may be dampened by (immuno)incompatibility. Other observations also point towards a immunological regulatory role of specific plasma metabolites that are derived from diet and converted by intestinal microbiota. While the overall clinical effects of FMT were modest and show a wide variety between new onset T1D subjects, the intervention was safe and had no side effects. We propose that changes in plasma metabolites, predominantly fatty acids and tryptophan derivatives, as a consequence of the altered intestinal microbiota composition, may explain the observed beneficial effects of FMT on residual beta cell function in patients with new onset T1D.

*Preservation of beta cell function is associated with changes in specific gut microbiota strains D. piger* may dampens autoimmunity in T1D via *plasma 1-arachidonoyl-GPC.* Predictive modeling showed that baseline fecal microbiota taxonomy and metabolic pathways accurately predicted response at 12 months. However, the identified microbes (e.g. *B. caccae* and *C*. *catus*) did not correlate with any of our relevant immune parameters, small intestinal genes or plasma metabolites. This suggests that fecal microbiota composition is consequence rather than cause of the host immunological characteristics that associate with response. The exception to this was *D. piger,* a sulfate-reducing bacterial strain. Its beneficial effects may be mediated by its production of hydrogen sulfide. Moreover, we identified *D. piger* as outstanding fecal microbial predictor of FMT treatment group allocation. Interestingly, this small intestinal bacterial strain was also beneficially associated with change in stimulated C-peptide reponses upon FMT and its abundance increased in the autologous group and in the overall responders. Interestingly, *D. piger* correlated positively with levels of plasma 1-arachidonoyl-GPC (Figure 2J), one of the key metabolites that also associated with improved C-peptide production. In conclusion, *D. piger* could be a strong candidate to dampen autoimmunity through production of A-GPC, e.g. through uptake by protruding dendrites of immune cells into the intestinal lumen. Interestingly, *D. piger* was recently cultured from the human intestinal tract, enabling testing this bacterial strain in human T1D (Chen et al 2019 Letters in Applied Microbiology 68(6) 553-561). Other bacterial species in the duodenum that best differentiated between treatment groups were two unnamed *Prevotella spp.* and *Streptococcus oralis.* Our explorative integration of multiomics analyses subsequently show that these *Prevotella spp.* and *S*. *oralis* are negatively associated with our key beneficial metabolite MA-GPC, a glycerophospholipid. *B. stercoris* correlated positively with *D. piger* and A-GPC and negatively with *S*. *oralis,* but did not correlate positively with C-peptide. Finally, changes in *Ruminococcus bromii* (autologous FMT group) and *Roseburia intestinalis* (allogenic FMT group) were negatively associated with changes in C-peptide, although both strains are generally regarded as beneficial microbes that thrive during fibre-rich diets, produce SCFA's and promote intestinal integrity.

### Conclusions

Fecal transplantation of colon-derived microbiome into the host small and large intestine in T1D patients effectively prolongs residual beta cell function and thus honeymoon phase. Moreover, several novel bacterial strains including fecal *D. piger* and *B*. *stercoris* as well as duodenal *Prevotella spp.* and *S*. *oralis* were identified with therapeutic potential. Accordingly, increases in plasma metabolites such as 1-myristoyl-2-arachidonoyl-GPC, 1-arachidonoyl-GPC, and 6-bromotryptophan upon FMT associated with beneficial changes.

### EXAMPLE 3

In this Example, the effects of the following compounds were assessed in cell-based assays:
- 6-bromotryptophan (6-BT)
- 1-arachidonoyl-glycero-phosphocholine (20:0) (A-GPC)
- 1-palmitoyl-2-linoleoyl-sn-glycero-3-phosphoethanolamine (16:0-18:2 PE)
- 1-myristoyl-2-arachidonoyl-glycero-phosphocholine (MA-GPC)

### Materials and methods

### Metabolite preparation and cell culture

6-bromotryptophan (6-BT) (Alichem) was purchased as powder and dissolved in DMSO at 50mM.

1-arachidonoyl-glycero-phosphocholine (20:0) (LysoPC(20:0) (Avanti Polar) was purchased as powder and was dissolved in PBS at 0,9mM.

1-palmitoyl-2-linoleoyl-sn-glycero-3-phosphoethanolamine (16:0-18:2 PE) (Avanti Polar) was purchased in chloroform at 10mg/ml; 200 µl (equivalent to 2mg) were transferred to a glass tube and chloroform was evaporated under nitrogen flow to obtain a transparent film, which was afterwards dissolved in PBS at 1mM.

1-myristoyl-2-arachidonoyl-glycero-phosphocholine (MA-GPC) (Syncom, custom made synthesized) was purchased as powder and dissolved in DMSO at 10mM.

All cell-types were culture at 37°C in a 5% CO₂ atmosphere and treated with the metabolites for no longer than 24h, in control condition the appropriate vehicle (DMSO or PBS) was added in the medium.

### NF-κB reporter macrophage and Luciferase assay

NF-κB signaling activation was assessed by luciferase activity assay. RAW264.7 cells stably transfected with the 3×-κB-luc plasmid (DNA construct containing three NF-κB sites from the Ig κ light chain promoter coupled to the gene encoding firefly luciferase) were grown in DMEM medium supplemented with 10% heat-inactivated fetal bovine serum, penicillin (100 U/ml), streptomycin (100 µg/ml), L-glutamine (2 mM). Cells were seeded in F-bottom 96-well plates at a density of 0,5×10⁵ per well and the following day stimulated with LPS (10/100 ng/ml) for 2h with/without addition of metabolites at different concentrations (6-BT 0,1-100 µM, LysoPC(20:0) 1-10 µM, 16:0-18:2 PE 1-50 µM, MA-GPC 1-100 µM). Afterwards, cells were lysed with 25µl/well 1×passive lysis buffer and firefly luciferase activity was measured by Luciferase^{®} Assay System (Promega, E1500) on a GloMax^{®}-Multi Detection System (Promega).

### In vitro stimulation of primary monocytes

Naïve bone marrow monocytes were isolated from BM cells. Briefly, hind legs were removed from 3 mice, cleaned from surrounding muscles. The femur and tibia bones were trimmed at their extremities, the BM content was flushed out with cold PBS using a 10ml syringe and a 25G needle and filtered through a 40µm strainer. Red blood cells (RBC) were lysed with 1x RBC lysis buffer (Biolegend) for 5min on ice. CD11b+ monocytes were further purified by positive selection using a cocktail of CD11b magnetic beads (Miltenyi Biotec, # 130-049-601) and magnetizing MS colums (Miltenyi Biote) according to the manufacturer's instructions. Subsequently, monocytes were seeded in F-bottom 96-well plates at 1×10⁵ cells per well in RPMI1640 supplemented with 10% heat-inactivated fetal bovine serum, penicillin (100 U/ml), streptomycin (100 µg/ml), L-glutamine (2 mM) and activated with 10 µg/ml poly(I:C). Monocytes were activated with 10ng/ml LPS (*Sigma*-Aldrich) and subjected to treatment with 6-BT (10/100 µM), LysoPC(20:0) (10/50 µM), MA-GPC (10/50/100 µM), or appropriate vehicle. Cells were kept in a final volume of 200 µl/well for 24h, after which supernatant was harvested and stored at -80°C.

### In vitro stimulation of murine macrophages/dendritic cells (DC)

Bone marrow-derived macrophages (BMDM) and DC (BMDC) were obtained by differentiating freshly isolated BM cells from femur and tibia bones (as above described) of C57/BI6 mice (N=3 per experiment). For BMDM, BM cells were seeded at 3×10⁶ cells per 10 cm dish and cultured for 7 days in 12 ml RPMI1640 medium with 20% fetal bovine serum, 30% L-929 cell conditioned media, as source of murine macrophage colony-stimulating factor (M-CSF). For BMDC, BM cells were seeded at 0,5×10⁶ /ml cells in 25ml in 10 cm dishes and cultured for 7 days in presence of GM-CSF in 5%FBS- RPMI1640 medium. After differentiation, BM DM/DCs were harvested, counted and seeded at a density of 1×10⁵ cells per well in a F-bottom 96-well plate and left to adhere for 20 hours before experiments were performed. Macrophages were activated with 10 µg/ml polyinosinic-polycytidylic acid (poly(I:C) (InvivoGen) for 24h in presence or not of 6-BT (10/100 µM), LysoPC(20:0) (10/50 µM), or MA-GPC (10/50/100 µM) in a final volume of 200 µl/well. At the end of the assay, supernatant was harvested and stored at -80°C.

### In vitro CD4+ T cell activation assay

Primary CD4+ T cells were freshly isolated by negative selection from spleens of C57/BI6 mice (N=3 per experiment). Briefly, spleens were smashed in a culture dish and passed twice through a strainer (70µm and 40µm, respectively) to obtain a single-cell suspension. After red blood cell lysis (10min on ice) with 1x RBC lysis buffer (Biolegend), cells were counted, stained with a cocktail of biotin-conjugated antibodies against CD8a, CD11b, CD11c, CD19, CD45R (B220), CD49b (DX5), CD105, Anti-MHC-class II, Ter-119 and TCRγ/δ and subsequently stained wiith magnetically labeled with Anti-Biotin MicroBeads. using the negative selection (CD4+ T Cell Isolation kit, Miltenyi Biotec, #130-104-454). Non-CD4 T cells were depleted by retaining them in LS magnetizing column (Miltenyi Biotec).

Isolated CD4 T cells were cultured in 96-well plates (1×10⁵ per well) in RPMI1640 medium supplemented with 10% fetal bovine serum, 100 U/ml penicillin, 100 µg/ml streptomycin, 2 mM L-glutamine. Treatment with the metabolites 6-BT (1/10 µM), LysoPC(20:0) (10/50 µM) or MA-GPC (10/50/100 µM) and activation with 2,5 µg/ml soluble anti-CD3 (145-2C11, eBioscience) and 1 µg/ml soluble anti-CD28 (37.51, eBioscience) antibodies started immediately after seeding in 200µl/well complete RPMI1640 media for a 24h-period, at the end of which supernatant was harvested and stored at -80°C.

### In vitro stimulation of human monocytes

The mononuclear cell fraction was isolated from the blood of healthy volunteers (Sanquin Bloodbank, Amsterdam, The Netherlands) by density centrifugation using Lymphoprep^{™} (Axis-Shield) and isolated using human CD14 magnetic beads and MACS^{®} cell separation columns (Miltenyi Biotec) according to the manufacturer's instructions. Isolated primary human monocytes were counted and seeded at 1×10⁵ cells/mL in 24-well plates with 1ml medium supplemented with 10% fetal bovine serum, 100 U/ml penicillin, 100 µg/ml streptomycin, 2 mM L-glutamine. After seeding, cells were stimulated with 10 ng/ml LPS or 25mM D-glucose (both Sigma-Aldrich) for 24h with/without 100µM 6-BT. Afterwards, cells were lysed with Tripure isolation reagent (Roche) and stored at -80°C until RNA isolation.

### In vitro stimulation of pancreatic beta cells

INS1E cells (rat pancreatic beta-cell line) were maintained in complete RPMI1640 supplemented with 5% Fetal Bovine Serum, 2 mM L-glutamine, 5 µM beta-mercaptoethanol, 1 mM sodium pyruvate, 10 mM HEPES, 100 units/ml of penicillin and 100 µg/ml streptomycin. INSIE cells were seeded in 48-well plates at a density of 1×10⁵ cells per well and left resting for one day. Subsequently, medium was replaced with 0,5ml/well of INS1E complete RPMI1640 medium containing vehicle or the metabolites: 6-BT (1/10/25 µM), LysoPC(20:0) (5/10/50 µM), or MA-GPC (10/50/100 µM). After 24h, cells and supernatant were harvested for further analysis (gene expression and insulin secretion, respectively) and stored at -80°C. Cells were lysed in Tripure isolation reagent (Roche) before storage.

For glucose-stimulated insulin secretion (GSIS) assay, cells were preincubated in a Krebs-Ringer bicarbonate buffer (KRB) [115mMNaCl, 5mMKCl, 2.56 mMCaCl2, 1mMMgCl2, 10mM NaHCO3, 15 mM HEPES, and 0.3% of BSA (pH 7.4)] for 30 min at 37°C, following by stimulation with 1mM glucose in KRB for 1 h (0,5ml/well) and stimulation with 22 mM glucose in KRB for another hour (0,5ml/well) at 37°C. Supernatant was recovered after treatment with 1mM glucose (Sigma-Aldrich) and 22mM glucose and cells stored at -80°C. GSIS was performed on beta cells after treatment for 24h treatment with 10 µM 6-BT.

### ELISA

Specific ELISAs (R&D Systems) were utilized to measure the concentration of TNFα, IFNβ, and IFNγ in cell supernatant of murine monocytes, macrophages ad T cells, respectively, according to the manufacter's instructions. The concentration of insulin after 24h treatment with metabolites or after GSIS were determined using Rat insulin ELISA (Mercodia) according to the manufacturer's instructions. GSIS was calculated by subtracting the concentration of insulin at 1mM glucose to the insulin rate at 22mM glucose.

### Gene expression analysis

Total RNA was extracted from Tripure isolation reagent cell lysates. RNA was converted to cDNA by with iScript kit (BioRad ). Quantitative polymerase chain reaction (qPCR) was performed using SYBR Green-SensiMix (Bioline) on a CFX384 Touch Real-Time PCR Detection System (BioRad). The delta delta Ct method was used to calculated gene expression as fold-change compared to control (unstimulated conditions).

### Statistics

Statistical analysis was performed using Student t tests for two group comparison and One-Way ANOVA and Dunnett's tests for multiple group comparison. Data are presented as mean and standard error of the mean (SEM). P < 0.05 was considered to be significant.

### Results

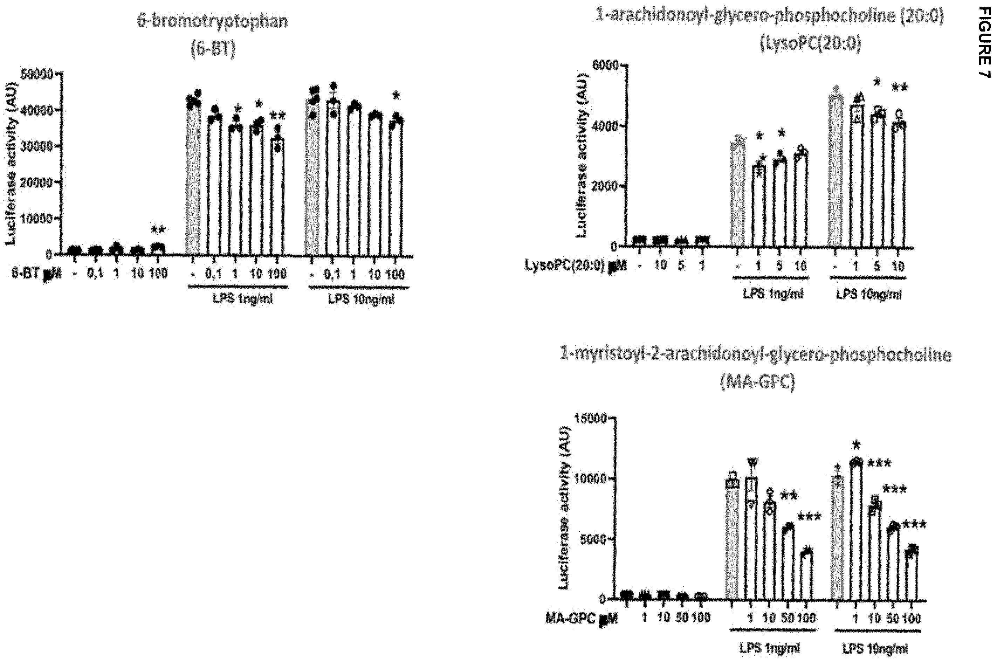

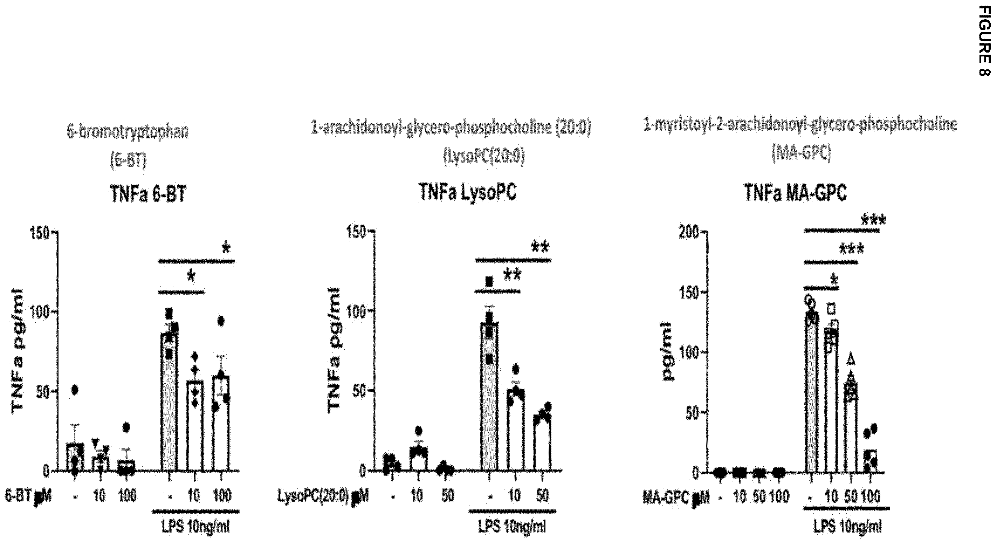

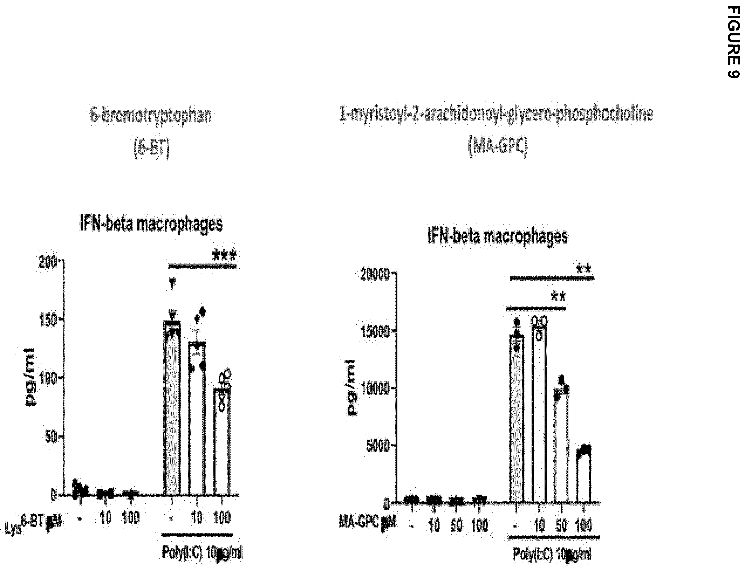

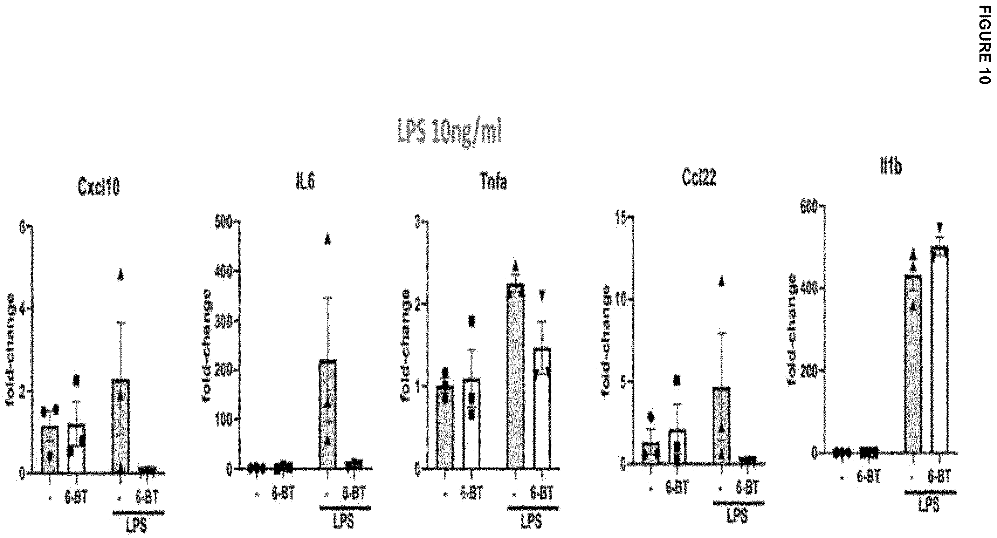

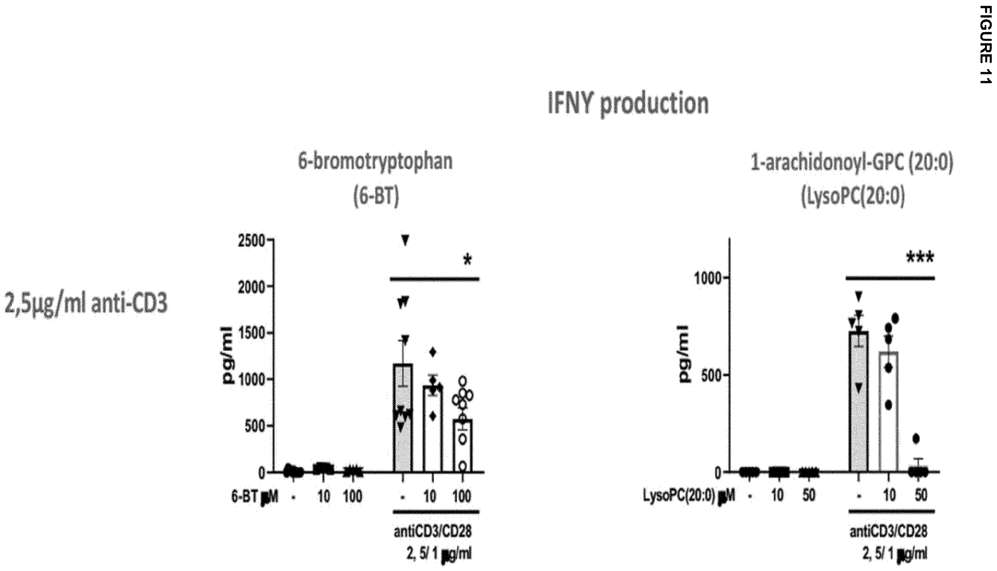

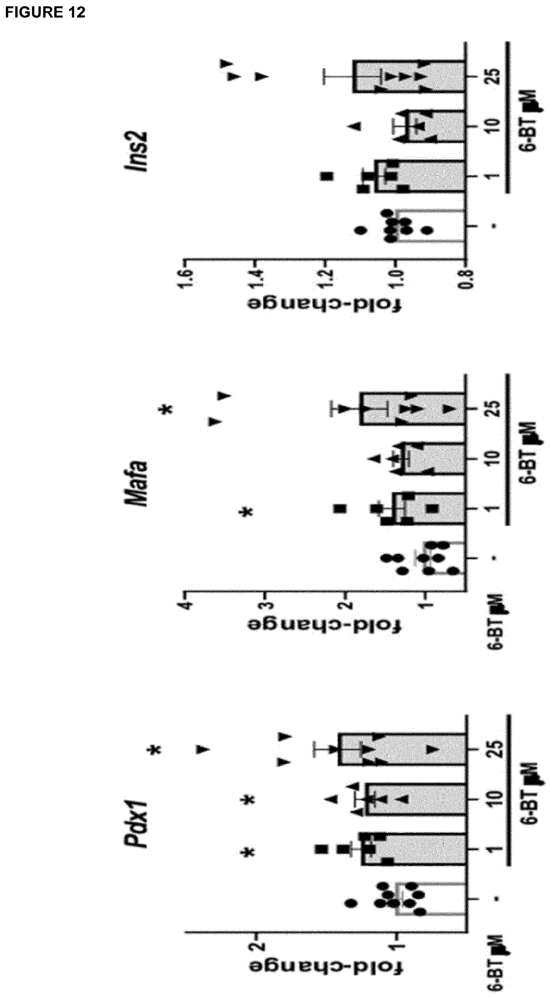

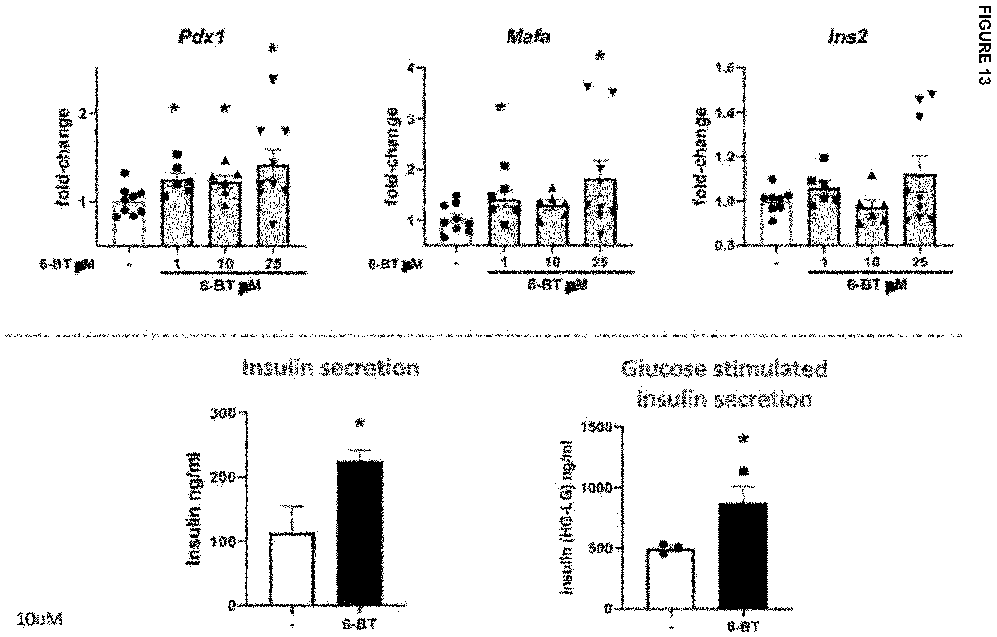

The results are shown in Figures 7-13:
- 6-BT, A-GPC, and MA-GPC can inhibit NFκB pathway activation in macrophages at different doses (Figure 7).
- 6-BT, A-GPC, and MA-GPC halt cytokine secretion by monocytes (Figure 8);
- 6-BT and MA-GPC impair type 1 IFN secretion (Figure 9);
- 6-BT reduces cytokine production by human monocytes (Figure 10);
- 6-BT and A-GPC dampen Th1 responses in CD4 T cells (Figure 11);
- 6-BT enhances pancreatic beta-cell function (Figures 12 and 13);

Of specific interest is 6-bromotryptophane which thus inhibits NFκB pathway activation, hampers immune responses in monocytes/macrophages and CD4 T cells, and improves pancreatic beta cell function. MA-GPC inhibits NFκB pathway activation, hampers immune responses in monocytes/macrophages and CD4 T cells.

### EXAMPLE 4

### Plasma 6-bromotrypophan levels inversely correlate with presence of Type 2 Diabetes mellitus and glycemic control in cross-sectional cohort (n=369 subjects)

In a 369 subjects cohort, evidence was found that 6-bromotrypophan may protect against the onset and progression of Type 2 Diabetes mellitus. More specifically, it was found that plasma 6-bromotrypophan levels inversely correlate with presence of Type 2 Diabetes mellitus and glycemic control. This suggest that 6-BT may contribute to Type 2 Diabetes prevention and treatment and thus improved (micro and macrovascular) cardiovacsular complications in Type 2 Diabetes. 6-BT may help to reduce macrovascular disease (i.e. cardiovascular disease) and microvascular complications also in non Type 2 Diabetes patients.

### Materials and methods

Fasting plasma targeted metabolite measurements were done by Metabolon (Durham, NC), using ultra high performance liquid chromatography coupled to tandem mass spectrometry (UPLC-MS/MS), as previously described [Koh A, Molinaro A, Stahlman M, et al. Microbially Produced Imidazole Propionate Impairs Insulin Signaling through mTORC1. Cell 2018;175:947-961.e17. doi:10.1016/j.cell.2018.09.055 ].

### Results

Results are shown in Tables 2 and 3.

**Table 2**

| | | **Total** | **CKD-T2DM** | **CKD+T2DM** | **Controls** | **p** |
|---|---|---|---|---|---|---|
| n | | 369 | 124 | 45 | 200 | |
| Age (mean (SD)) | | 51.72 (11.27) | 52.13 (10.98) | 60.24 (6.04) | 49.55 (11.44) | <0.001 |
| Sex = 2 (%) | | 196 (53.1) | 68 (54.8) | 19 (42.2) | 109 (54.5) | 0.294 |
| Etnicity (%) | | | | | | <0.001 |
| | Dutch | 84 (22.8) | 34 (27.4) | 0 (0.0) | 50 (25.0) | |
| | South Asian Surinamese | 101 (27.4) | 24 (19.4) | 27 (60.0) | 50 (25.0) | |
| | African Surinamese | 105 (28.5) | 37 (29.8) | 18 (40.0) | 50 (25.0) | |
| | Ghanaian | 79 (21.4) | 29 (23.4) | 0 (0.0) | 50 (25.0) | |
| BMI (mean (SD)) | | 27.48 (5.18) | 28.56 (5.66) | 30.06 (5.15) | 26.22 (4.48) | <0.001 |
| WHR (mean (SD)) | | 0.93 (0.09) | 0.94 (0.08) | 1.00 (0.08) | 0.91 (0.08) | <0.001 |
| SBP (mean (SD)) | | 136.22 (21.15) | 141.75 (22.56) | 148.43 (22.16) | 130.04 (17.69) | <0.001 |
| DBP (mean (SD)) | | 83.23 (11.52) | 86.54 (13.29) | 83.90 (9.42) | 81.03 (10.24) | <0.001 |
| HT (%) | | 215 (58.3) | 87 (70.2) | 39 (86.7) | 89 (44.5) | <0.001 |
| AntiHT (%) | | 107 (29.0) | 39 (31.5) | 28 (62.2) | 40 (20.0) | <0.001 |
| MDRD (mean (SD)) | | 96.03 (21.56) | 94.19 (21.34) | 89.81 (28.91) | 98.58 (19.38) | 0.024 |
| CKDEPI (mean (SD)) | | 94.99 (19.49) | 93.29 (20.02) | 84.31 (23.43) | 98.46 (17.13) | <0.001 |
| Microalb (mean (SD)) | | 0.44 (0.50) | 0.92 (0.27) | 0.96 (0.21) | 0.02 (0.16) | <0.001 |
| HbA1C (mean (SD)) | | 40.73 (9.58) | 39.52 (4.64) | 58.16 (15.74) | 37.56 (4.62) | <0.001 |
| Kreat (mean (SD)) | | 78.67 (21.61) | 80.17 (22.23) | 86.82 (36.63) | 75.90 (15.42) | 0.006 |

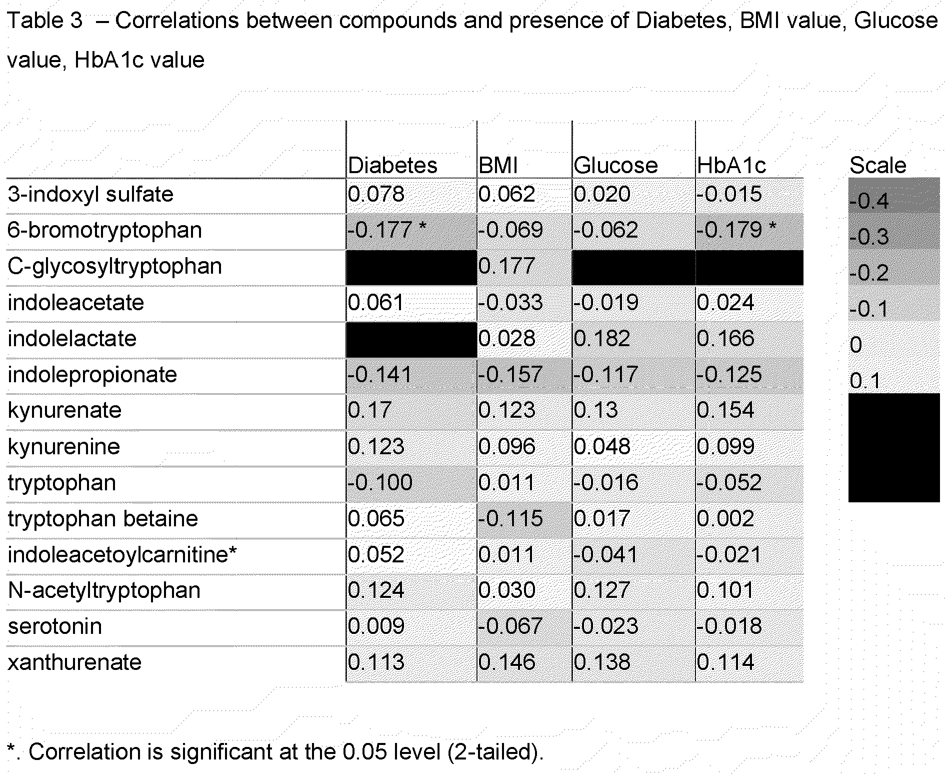

### 6-bromotryptophan halts inflammatory responses in myeloid cells

6-BT is a bromoindole derivative of tryptophan, which is known to be metabolized by indigenous gut microbes of the colon as well as of small intestine. So far, the physiological role of 6-BT is completely unknown. Clinical findings of the present inventors suggest a protective function against inflammation and diabetes.

With a series of in vitro/ ex vivo experiments, the present inventors elucidated some of 6-BT functions on immune cells as well as on insulin-producing pancreatic beta cells.

### 6-BT can inhibit the secretion of proinflammatory cytokine TNFa upon TLR4 and TLR2 engagements and of IFNbeta upon TLR3 activation

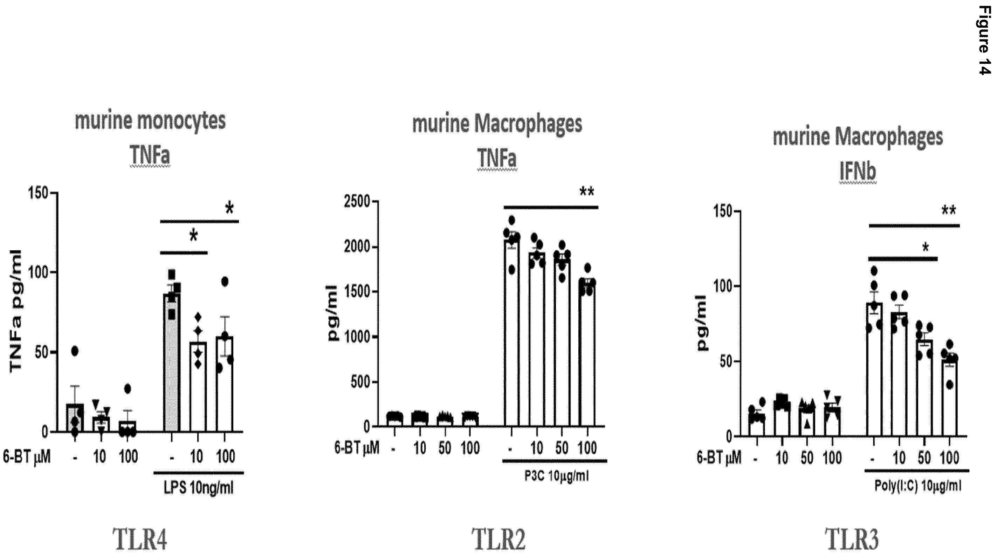

Here, murine bone marrow-isolated monocytes (Christ A, Western Diet Triggers NLRP3-Dependent Innate Immune Reprogramming. Cell 2018) or bone marrow-derived macrophages (Swanson KV, A noncanonical function of cGAMP in inflammasome priming and activation. JEM 2017) were exposed for 24h to the indicated concentrations of 6-BT (10-100uM) in presence or not of 10ng/ml LPS, 10ug/ml P3C or 10ug/ml poly(I:C). By means of ELISA assays, the present inventors found that 6-BT can inhibit the secretion of proinflammatory cytokine TNFa upon TLR4 and TLR2 engagements and of IFNbeta upon TLR3 activation. See Figure 14.

The inhibition of TLR signaling induced cytokine secretion is particularly important for therapeutic approaches against inflammatory and infectious diseases in which the damage is driven by an excessive inflammatory repose (such as sepsis and systemic inflammatory response syndrome, SIRS).

### 6-BT inhibits the secretion of the proinflammatory cytokines TNFa and IFNbeta

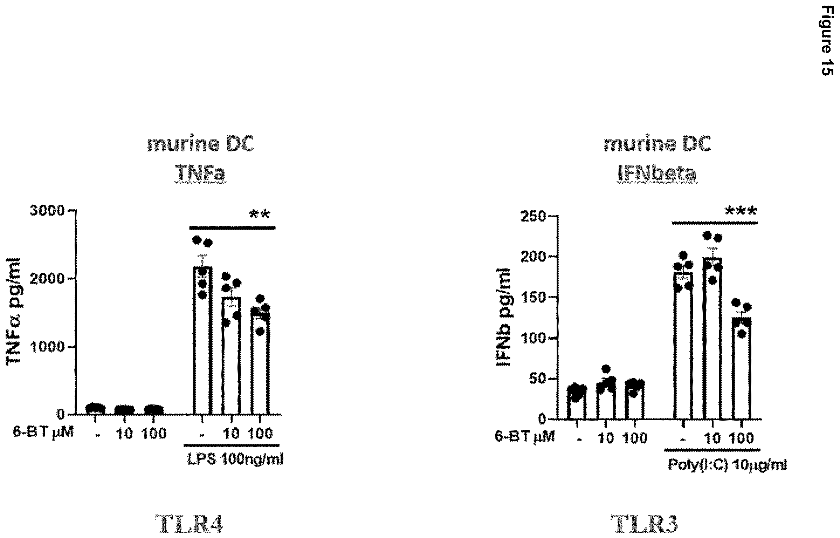

As dendritic cells (DC) are the major antigen-presenting cells crucial in the activation of T cells, the inventors next investigated the effect of 6-BT on murine DC, differentiated with GM-CSF (40ng/ml) by bone marrow cells. As for the monocytes/macrophages, 6-BT inhibits the secretion of the proinflammatory cytokines TNFa and IFNbeta by DC after activation of , respectively, TLR4 (with 100ng/ml LPS) or TLR3 (10ug/ml poly(I:C) ). See Figure 15.

### 6-BT significantly reduced the production of the Th1 cytokine IFNgamma

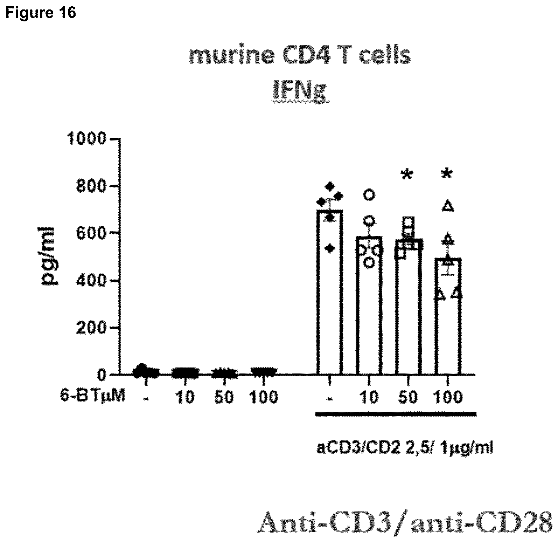

Particularly in the context of autoimmune diabetes, the activity of T cells is driving disease onset and progression. Hence, the inventors further studied the impact of 6-BT on CD4 T cells. To mimic antigen presentation, murine CD4 T cells (isolated from spleens; Uchimura T, The Innate Immune Sensor NLRC3 Acts as a Rheostat that Fine-Tunes T Cell Responses in Infection and Autoimmunity. Immunity 2019) were activated by monoclonal antibodies against CD3 and CD28 (2,5 and 1ug/ml, respectively). In line with the findings on myeloid cells, 6-BT significantly reduced the production of the Th1 cytokine IFNgamma. See Figure 16.

### 6-BT stimulates β-cells differentiation & insulin production

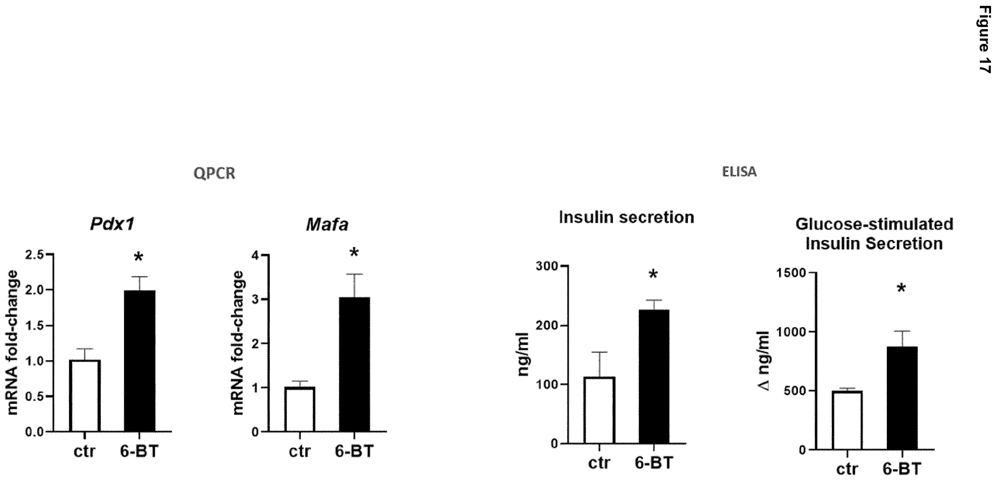

Given the positive association between plasma 6-BT levels and C-peptide concentrations (found in the clinical study), the present inventors next questioned whether 6-BT can exert a direct effect on beta cells. Indeed, the present inventors here see that 6-BT induces, in IS1E beta cells, the gene expression of the transcription factors PDX1 and MAFA, which are important for beta cell maturation and functionality. In agreement, 6-BT also promotes insulin secretion at steady-state and during glucose-stimulated insulin secretion (data shown as the difference between insulin release at starving condition [1mM glucose] and at hyperglycemic state [22mM]), (Paula S, Exercise increases pancreatic β-cell viability in a model of type 1 diabetes through IL-6 signaling. FASEB J 2015). See Figure 17.

### Mechanism(s) of action of 6-bromotryptophan

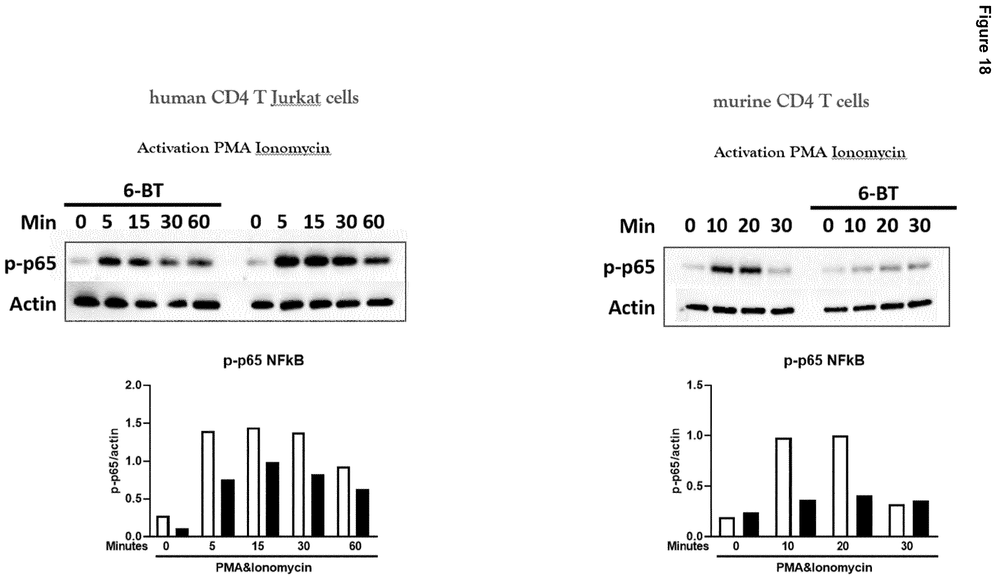

Seeking for the molecular mechanism(s) underlying 6-BT actions, the present inventors first checked 6-BT impact on the activation of the NF-kB pathway, a central pathway in all inflammatory diseases (beyond autoimmunity). Hence, the present inventors quantified the expression of the phosphorylated form of the p65 subunit, regarded as a marker of NFkB activation. Upon T cell activation with PMA (50ng/ml) and ionomycin (1ug/ml), 6-BT could inhibit, at very early time-points (5-10minutes after activation), the NF-kB signaling. This effect was found in both murine and human (Jurkat cells) CD4 T cells. See Figure 18.

### 6-BT inhibits the activation of NFkB in macrophages

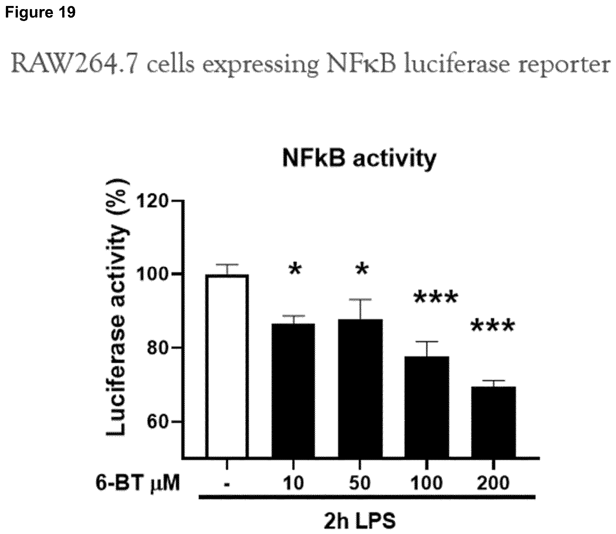

Similarly to what the inventors observed in lymphocytes, 6-BT inhibits the activation of NFkB in macrophages. Using the RAW264.7 murine macrophage cell line stably expressing an NFkB luciferase reporter (Groeneweg M, Lipopolysaccharide-induced gene expression in murine macrophages is enhanced by prior exposure to oxLDL. J Lipid Res 2006.), the inventors disclosed that overnight exposure of macrophages to 6-BT (10-200uM) inhibits the transcriptional activity of the NFkB complex in a dose-dependent manner upon 2h stimulation with LPS (10ng/ml). See Figure 19.

### 6-BT and tryptophan elicit distinct biological activities

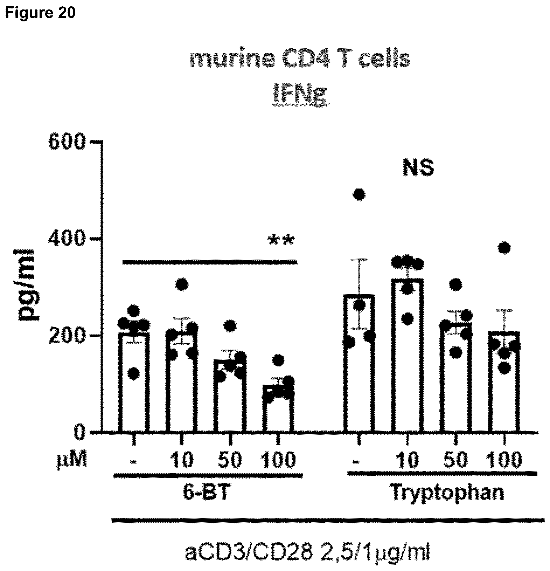

Next, the present inventors questioned whether the effect of 6-BT are specific or can be exerted by tryptophan as well. In murine CD4 T lymphocytes (isolated from murine spleens; Uchimura T, The Innate Immune Sensor NLRC3 Acts as a Rheostat that Fine-Tunes T Cell Responses in Infection and Autoimmunity. Immunity 2019), 6-BT, but not tryptophan, exerted inhibitory effect on IFNgamma production upon CD3/CD28 engagement. This indicates that 6-BT and tryptophan elicit distinct biological activities. See Figure 20.

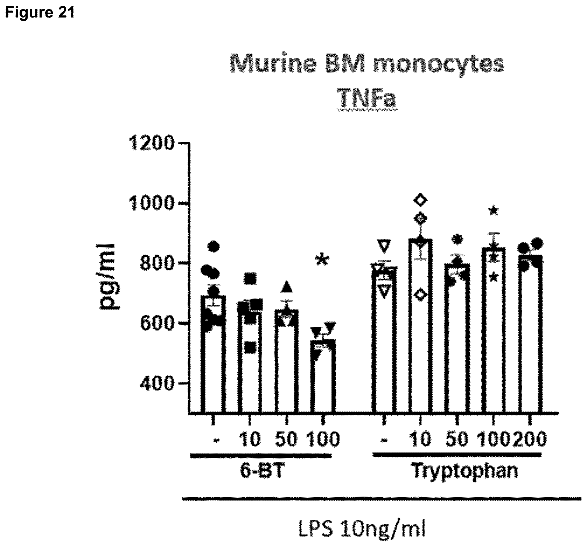

In agreement with the results on DC, exposure of monocytes (isolated from murine bone marrow; Christ A, Western Diet Triggers NLRP3-Dependent Innate Immune Reprogramming. Cell 2018) to 6-BT or tryptophan, shows that the anti-inflammatory effects are specific for the 6-bromotryptophan molecule and not for tryptophan. See Figure 21.

### 6-BT impacts the intracellular metabolism

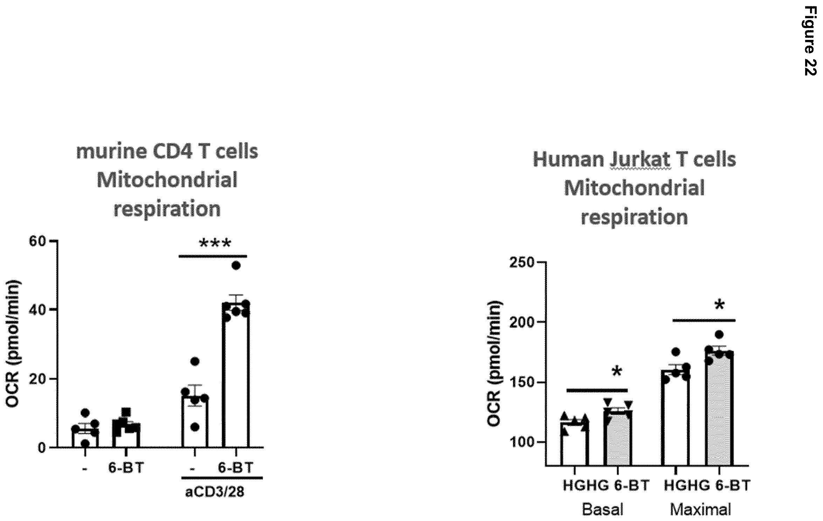

Finally, the present inventors uncover that 6-BT also impacts the intracellular metabolism. Particularly it was found that 6-BT (100uM) promotes the mitochondrial metabolism in murine and human (Jurkat) CD4 T cells. See Figure 22.

OCR=oxygen consumption rate, used as a proxy of cellular utilization of mitochondrial oxidative phosphorylation. OCR was measured using a Seahorse XF Analyzer Uchimura T, The Innate Immune Sensor NLRC3 Acts as a Rheostat that Fine-Tunes T Cell Responses in Infection and Autoimmunity. Immunity 2019; Chou, AIM2 in regulatory T cells restrains autoimmune diseases, Nature 2021).

### 6-BT enhances mitochondrial metabolism

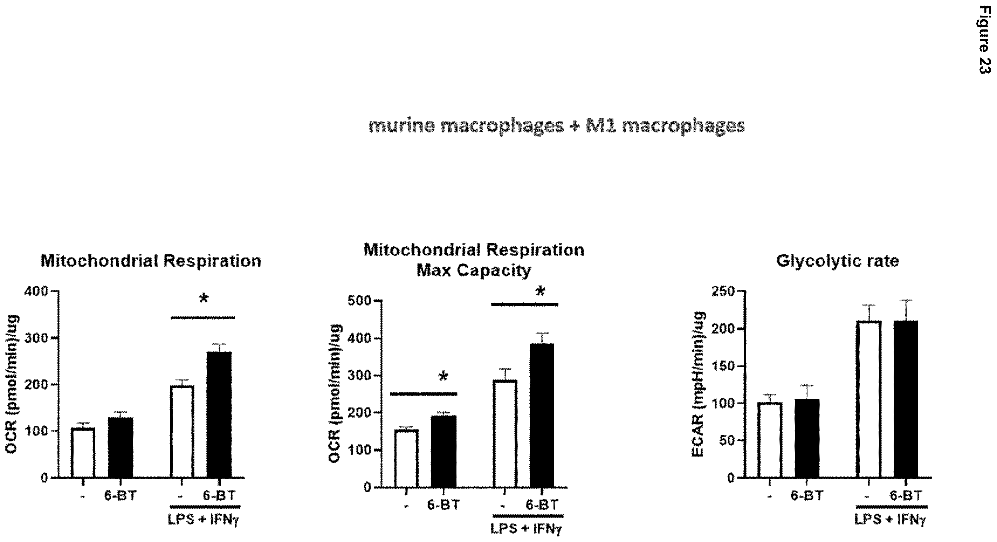

Similarly, 6-BT exposure could enhance the mitochondrial metabolism of pro-inflammatory M1 macrophages (differentiated in presence of LPS and IFNgamma; Cheng et al JCI Insight. 2018;3(22):e120638), without affecting the glycolytic flux. Intracellular metabolism measured using a Seahorse XF Analyzer, Uchimura T, The Innate Immune Sensor NLRC3 Acts as a Rheostat that Fine-Tunes T Cell Responses in Infection and Autoimmunity. Immunity 2019; Chou, AIM2 in regulatory T cells restrains autoimmune diseases, nature 2021). See Figure 23.

### 6-BT could rescue beta cell dysfunctionality in both type 1 and type 2 diabetes

Lastly, the present inventors investigated whether 6-BT may influence the mitochondrial metabolism of beta cells, which rely on ATP and mitochondrial metabolite production for insulin exocytosis. 6-BT increased mitochondria metabolism both at steady-state and under hyperglycemia (25mM glucose) in beta cells (INS1E beta cells). In addition, the present inventors tested the effect of tryptophan on intracellular metabolism and found that, as for the inflammatory markers, it exerted a different effect than 6-BT. Intracellular metabolism measured using a Seahorse XF Analyzer Uchimura T, The Innate Immune Sensor NLRC3 67

Acts as a Rheostat that Fine-Tunes T Cell Responses in Infection and Autoimmunity. Immunity 2019.; Chou, AIM2 in regulatory T cells restrains autoimmune diseases, nature 2021).

Of importance, defects in mitochondria and oxidative metabolism have been reported in T2D (Haythorne, Nature Communications volume 10, Article number: 2474 (2019)), suggesting that 6-BT could rescue beta cell dysfunctionality in both type 1 and type 2 diabetes. Moreover, increase utilization of mitochondria metabolism may counteract ectopic intracellular lipid accumulation and hence counteract obesity.

In conclusion:
- 6-BT exerts pleiotropic effect on multiple cell-types
- It has anti-inflammatory effect on myeloid and lymphoid cells
- It promotes insulin secretion by beta cells

Mechanistically, its biological actions are distinct from the ones of tryptophan.

6-BT does not act through activation of AhR, but it does inhibit NFkB activation and enhance mitochondrial metabolism. The latter is typically used by cells harboring an anti-inflammatory phenotype.

### Applications

Because of its broad effects on multiple cell types, its inhibitory action on NFkB signaling and its promotion of mitochondrial metabolism and fitness, 6-BT may be a novel therapeutic not only in the context of type 1 and type 2 diabetes but also in many other inflammation related disorders such as sepsis, Systemic Inflammatory Response Syndrome (SIRS), and cardiovascular diseases.

### EXAMPLE 5 (does not form part of the invention)

### Desulfovibrio genus levels inversely correlate with presence of Type 2 Diabetes mellitus and glycemic control in cross-sectional cohort (n=369 subjects)

In the same 369 subjects cohort as discussed herein previously, evidence was found that bacteria belonging to the *Desulfovibrio* genus (e.g. *Desulfovibrio piger*) may protect against the onset and progression of Type 2 Diabetes mellitus. More specifically, it was found that the relative abundance of fecal bacteria belonging to the *Desulfovibrio* genus inversely correlates with presence of Type 2 Diabetes mellitus and glycemic control. This suggests that administration of *Desulfovibrio* genus may contribute to Type 2 Diabetes prevention and treatment and thus improved (micro and macrovascular) cardiovacsular complications in Type 2 Diabetes. *Desulfovibrio* genus may help to reduce macrovascular disease (i.e. cardiovascular disease) and microvascular complications also in non-Type 2 Diabetes patients.

### Materials and methods

Fasting plasma targeted metabolite measurements were done by Metabolon (Durham, NC), using ultra high performance liquid chromatography coupled to tandem mass spectrometry (UPLC-MS/MS), as previously described [Koh A, Molinaro A, Stahlman M, et al. Microbially Produced Imidazole Propionate Impairs Insulin Signaling through mTORC1. Cell 2018;175:947-961.e17. doi:10.1016/j.cell.2018.09.055 ].

### Results

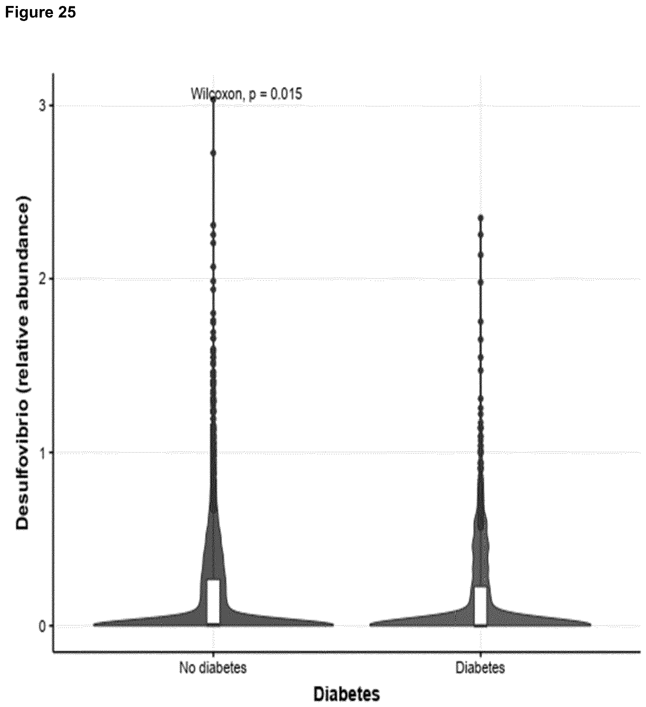

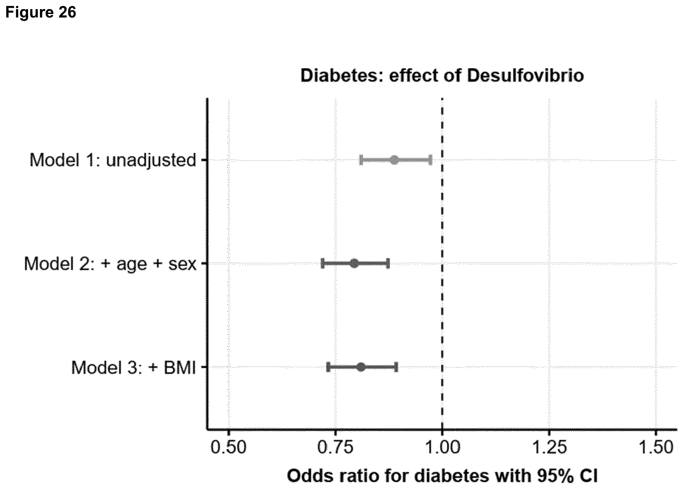

Results are shown in Table 4 and in Figures 25 and 26.

**Table 4 - Correlations between fecal Desulfovibrio genus relative abundance and presence of Diabetes, BMI value, Glucose value, HbA1c value**

| | Diabetes | BMI | Glucose | HbA1c |
|---|---|---|---|---|
| *Desulfovibrio* genus | -0,03* | -0.03* | 0,02 | -0,02 |

| | | | | |
|---|---|---|---|---|
| *. Correlation is significant at the 0.05 level (2-tailed). | | | | |

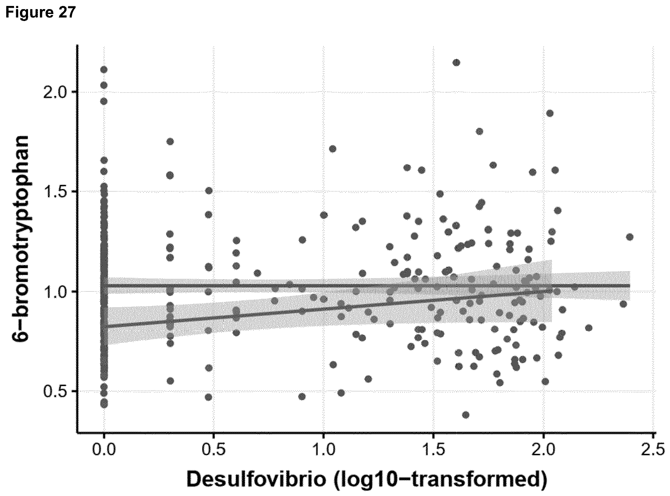

In addition, in type 2 Diabates mellitus patients, a clear relationship can be seen between fecal *Desulfovibrio* relative abundance and plasma 6BT levels. See Figure 27.

## Claims

1. *Desulfovibrio* species, optionally comprised in fecal matter, for use in the prevention or treatment of an inflammation-related disease chosen from the group consisting of type 1 Diabetes mellitus, autoimmune disease, and inflammatory disease, wherein the inflammation-related disease is not type 2 Diabetes mellitus, psoriasis or inflammatory bowel disease, and wherein if the use is in the prevention or treatment of type 1 Diabetes mellitus or autoimmune disease and the *Desulfovibrio* species is comprised in fecal matter, said fecal matter comprises at least 10⁷ *Desulfovibrio* cells per g fecal matter.

2. *Desulfovibrio* species for use according to claim 1, wherein the Diabetes mellitus is chosen from Type 1 Diabetes mellitus.

3. *Desulfovibrio* species for use according to claim 1, wherein the autoimmune disease is chosen from the group consisting of Type 1 Diabetes mellitus, Hashimoto's disease, Graves' disease, Addison's disease, Vitiligo, Rheumatoid arthritis, Bechterew's disease, Celiac disease, Asthma, Chronic Obstructive Pulmonary Disease (COPD), Addison's disease, vasculitis, Multiple sclerosis (MS), Chronic inflammatory demyelinating polyneuropathy (CDIP), and Guillain-Barré syndrome (GBS).

4. *Desulfovibrio* species for use according to claim 1, wherein the inflammatory disease is chosen from the group consisting of cardiovascular inflammation, carditis, endocarditis, myocarditis, pericarditis, vasculitis, arteritis, phlebitis, capillaritis, inflammation of the gastrointestinal tract, esophagitis, gastritis, gastroenteritis, enteritis, colitis, enterocolitis, duodenitis, ileitis, caecitis, appendicitis, proctitis, hepatic inflammation, pulmonary inflammation, skeletal inflammation, Systemic Inflammatory Response Syndrome (SIRS), and sepsis.

5. *Desulfovibrio* species for use according to any one of the previous claims, wherein the *Desulfovibrio* species is chosen from the group consisting of *Desulfovibrio piger, Desulfovibrio fairfieldensis, Desulfovibrio desulfuricans, desulfovibrio indonensis, Desulfovibrio alaskensis, Desulfovibrio vulgaris, Desulfovibrio vietnamensis, Desulfovibrio intestinalis, Desulfovibrio longreachensis, Desulfovibrio termitidis* and *Desulfovibrio gigas.*

6. *Desulfovibrio* species for use according to any one of the previous claims, wherein the *Desulfovibrio* species is *Desulfovibrio piger* or a relative thereof having at least 90%, preferably at least 95%, more preferably at least 97% sequence identity with the 16S rDNA sequence of *Desulfovibrio piger* (SEQ ID NO:1).

7. *Desulfovibrio* species for use according to any one of the previous claims, wherein the *Desulfovibrio* species is combined with a tumor necrosis factor alpha (TNFα) inhibitor, preferably chosen from the group consisting of infliximab, adalimumab, certolizumab pegol, and golimumab.

8. *Desulfovibrio* species for use according to any one of the previous claims, wherein the *Desulfovibrio* species is combined with bacteria from the genus *Eubacterium, Intestinimonas, Bifidobacteria, Lactobacillales* and/or *Akkermansia,* preferably chosen from the group consisting of *Bifidobacterium animalis sub lactis or Bifidobacterium breve, Lactobacillus plantarum. Lactobacillus rhamnosus, Lactobacillus acidophilus, Eubacterium hallii, Intestinimonas butyriciproducens,* and/or *Akkermansia muciniphila.*

9. *Desulfovibrio* species for use according to any one of the previous claims, wherein
- said *Desulfovibrio* species is administered by enteral, preferably oral, or nasal or by rectal administration, and/or by nasoduodenal tube administration; and/or
- the use involves administering said *Desulfovibrio* species to the small intestine, preferably the duodenum.

10. *Desulfovibrio* species for use according to any one of the previous claims, wherein if the *Desulfovibrio* species is comprised in fecal matter, said fecal matter comprises at least 10⁸, 10⁹, or 10¹⁰ *Desulfovibrio* cells per g fecal matter.

11. *Desulfovibrio* species for use according to any one of the previous claims, wherein the *Desulfovibrio* species is not comprised in fecal matter.

12. *Desulfovibrio* species for use according to any of the previous claims, wherein the *Desulfovibrio* species is comprised in a composition, preferably a pharmaceutical composition, more preferably a liquid or solid dosage form, most preferably a capsule, a tablet, or a powder, wherein preferably
- said *Desulfovibrio* species is comprised in said composition in an amount of at least 10⁴, 10⁵, 10⁶, 10⁷, 10⁸ cells; and/or
- attenuated or dead cells of said *Desulfovibrio* species are used.

13. *Desulfovibrio* species for use according to any one of the previous claims, wherein the *Desulfovibrio* species is comprised in and/or encapsulated by an enteric coating, preferable wherein said enteric coating does not dissolute and/or disintegrate in a gastric environment.

14. *Desulfovibrio* species for use according to any one of the previous claims, wherein
- the use involves at least 1, 2, 3, 4, 5, 6, 7, 8, 9, 10 separate administrations of said *Desulfovibrio* species, preferably with intervals of at least 1, 2, 3, 4, 5, 6, 7, 8 weeks between said separate administrations; and/or
- the subject to be treated is a mammal, preferably a human.

## Patentansprüche

1. *Desulfovibrio-*Spezies, gegebenenfalls in Fäkalsubstanz enthalten, zur Verwendung bei der Prävention oder Behandlung einer entzündungsbezogenen Erkrankung, die aus der Gruppe bestehend aus Diabetes mellitus Typ 1, Autoimmunerkrankung und entzündlicher Erkrankung gewählt ist, wobei die entzündungsbezogene Erkrankung nicht Diabetes mellitus Typ 2, Psoriasis oder entzündliche Darmerkrankung ist, und wobei, wenn die Verwendung bei der Prävention oder Behandlung von Diabetes mellitus Typ 1 oder Autoimmunerkrankung erfolgt und die *Desulfovibrio*-Spezies in Fäkalsubstanz enthalten ist, die Fäkalsubstanz mindestens 10⁷ *Desulfovibrio*-Zellen pro g Fäkalsubstanz umfasst.

2. *Desulfovibrio*-Spezies zur Verwendung nach Anspruch 1, wobei der Diabetes mellitus aus Diabetes mellitus Typ 1 gewählt ist.

3. *Desulfovibrio*-Spezies zur Verwendung nach Anspruch 1, wobei die Autoimmunerkrankung aus der Gruppe bestehend aus Diabetes mellitus Typ 1, Hashimoto-Krankheit, Morbus Basedow, Morbus Addison, Vitiligo, rheumatoider Arthritis, Morbus Bechterew, Zöliakie, Asthma, chronisch obstruktiver Lungenerkrankung (COPD), Morbus Addison, Vaskulitis, multipler Sklerose (MS), chronisch entzündlicher demyelinisierender Polyneuropathie (CDIP) und Guillain-Barré-Syndrom (GBS) gewählt ist.

4. *Desulfovibrio*-Spezies zur Verwendung nach Anspruch 1, wobei die entzündliche Erkrankung aus der Gruppe bestehend aus kardiovaskulärer Entzündung, Karditis, Endokarditis, Myokarditis, Perikarditis, Vaskulitis, Arteriitis, Phlebitis, Capillaritis, Entzündung des Magen-Darm-Trakts, Ösophagitis, Gastritis, Gastroenteritis, Enteritis, Colitis, Enterocolitis, Duodenitis, Ileitis, Typhlitis, Appendizitis, Proktitis, Leberentzündung, Lungenentzündung, Skelettentzündung, SIRS (Systemic Inflammatory Response Syndrome) und Sepsis gewählt ist.

5. *Desulfovibrio-*Spezies zur Verwendung nach einem der vorhergehenden Ansprüche, wobei die *Desulfovibrio-*Spezies aus der Gruppe bestehend aus *Desulfovibrio piger, Desulfovibrio fairfieldensis, Desulfovibrio desulfuricans, desulfovibrio indonensis, Desulfovibrio alaskensis, Desulfovibrio vulgaris, Desulfovibrio vietnamensis, Desulfovibrio intestinalis, Desulfovibrio longreachensis, Desulfovibrio termitidis* und *Desulfovibrio gigas* gewählt ist.

6. *Desulfovibrio-*Spezies zur Verwendung nach einem der vorhergehenden Ansprüche, wobei es sich bei der *Desulfovibrio*-Spezies um *Desulfovibrio piger* oder eine Verwandte davon mit mindestens 90 %, bevorzugt mindestens 95 %, stärker bevorzugt mindestens 97 % Sequenzidentität mit der 16S-rDNA-Sequenz von *Desulfovibrio piger* (SEQ ID NO:1) handelt.

7. *Desulfovibrio-*Spezies zur Verwendung nach einem der vorherigen Ansprüche, wobei die *Desulfovibrio-*Spezies mit einem Tumornekrosefaktor-alpha(TNFα)-Inhibitor kombiniert ist, der vorzugsweise aus der Gruppe bestehend aus Infliximab, Adalimumab, Certolizumab-Pegol und Golimumab gewählt ist.

8. *Desulfovibrio-*Spezies zur Verwendung nach einem der vorherigen Ansprüche, wobei die *Desulfovibrio-*Spezies mit Bakterien aus der Gattung *Eubacterium, Intestinimonas, Bifidobacteria, Lactobacillales* und/oder *Akkermansia* kombiniert ist, die vorzugsweise aus der Gruppe bestehend aus *Bifidobacterium animalis sub lactis* oder *Bifidobacterium breve*, *Lactobacillus plantarum*, *Lactobacillus rhamnosus, Lactobacillus acidophilus, Eubacterium hallii, Intestinimonas butyriciproducens* und/oder *Akkermansia muciniphila* gewählt sind.

9. *Desulfovibrio-*Spezies zur Verwendung nach einem der vorhergehenden Ansprüche, wobei
- die *Desulfovibrio-*Spezies durch enterale, vorzugsweise orale, oder nasale oder rektale Verabreichung und/oder durch nasoduodenale Sondenverabreichung verabreicht wird; und/oder
- bei der Verwendung die *Desulfovibrio*-Spezies dem Dünndarm, vorzugsweise dem Zwölffingerdarm, verabreicht wird.

10. *Desulfovibrio*-Spezies zur Verwendung nach einem der vorhergehenden Ansprüche, wobei, wenn die *Desulfovibrio*-Spezies in Fäkalsubstanz enthalten ist, die Fäkalsubstanz mindestens 10⁸, 10⁹ oder 10¹⁰ *Desulfovibrio*-Zellen pro g Fäkalsubstanz umfasst.

11. *Desulfovibrio*-Spezies zur Verwendung nach einem der vorhergehenden Ansprüche, wobei die *Desulfovibrio*-Spezies nicht in Fäkalsubstanz enthalten ist.

12. *Desulfovibrio*-Spezies zur Verwendung nach einem der vorherigen Ansprüche, wobei die *Desulfovibrio*-Spezies in einer Zusammensetzung, bevorzugt einer pharmazeutischen Zusammensetzung, stärker bevorzugt einer flüssigen oder festen Darreichungsform, am meisten bevorzugt einer Kapsel, einer Tablette oder einem Pulver, enthalten ist, wobei vorzugsweise
- die *Desulfovibrio-*Spezies in der Zusammensetzung in einer Menge von mindestens 10⁴, 10⁵, 10⁶, 10⁷, 10⁸ Zellen enthalten ist; und/oder
- attenuierte oder tote Zellen der *Desulfovibrio*-Spezies verwendet werden.

13. *Desulfovibrio-*Spezies zur Verwendung nach einem der vorherigen Ansprüche, wobei die *Desulfovibrio-*Spezies in einer magensaftresistenten Beschichtung enthalten ist und/oder durch diese verkapselt ist, bevorzugt wobei sich die magensaftresistente Beschichtung nicht in einem Magenmilieu auflöst und/oder zersetzt.

14. *Desulfovibrio-*Spezies zur Verwendung nach einem der vorhergehenden Ansprüche, wobei
- die Verwendung mindestens 1, 2, 3, 4, 5, 6, 7, 8, 9, 10 getrennte Verabreichungen der *Desulfovibrio-*Spezies, vorzugsweise mit Intervallen von mindestens 1, 2, 3, 4, 5, 6, 7, 8 Wochen zwischen den genannten getrennten Verabreichungen umfasst; und/oder
- das zu behandelnde Individuum ein Säuger, vorzugsweise ein Mensch ist.

## Revendications

1. Espèce de *Desulfovibrio*, facultativement présente dans des matières fécales, pour une utilisation dans la prévention ou le traitement d'une maladie liée à une inflammation choisie dans le groupe constitué par le diabète sucré de type 1, une maladie auto-immune et une maladie inflammatoire, la maladie liée à l'inflammation n'étant pas le diabète sucré de type 2, le psoriasis ou une maladie inflammatoire chronique de l'intestin, et, si l'utilisation est la prévention ou le traitement du diabète sucré de type 1 ou d'une maladie auto-immune et que l'espèce de *Desulfovibrio* est présente dans les matières fécales, lesdites matières fécales comprennent au moins 10⁷ cellules de *Desulfovibrio* par g de matières fécales.

2. Espèce de *Desulfovibrio* selon la revendication 1, le diabète sucré étant choisi parmi le diabète sucré de type 1.

3. Espèce de *Desulfovibrio* selon la revendication 1, dans lequel la maladie auto-immune est choisie dans le groupe constitué par le diabète sucré de type 1, la maladie de Hashimoto, la maladie de Graves, la maladie d'Addison, le vitiligo, la polyarthrite rhumatoïde, la maladie de Bechterew, la maladie cœliaque, l'asthme, la bronchopneumopathie chronique obstructive (BPCO), la maladie d'Addison, la vascularite, la sclérose en plaques (SEP), la polyneuropathie inflammatoire démyélinisante chronique (PIDC) et le syndrome de Guillain-Barré (SGB).

4. Espèce de *Desulfovibrio* selon la revendication 1, la maladie inflammatoire étant choisie dans le groupe constitué par l'inflammation cardiovasculaire, la cardite, l'endocardite, la myocardite, la péricardite, la vascularite, l'artérite, la phlébite, la capillarite, l'inflammation du tractus gastro-intestinal, l'œsophagite, la gastrite, la gastro-entérite, l'entérite, la colite, l'entérocolite, la duodénite, l'iléite, la cæcite, l'appendicite, la proctite, l'inflammation hépatique, l'inflammation pulmonaire, l'inflammation squelettique, le syndrome de réponse inflammatoire systémique (SRIS) et la septicémie.

5. Espèce de *Desulfovibrio* selon l'une quelconque des revendications précédentes, l'espèce de *Desulfovibrio* étant choisie dans le groupe constitué par *Desulfovibrio piger*, *Desulfovibrio fairfieldensis*, *Desulfovibrio desulfuricans*, *Desulfovibrio indonensis*, *Desulfovibrio alaskensis*, *Desulfovibrio vulgaris*, *Desulfovibrio vietnamensis*, *Desulfovibrio intestinalis*, *Desulfovibrio longreachensis*, *Desulfovibrio termitidis* et *Desulfovibrio gigas*.

6. Espèce de *Desulfovibrio* selon l'une quelconque des revendications précédentes, l'espèce de *Desulfovibrio* étant *Desulfovibrio piger* ou une espèce apparentée présentant au moins 90 %, de préférence au moins 95 %, plus préférentiellement au moins 97 % d'identité de séquence avec la séquence d'ADNr 16S de *Desulfovibrio piger* (SEQ ID NO : 1).

7. Espèce de *Desulfovibrio* selon l'une quelconque des revendications précédentes, l'espèce de *Desulfovibrio* étant combinée avec un inhibiteur du facteur alpha de nécrose tumorale (TNFα), de préférence choisi dans un groupe constitué par l'infliximab, l'adalimumab, le certolizumab pegol et le golimumab.

8. Espèce de *Desulfovibrio* selon l'une quelconque des revendications précédentes, l'espèce de *Desulfovibrio* étant combinée avec des bactéries du genre *Eubacterium*, *Intestinimonas*, *Bifidobacteria*, *Lactobacillales* et/ou *Akkermansia*, de préférence choisi dans un groupe constitué par *Bifidobacterium animalis sub lactis* ou *Bifidobacterium breve*, *Lactobacillus plantarum*, *Lactobacillus rhamnosus*, *Lactobacillus acidophilus*, *Eubacterium hallii*, *Intestinimonas butyriciproducens* et/ou *Akkermansia muciniphila*.

9. Espèce de *Desulfovibrio* selon l'une quelconque des revendications précédentes, **caractérisée en ce que**
- ladite espèce de *Desulfovibrio* est administrée par administration entérale, de préférence orale ou nasale ou rectale, et/ou par administration par sonde nasoduodénale ; et/ou
- L'utilisation implique l'administration de ladite espèce de *Desulfovibrio* à l'intestin grêle, de préférence au duodénum.

10. Espèce de *Desulfovibrio* selon l'une quelconque des revendications précédentes, **caractérisée en ce que**, si l'espèce de *Desulfovibrio* est présente dans des matières fécales, lesdites matières fécales comprennent au moins 10⁸, 10⁹ ou 10¹⁰ cellules de *Desulfovibrio* par g de matières fécales.

11. Espèce de *Desulfovibrio* selon l'une quelconque des revendications précédentes, l'espèce de *Desulfovibrio* n'étant pas présente dans des matières fécales.

12. Espèce de *Desulfovibrio* selon l'une quelconque des revendications précédentes, l'espèce de *Desulfovibrio* étant comprise dans une composition, de préférence une composition pharmaceutique, plus préférentiellement une forme posologique liquide ou solide, tout particulièrement de préférence une capsule, un comprimé ou une poudre, **caractérisée en ce que**, de préférence
- ladite espèce de *Desulfovibrio* est comprise dans ladite composition à raison d'au moins 10⁴, 10⁵, 10⁶, 10⁷, 10⁸ cellules ; et/ou
- des cellules atténuées ou mortes de ladite espèce de *Desulfovibrio* sont utilisées.

13. Espèce de *Desulfovibrio* selon l'une quelconque des revendications précédentes, l'espèce de *Desulfovibrio* étant comprise et/ou encapsulée dans un enrobage entérique, de préférence, ledit revêtement entérique ne se dissolvant pas et/ou ne se désintégrant pas dans un environnement gastrique.

14. Espèce de *Desulfovibrio* selon l'une quelconque des revendications précédentes, **caractérisée en ce que**
- l'utilisation implique au moins 1, 2, 3, 4, 5, 6, 7, 8, 9, 10 administrations séparées de ladite espèce de *Desulfovibrio*, de préférence avec des intervalles d'au moins 1, 2, 3, 4, 5, 6, 7, 8 semaines entre lesdites administrations séparées ; et/ou
- le sujet à traiter est un mammifère, de préférence un humain.
